# EUROPEAN PATENT SPECIFICATION

(11) **EP 3 366 749 B1**
(45) Date of publication and mention of the grant of the patent: **08.12.2021**
(21) Application number: 16857700.5
(22) Date of filing: 10.10.2016
(51) Int. Cl.: C09K 11/06, C07D 403/10, C07D 251/12, C07D 209/82, C07D 307/91, C07D 333/76, H01L 51/50, H01L 51/00

(54) **COMPOSITION FOR ORGANIC OPTOELECTRONIC ELEMENT, ORGANIC OPTOELECTRONIC ELEMENT, AND DISPLAY DEVICE**
ZUSAMMENSETZUNG FÜR ORGANISCHES OPTOELEKTRONISCHES ELEMENT, ORGANISCHES OPTOELEKTRONISCHES ELEMENT UND ANZEIGEVORRICHTUNG
COMPOSITION POUR ÉLÉMENT OPTOÉLECTRONIQUE ORGANIQUE, ÉLÉMENT OPTOÉLECTRONIQUE ORGANIQUE ET DISPOSITIF D'AFFICHAGE

(30) Priority: 23.10.2015 KR 20150148231; 07.10.2016 KR 20160129962
(43) Date of publication of application: 29.08.2018
(73) Proprietor: Samsung SDI Co., Ltd, Gyeonggi-do 17084 (KR)
(72) Inventor: LEE, Sangshin, Suwon-si Gyeonggi-do 16678 (KR); KANG, Dong Min, Suwon-si Gyeonggi-do 16678 (KR); KIM, Jun Seok, Suwon-si Gyeonggi-do 16678 (KR); LEE, Byoungkwan, Suwon-si Gyeonggi-do 16678 (KR); LEE, Hanill, Suwon-si Gyeonggi-do 16678 (KR); JANG, Kipo, Suwon-si Gyeonggi-do 16678 (KR); HAN, Sujin, Suwon-si Gyeonggi-do 16678 (KR); KIM, Youngkwon, Suwon-si Gyeonggi-do 16678 (KR); YU, Eun Sun, Suwon-si Gyeonggi-do 16678 (KR)
(74) Representative: Michalski Hüttermann & Partner Patentanwälte mbB
(86) International application number: PCT/KR2016/011323
(87) International publication number: WO 2017/069442

(56) References cited:
- EP-A1- 2 776 443
- EP-A2- 2 452 997
- KR-A- 20120 132 423
- KR-A- 20140 094 408
- KR-A- 20150 037 318
- KR-A- 20150 042 603
- KR-A- 20150 104 260
- KR-B1- 101 502 316

## Description

### [Technical Field]

An organic optoelectronic device and a display device are disclosed.

### [Background Art]

An organic optoelectronic device (organic optoelectric diode) is a device that converts electrical energy into photoenergy, and vice versa.

An organic optoelectronic device may be classified as follows in accordance with its driving principles. One is a photoelectric device where excitons are generated by photoenergy, separated into electrons and holes, and are transferred to different electrodes to generate electrical energy, and the other is a light emitting device where a voltage or a current is supplied to an electrode to generate photoenergy from electrical energy.

Examples of the organic optoelectronic device are an organic photoelectric device, an organic light emitting diode, an organic solar cell, and an organic photo conductor drum.

Of these, an organic light emitting diode (OLED) has recently drawn attention due to an increase in demand for flat panel displays. The organic light emitting diode is a device converting electrical energy into light by applying current to an organic light emitting material, and has a structure in which an organic layer is disposed between an anode and a cathode.

A blue organic light emitting diode having a long life-span is considered to be one of the critical factors for realizing a long life-span full color display. Accordingly, development of a blue organic light emitting diode having a long life-span is being actively researched. In order to solve this problem, a blue organic light emitting diode having a long life-span is provided in this invention.

The documents EP 2452997 A2, EP 2777443 A1 and KR 2015 0037318 A disclose an OLED device comprising a heterocyclic compound electron transporting material and a hole transporting arylamine compound.

### [DISCLOSURE]

### [Technical Problem]

An embodiment provides an organic optoelectronic device capable of realizing high efficiency and long life-span characteristics.

Another embodiment provides an organic optoelectronic device including the composition for an organic optoelectronic device.

Yet another embodiment provides a display device including the organic optoelectronic device.

### [Technical Solution]

According to one embodiment, a composition for an organic optoelectronic device includes at least one first compound represented by formula 1 and at least one second compound represented by formula 2.

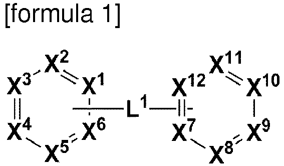

In formula 1,
X¹ to X¹² are independently N, C, or CR^{a},
   at least one of X¹ to X⁶ is N,
   at least one of X⁷ to X¹² is N,
R^{a'}s are independently hydrogen, deuterium, a substituted or unsubstituted C1 to C30 alkyl group, a substituted or unsubstituted C1 to C30 alkenyl group, a substituted or unsubstituted C1 to C30 alkynyl group, a substituted or unsubstituted C6 to C30 aryl group, a substituted or unsubstituted C6 to C30 aryloxy group, a substituted or unsubstituted C6 to C30 arylthio group, a substituted or unsubstituted C2 to C30 heteroaryl group, a hydroxyl group, a thiol group, or a combination thereof,
R^{a'}s are independently present or adjacent R^{a'}s are linked with each other to provide a ring, and
L¹ is a C6 to C30 arylene group that is unsubstituted or substituted with deuterium, a C1 to C40 silyl group, a C1 to C30 alkyl group, a C3 to C30 cycloalkyl group, a C2 to C30 heterocycloalkyl group, or a C6 to C30 aryl group;

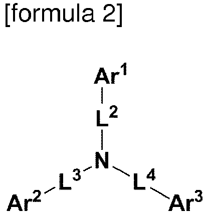

In formula 2,
L² to L⁴ are independently a single bond, a substituted or unsubstituted C6 to C30 arylene group, or a substituted or unsubstituted C2 to C30 heteroarylene group,
Ar¹ to Ar³ are independently, a substituted or unsubstituted C6 to C30 aryl group, a substituted or unsubstituted C2 to C30 heterocyclic group, or a combination thereof, and
when specific definition is not otherwise provided, "substituted" of formulas 1 and 2 refers to replacement of at least one hydrogen by deuterium, a halogen, a hydroxyl group, an amino group, a C1 to C30 amine group, a C6 to C30 arylamine group, a nitro group, a C1 to C40 silyl group, a C1 to C30 alkyl group, a C3 to C30 cycloalkyl group, a C2 to C30 heterocycloalkyl group, a C6 to C30 aryl group, a C2 to C30 heterocyclic group, a C1 to C20 alkoxy group, a C1 to C10 trifluoroalkyl group, or a cyano group; and the first compound and the second compound are included in a weight ratio of 1:9 to 9:1.

According to another embodiment, an organic optoelectronic device including the composition for an organic optoelectronic device is provided.

According to yet another embodiment, a display device including the organic optoelectronic device is provided.

### [Advantageous Effects]

An organic optoelectronic device having high efficiency and a long life-span may be realized.

### [Description of the Drawings]

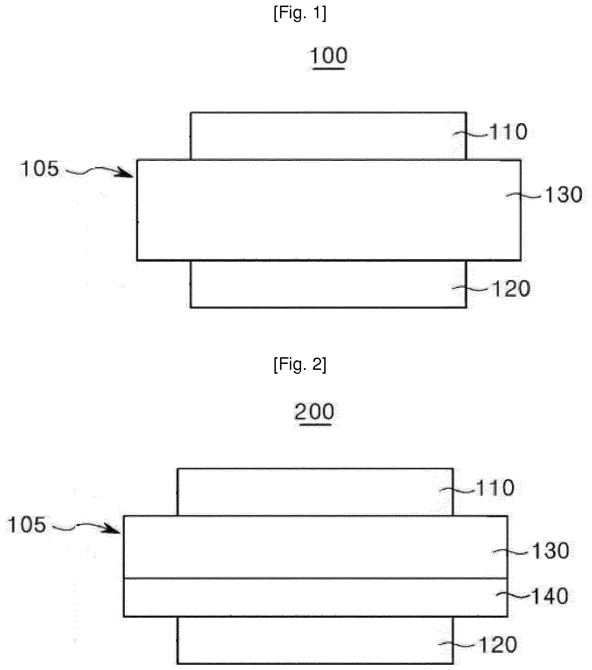

FIGS. 1 and 2 are schematic cross-sectional views of an organic optoelectronic device according to an embodiment.

### <Description of symbols>

- 100, 200:: organic light emitting diode
- 105:: organic layer
- 110:: cathode
- 120:: anode
- 130:: light emitting layer
- 140:: auxiliary layer

### [Best Mode]

Hereinafter, embodiments of the present invention are described in detail. However, these embodiments are exemplary, the present invention is not limited thereto and the present invention is defined by the scope of claims.

In the present specification, when a definition is not otherwise provided, "substituted" refers to replacement of at least one hydrogen of a substituent or a compound by deuterium, a halogen, a hydroxy group, an amino group, a C1 to C30 amine group, a C6 to C30 arylamine group, a nitro group, a C1 to C40 silyl group, a C1 to C30 alkyl group, a C3 to C30 cycloalkyl group, a C2 to C30 heterocycloalkyl group, a C6 to C30 aryl group, a C2 to C30 heterocyclic group, a C1 to C20 alkoxy group, a C1 to C10 trifluoroalkyl group, or a cyano group.

In addition, two adjacent substituents of the substituted C1 to C30 amine group, C1 to C40 silyl group, C1 to C30 alkyl group, C1 to C10 alkylsilyl group, C3 to C30 cycloalkyl group, C2 to C30 heterocycloalkyl group, C6 to C30 aryl group, C2 to C30 heterocyclic group, or C1 to C20 alkoxy group may be linked with each other to form a fused ring. For example, the substituted C6 to C30 aryl group may be linked with another adjacent substituted C6 to C30 aryl group to form a substituted or unsubstituted fluorene ring and the substituted C6 to C30 aryl group may be linked with an adjacent C1 to C30 alkenyl group to form a triphenylene ring, a naphthalene ring, a pyrazine ring, a quinazoline ring, a quinoxaline ring, a phenanthroline ring, or the like.

In the present specification when specific definition is not otherwise provided, "hetero" refers to one including one to three heteroatoms selected from N, O, S, P, and Si, and remaining carbons in one functional group.

In the present specification, when a definition is not otherwise provided, "alkyl group" refers to an aliphatic hydrocarbon group. The alkyl group may be "a saturated alkyl group" without any double bond or triple bond.

The alkyl group may be a C1 to C30 alkyl group. More specifically, the alkyl group may be a C1 to C20 alkyl group or a C1 to C10 alkyl group. For example, a C1 to C4 alkyl group may have one to four carbon atoms in the alkyl chain, and may be selected from methyl, ethyl, propyl, iso-propyl, n-butyl, isobutyl, sec-butyl, and t-butyl.

Specific examples of the alkyl group may be a methyl group, an ethyl group, a propyl group, an isopropyl group, a butyl group, an isobutyl group, a t-butyl group, a pentyl group, a hexyl group, a cyclopropyl group, a cyclobutyl group, a cyclopentyl group, a cyclohexyl group, and the like.

In the present specification, "an aryl group" refers to a group including at least one hydrocarbon aromatic moiety, and
all elements of the hydrocarbon aromatic moiety have p-orbitals which form conjugation, for example a phenyl group, a naphthyl group, and the like,
two or more hydrocarbon aromatic moieties may be linked by a sigma bond and may be, for example a biphenyl group, a terphenyl group, a quarterphenyl group, and the like, and
two or more hydrocarbon aromatic moieties are fused directly or indirectly to provide a non-aromatic fused ring. For example, it may be a fluorenyl group.

The aryl group may include a monocyclic, polycyclic, or fused ring polycyclic (i.e., rings sharing adjacent pairs of carbon atoms) functional group.

In the present specification, "a heterocyclic group" is a generic concept of a heteroaryl group, and may include at least one heteroatom selected from N, O, S, P, and Si instead of carbon (C) in a cyclic compound such as an aryl group, a cycloalkyl group, a fused ring thereof, or a combination thereof. When the heterocyclic group is a fused ring, the entire ring or each ring of the heterocyclic group may include one or more heteroatoms.

For example, "a heteroaryl group" may refer to an aryl group including at least one heteroatom selected from N, O, S, P, and Si instead of carbon (C). Two or more heteroaryl groups are linked by a sigma bond directly, or when the C2 to C60 heteroaryl group includes two or more rings, the two or more rings may be fused. When the heteroaryl group is a fused ring, each ring may include one to three heteroatoms.

Specific examples of the heteroaryl group may be a pyridinyl group, a pyrimidinyl group, a pyrazinyl group, a pyridazinyl group, a triazinyl group, a quinolinyl group, an isoquinolinyl group, and the like.

More specifically, the substituted or unsubstituted C6 to C30 aryl group and/or the substituted or unsubstituted C2 to C30 heterocyclic group may be a substituted or unsubstituted phenyl group, a substituted or unsubstituted naphthyl group, a substituted or unsubstituted anthracenyl group, a substituted or unsubstituted phenanthryl group, a substituted or unsubstituted naphthacenyl group, a substituted or unsubstituted pyrenyl group, a substituted or unsubstituted biphenyl group, a substituted or unsubstituted p-terphenyl group, a substituted or unsubstituted m-terphenyl group, a substituted or unsubstituted chrysenyl group, a substituted or unsubstituted triphenylenyl group, a substituted or unsubstituted perylenyl group, a substituted or unsubstituted fluorenyl group, a substituted or unsubstituted indenyl group, a substituted or unsubstituted furanyl group, a substituted or unsubstituted thiophenyl group, a substituted or unsubstituted pyrrolyl group, a substituted or unsubstituted pyrazolyl group, a substituted or unsubstituted imidazolyl group, a substituted or unsubstituted triazolyl group, a substituted or unsubstituted oxazolyl group, a substituted or unsubstituted thiazolyl group, a substituted or unsubstituted oxadiazolyl group, a substituted or unsubstituted thiadiazolyl group, a substituted or unsubstituted pyridinyl group, a substituted or unsubstituted pyrimidinyl group, a substituted or unsubstituted pyrazinyl group, a substituted or unsubstituted triazinyl group, a substituted or unsubstituted benzofuranyl group, a substituted or unsubstituted benzothiophenyl group, a substituted or unsubstituted benzimidazolyl group, a substituted or unsubstituted indolyl group, a substituted or unsubstituted quinolinyl group, a substituted or unsubstituted isoquinolinyl group, a substituted or unsubstituted quinazolinyl group, a substituted or unsubstituted quinoxalinyl group, a substituted or unsubstituted naphthyridinyl group, a substituted or unsubstituted benzoxazinyl group, a substituted or unsubstituted benzthiazinyl group, a substituted or unsubstituted acridinyl group, a substituted or unsubstituted phenazinyl group, a substituted or unsubstituted phenothiazinyl group, a substituted or unsubstituted phenoxazinyl group, a substituted or unsubstituted carbazolyl group, a substituted or unsubstituted dibenzofuranyl group, a substituted or unsubstituted dibenzothiophenyl group, or a combination thereof, but are not limited thereto.

In the present specification, a single bond refers to a direct bond not by carbon or a hetero atom except carbon, and specifically the meaning that L is a single bond means that a substituent linked with L directly bonds with a central core. That is, in the present specification, the single bond does not refer to methylene that is bonded via carbon.

In the present specification, hole characteristics refer to an ability to donate an electron to form a hole when an electric field is applied, and that a hole formed in the anode may be easily injected into the light emitting layer, and a hole formed in a light emitting layer may be easily transported into an anode and transported in the light emitting layer due to conductive characteristics according to a highest occupied molecular orbital (HOMO) level.

In addition, electron characteristics refers to an ability to accept an electron when an electric field is applied, and that an electron formed in a cathode may be easily injected into the light emitting layer, and an electron formed in a light emitting layer may be easily transported into a cathode and transported in the light emitting layer due to conductive characteristics according to a lowest unoccupied molecular orbital (LUMO) level.

Hereinafter, a composition for an organic optoelectronic device according to an embodiment is described.

A composition for an organic optoelectronic device according to an embodiment includes at least one first compound represented by formula 1 and at least one second compound represented by formula 2.

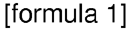

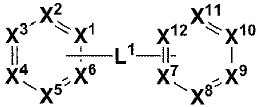

In formula 1, X¹ to X¹² are independently N, C, or CR^{a}, at least one of X¹ to X⁶ is N, at least one of X⁷ to X¹² is N, R^{a'}s are independently hydrogen, deuterium, a substituted or unsubstituted C1 to C30 alkyl group, a substituted or unsubstituted C1 to C30 alkenyl group, a substituted or unsubstituted C1 to C30 alkynyl group, a substituted or unsubstituted C6 to C30 aryl group, a substituted or unsubstituted C6 to C30 aryloxy group, a substituted or unsubstituted C6 to C30 arylthio group, a substituted or unsubstituted C2 to C30 heteroaryl group, a hydroxyl group, a thiol group, or a combination thereof, R^{a'}s are independently present or adjacent R^{a'}s are linked with each other to provide a ring, and
L¹ is a C6 to C30 arylene group that is unsubstituted or substituted with deuterium, a C1 to C40 silyl group, a C1 to C30 alkyl group, a C3 to C30 cycloalkyl group, a C2 to C30 heterocycloalkyl group, or a C6 to C30 aryl group;

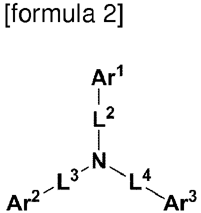

In formula 2,
L² to L⁴ are independently a single bond, a substituted or unsubstituted C6 to C30 arylene group, or a substituted or unsubstituted C2 to C30 heteroarylene group,
Ar¹ to Ar³ are independently, a substituted or unsubstituted C6 to C30 aryl group, a substituted or unsubstituted C2 to C30 heterocyclic group, or a combination thereof, and
when specific definition is not otherwise provided, "substituted" of formulas 1 and 2 refers to replacement of at least one hydrogen by deuterium, a halogen, a hydroxyl group, an amino group, a C1 to C30 amine group, a C6 to C30 arylamine group, a nitro group, a C1 to C40 silyl group, a C1 to C30 alkyl group, a C3 to C30 cycloalkyl group, a C2 to C30 heterocycloalkyl group, a C6 to C30 aryl group, a C2 to C30 heterocyclic group, a C1 to C20 alkoxy group, a C1 to C10 trifluoroalkyl group, or a cyano group.

A composition for an organic optoelectronic device according to one embodiment of the present invention uses a first compound including a compound in which nitrogen-containing heterorings are linked through an arylene linker and thus having excellent electron injection and transport characteristics and a second compound including at least one amine group substituted with at least one aryl group and/or heteroaryl group and thus having excellent hole injection and transport characteristics to form an light emitting layer and resultantly, may lower a driving voltage and simultaneously realize an organic light emitting diode having a long life-span and high efficiency.

The first compound respectively includes at least one nitrogen-containing ring in substituents positioned at both ends of the linking group, L¹ and thus has a structure of easily accepting an electron when an electric field is applied and thus may increase the injection amount of electrons and have relatively strong electron transport characteristics.

In particular, various characteristics such as charge injection characteristics, a deposition temperature, a glass transition temperature, and the like may be adjusted depending on the number of N included in the substituents at both ends, a linking direction of the linking group, L¹, the number of an arylene group linked thereby, and the like.

Accordingly, the first compound may lower a driving voltage of an organic optoelectronic device and also, improve its efficiency.

Formula 1 according to an example embodiment of the present invention may be, for example represented by one of formula 1-I to formula 1-IV in accordance with that adjacent R^{a'}s are linked to each other to form a ring.

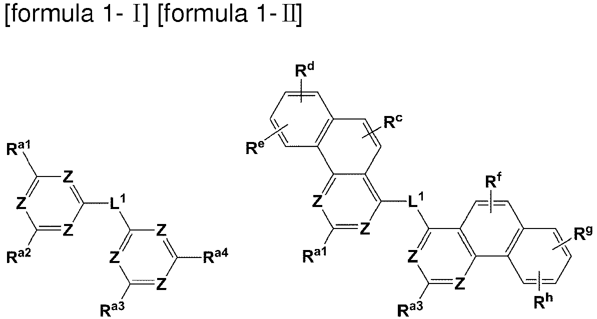

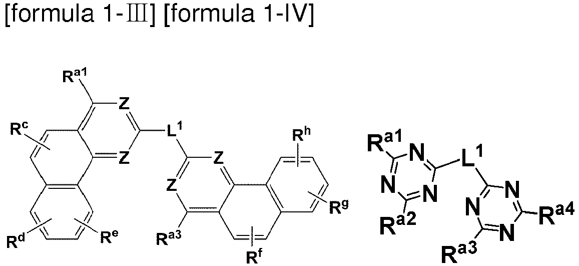

In formulas 1-1 to 1-IV, L¹ is the same described above, Z's are independently N or CR^{a}, wherein R^{a} is the same as described above, and in each ring including Z, at least one Z may be N.

Various characteristics such as charge injection characteristics, a deposition temperature, a glass transition temperature, and the like may be adjusted depending on the number of N included in a substituent at both ends. Specifically, when the entire number of the N is greater than or equal to 4, electron injection characteristics may be stronger. For example, the number of the N may be respectively (1 and 3), (2 and 2), (2 and 3), or (3 and 3) and in particular, when the number of the N is (3 and 3), stability and mobility of injected electrons may be particularly improved.

In an exemplary embodiment of the present invention, R^{a}, R^{a1} to R^{a4}, R^{c}, R^{d}, R^{e}, R^{f}, R^{g}, and R^{h} are independently hydrogen, deuterium, a substituted or unsubstituted C1 to C30 alkyl group, a substituted or unsubstituted C6 to C30 aryl group, a substituted or unsubstituted C2 to C30 heteroaryl group, or a combination thereof,
specifically, hydrogen or a substituted or unsubstituted C6 to C30 aryl group, and
more specifically hydrogen, a substituted or unsubstituted phenyl group, a substituted or unsubstituted biphenyl group, a substituted or unsubstituted terphenyl group, a substituted or unsubstituted quaterphenyl group, a substituted or unsubstituted naphthyl group, a substituted or unsubstituted anthracenyl group, a substituted or unsubstituted triphenylene group, a substituted or unsubstituted phenanthrenyl group, or a substituted or unsubstituted pyrenyl group.

For example, they may be substituted with deuterium, a C1 to C10 alkyl group, a C6 to C12 aryl group or they may be selected from the following unsubstituted groups of Group 1, but are not limited thereto.

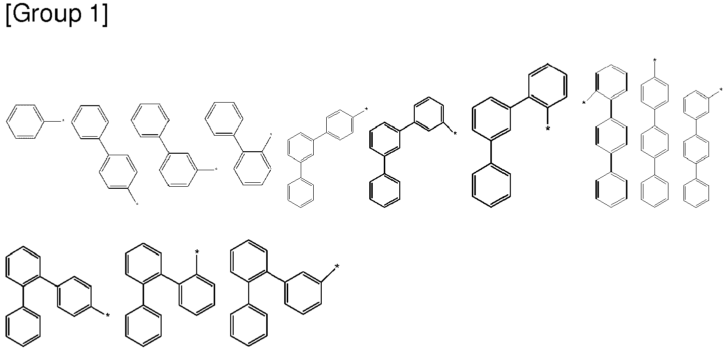

In Group 1, * is a linking point.

On the other hand, in another embodiment of the present invention, the adjacent R^{a'}s may be linked to each other to form a ring, wherein the ring formed by linking the R^{a'}s may be a substituted or unsubstituted quinolinyl group, a substituted or unsubstituted isoquinolinyl group, a substituted or unsubstituted quinolinyl group quinazolinyl group, or a substituted or unsubstituted phenanthrolinyl group.

Specifically, R^{a'}s are independently present to be a substituted or unsubstituted pyridinyl group, or a substituted or unsubstituted triazinyl group, or
the adjacent R^{a'}s are linked with each other to provide a substituted or unsubstituted quinazolinyl group.
L¹ of formula 1 according to an example embodiment of the present invention may be specifically a phenylene group that is unsubstituted or substituted with deuterium, a C1 to C40 silyl group, a C1 to C30 alkyl group, or a C6 to C30 aryl group; a biphenylene group that is unsubstituted or substituted with deuterium, a C1 to C40 silyl group, a C1 to C30 alkyl group, or a C6 to C30 aryl group; a terphenylene group that is unsubstituted or substituted with deuterium, a C1 to C40 silyl group, a C1 to C30 alkyl group, or a C6 to C30 aryl group; or a quarterphenylene group that is unsubstituted or substituted with deuterium, a C1 to C40 silyl group, a C1 to C30 alkyl group, or a C6 to C30 aryl group.

Particularly, various characteristics such as charge injection characteristics, a deposition temperature, a glass transition temperature, and the like may be adjusted depending on a linking direction of a linking group, L¹ and the number of an arylene group linked therewith, and the linking group, L¹ may be for example, selected from substituted or unsubstituted linking groups provided in Group 2 but is not limited thereto.

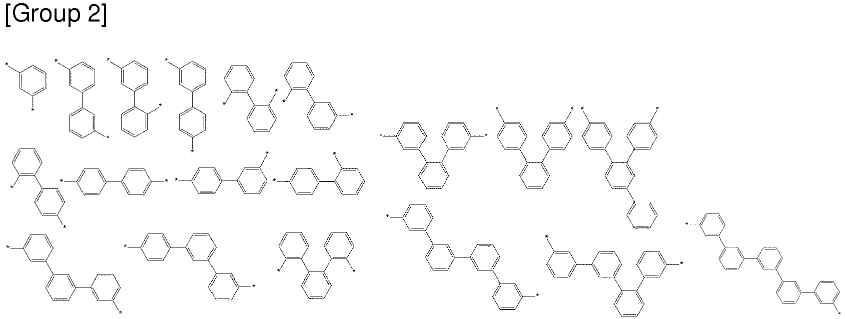

In Group 2, * is a linking point with an adjacent atom.

When the L¹ is the same as above, formula 1 may be a dimer including two N-containing heterorings, this dimer may easily adjust hole mobility and electron mobility characteristics depending on characteristics of a substituent and thus suppress formation of a crystalline phase compared with a trimer including three N-containing heterorings.

In particular, as a ratio of a moiety linked at a para position in the L¹ increases, a molecule itself becomes firm and thus may increase charge mobility.

In addition, a deposition temperature and a glass transition temperature may be adjusted by controlling ratios of moieties linked as a meta or ortho position in the L¹. Particularly, a LUMO energy level and thus charge injection characteristics may be adjusted by controlling the number of aryl group included in the L¹ and a kind of and a direction of substituents included in the heterorings.

In an example embodiment of the present invention, in formula 1, X¹ to X¹² are independently N, C, or CR^{a}, at least two of X¹ to X⁶ are N, at least two of X⁷ to X¹² are N, R^{a'}s are independently hydrogen, deuterium, a substituted or unsubstituted C1 to C30 alkyl group, a substituted or unsubstituted C6 to C30 aryl group, a substituted or unsubstituted C2 to C30 heteroaryl group, or a combination thereof, and L¹ is a C6 to C30 arylene group that is unsubstituted or substituted with deuterium, a C1 to C30 alkyl group, or a C6 to C30 aryl group, and
more specifically, a heterocyclic group composed of X¹ to X⁶ may be a pyrimidinyl group or a triazinyl group and a heterocyclic group composed of X⁷ to X¹² may be a pyrimidinyl group or a triazinyl group.

For example, X¹ to X¹² of formula 1 may independently be N, C, or CR^{a}, three of X¹ to X⁶ may be N, and three of X⁷ to X¹² may be N. For example, the heterocyclic group consisting of X¹ to X⁶ and the heterocyclic group consisting of X⁷ to X¹² may be a triazinyl group.

Herein, when specific definition is not otherwise provided, "substituted" refers to replacement of at least one hydrogen by deuterium, a halogen, a hydroxyl group, a C1 to C40 silyl group, a C1 to C30 alkyl group, a C3 to C30 cycloalkyl group, a C2 to C30 heterocycloalkyl group, a C6 to C30 aryl group, or a C2 to C30 heterocyclic group.

Specifically, R^{a'}s may be a substituted or unsubstituted C6 to C30 aryl group, and
the C6 to C30 aryl group may be a substituted or unsubstituted phenyl group, a substituted or unsubstituted biphenyl group, a substituted or unsubstituted terphenyl group, a substituted or unsubstituted quaterphenyl group, a substituted or unsubstituted naphthyl group, a substituted or unsubstituted anthracenyl group, a substituted or unsubstituted fluorenyl group, a substituted or unsubstituted triphenylene group, or a substituted or unsubstituted phenanthrenyl group. In addition, for example, in "substituted or unsubstituted" of R^{a}'s, "substituted" may be deuterium, a C1 to C10 alkyl group, a C6 to 20 aryl group, or a pyrimidine group.

The first compound represented by formula 1 may be for example compounds of Group 3, but is not limited thereto.

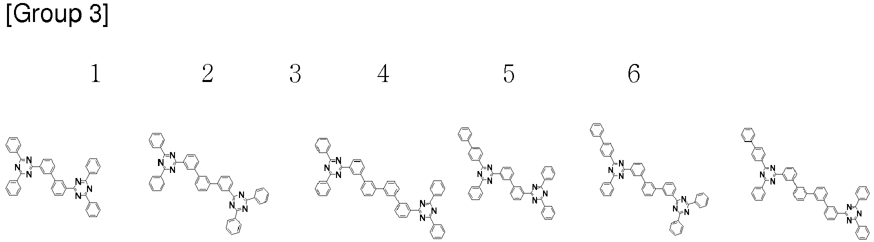

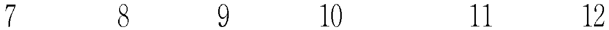

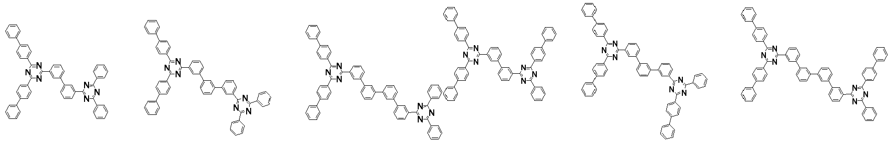

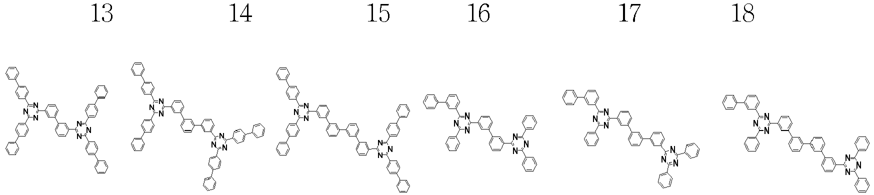

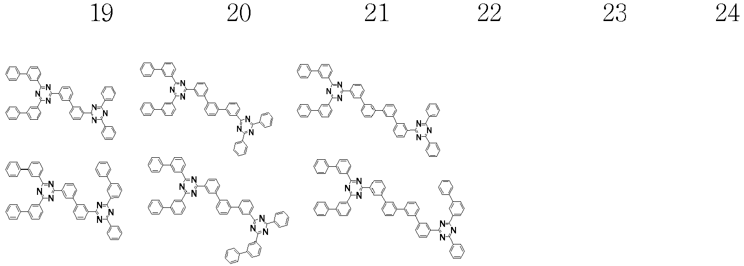

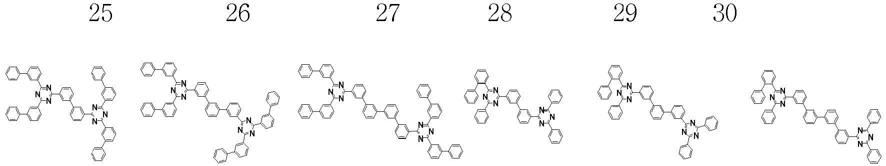

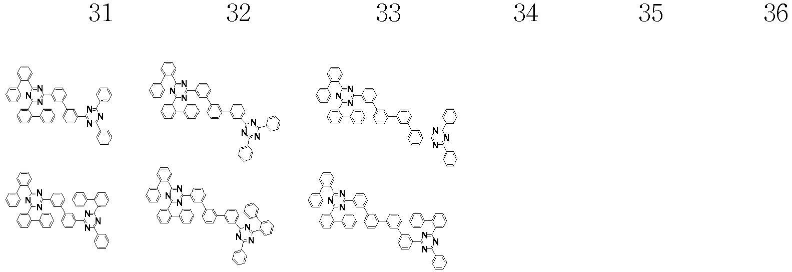

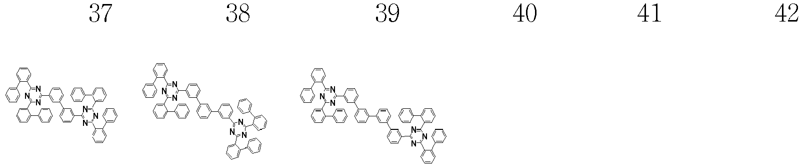

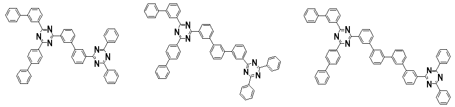

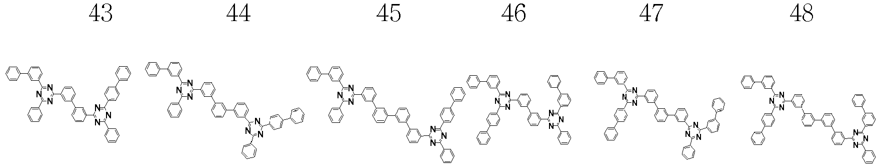

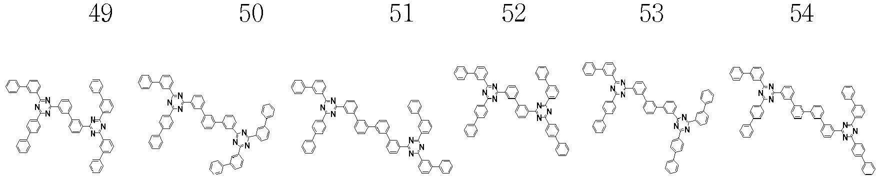

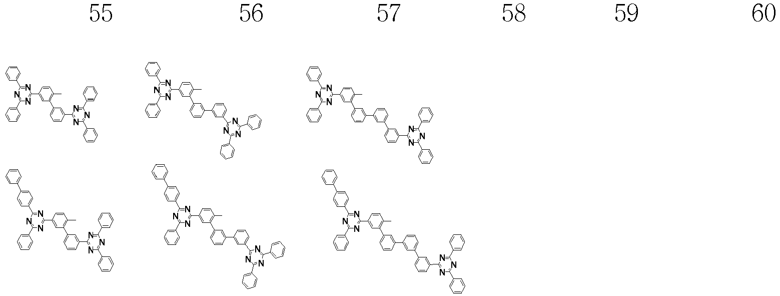

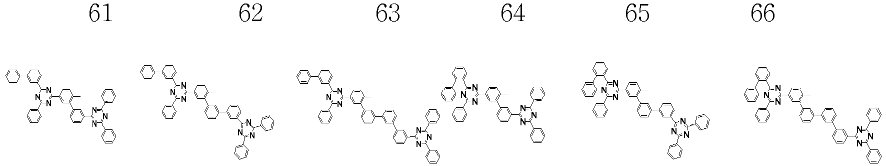

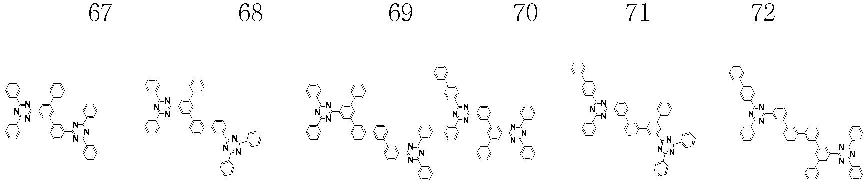

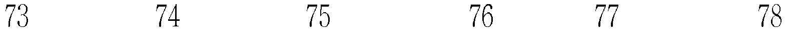

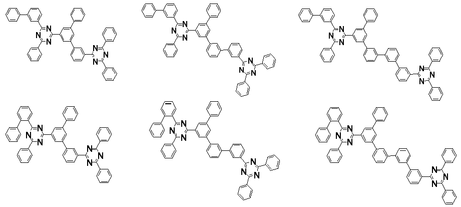

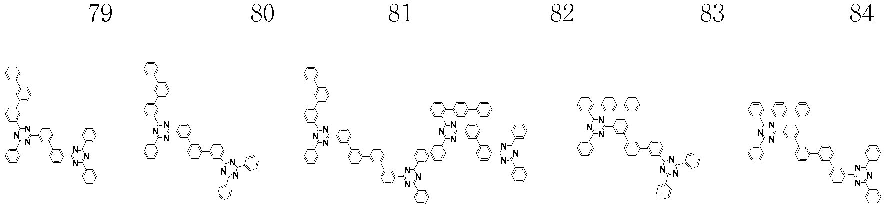

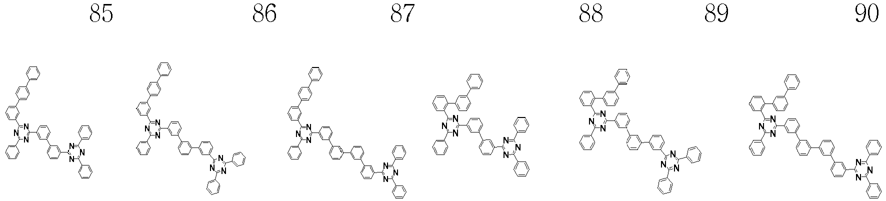

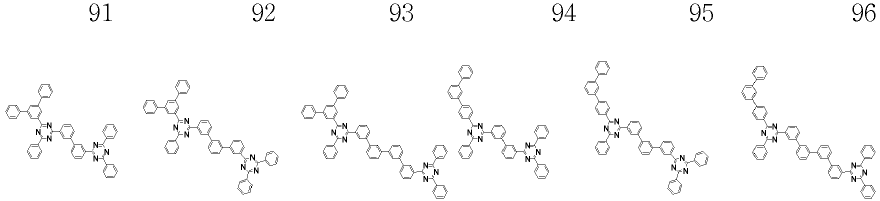

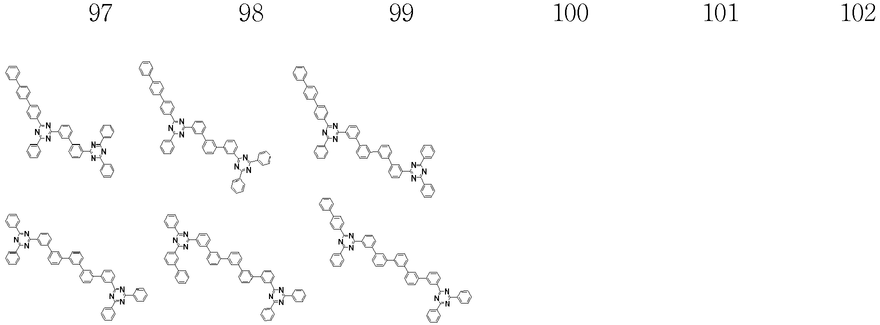

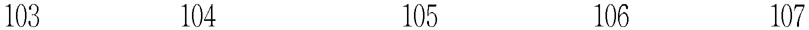

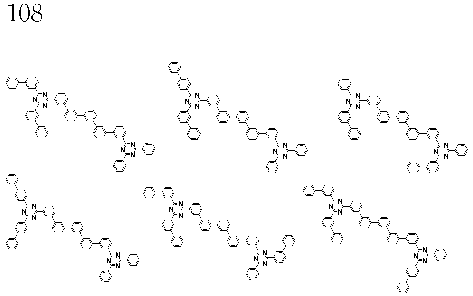

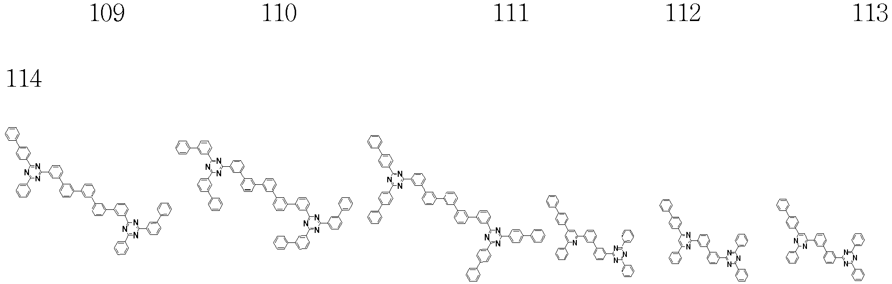

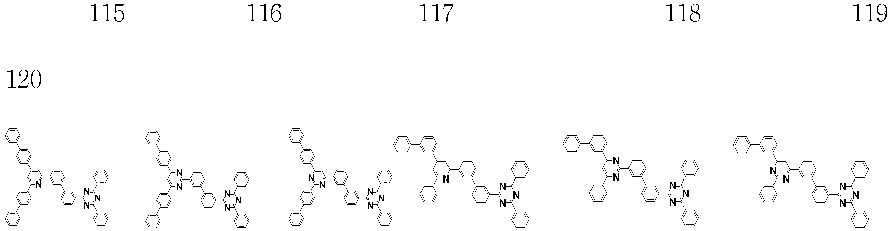

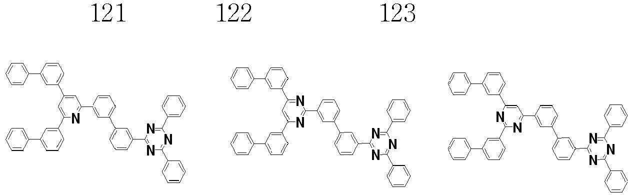

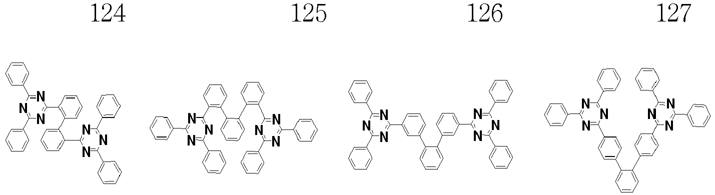

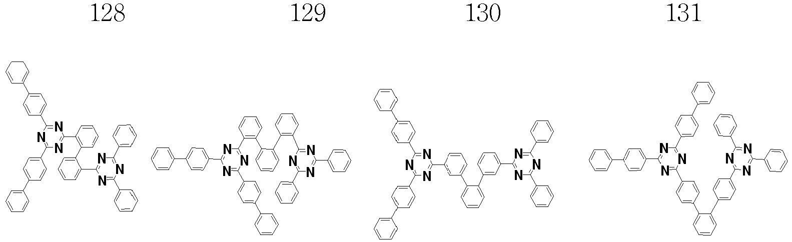

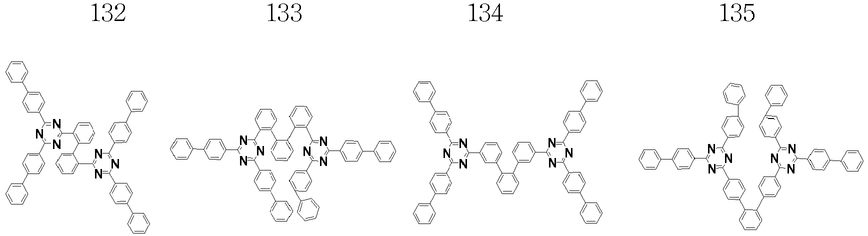

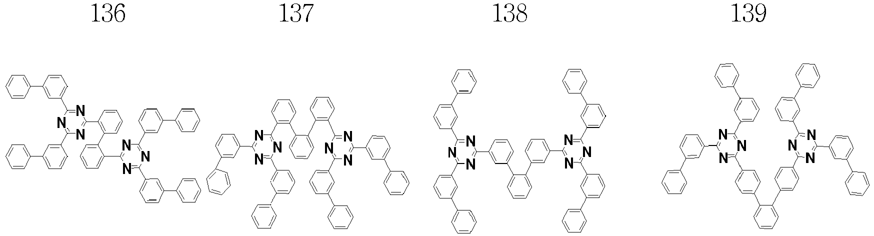

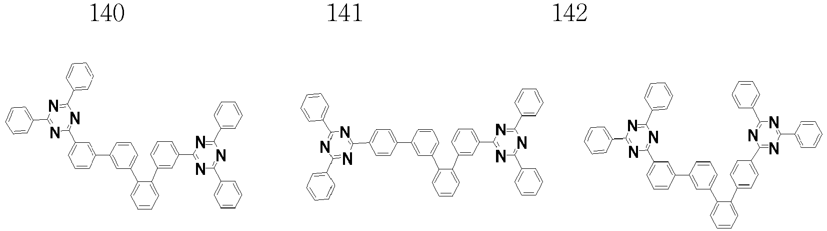

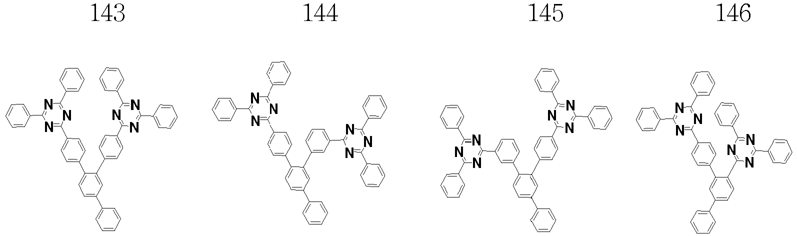

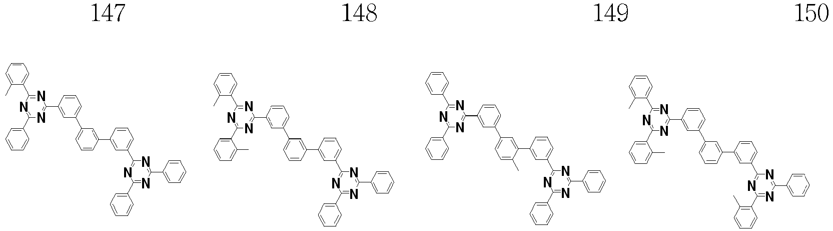

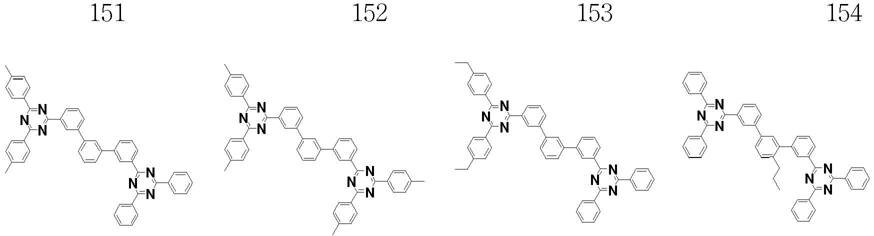

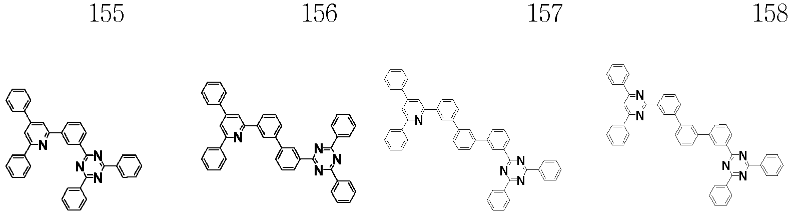

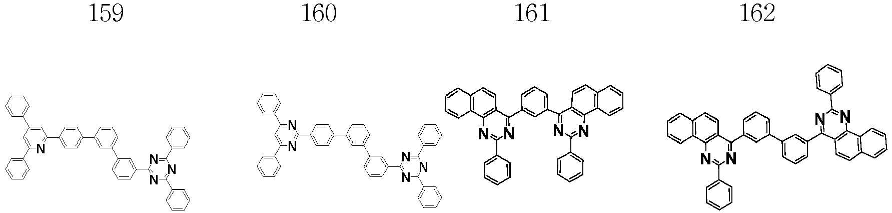

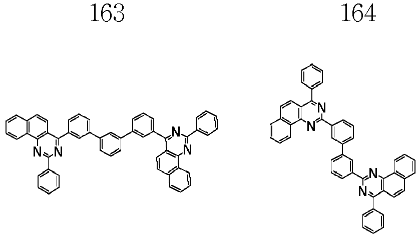

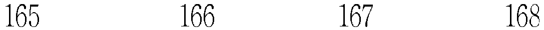

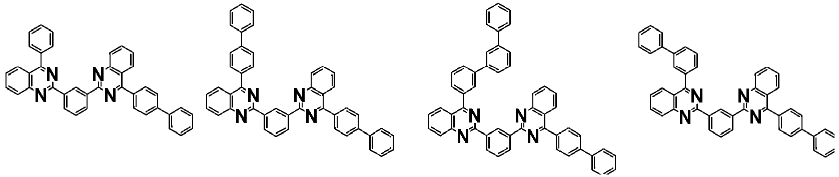

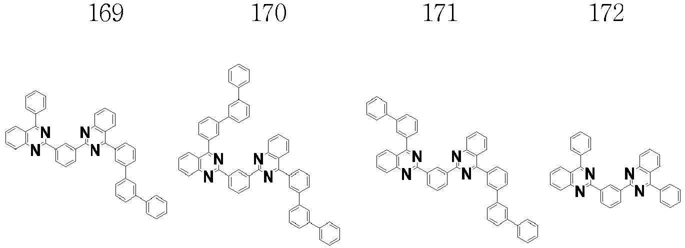

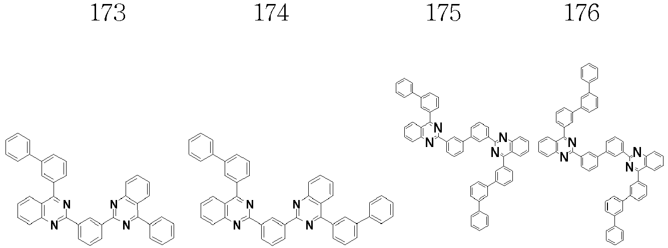

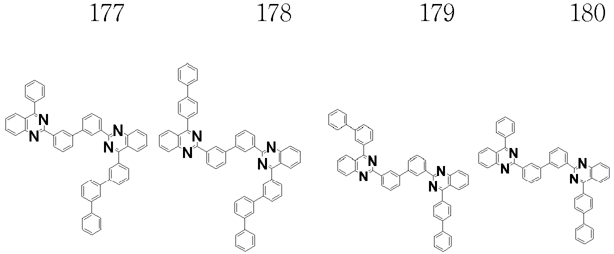

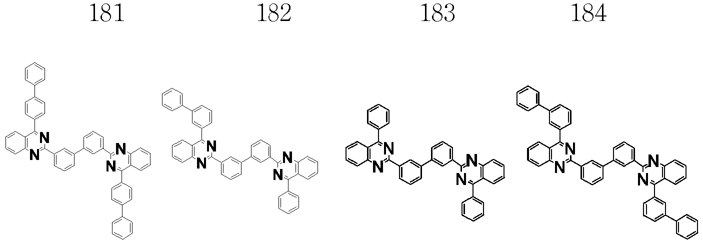

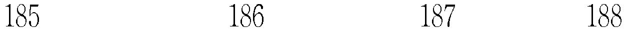

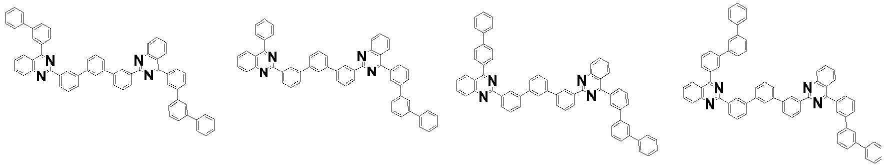

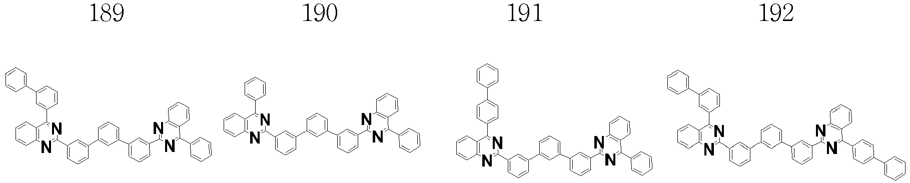

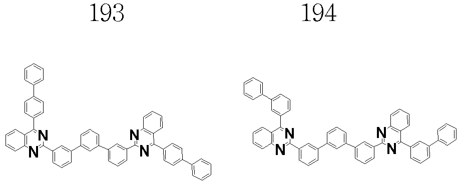

The first compound used in a light emitting layer has strong electron transport and inject characteristics, and thus crystallinity of a material may be increased.

Accordingly, the first compound may be used with a material having strong hole transport and injection characteristics rather than used alone to balance hole transport and injection characteristics/electron transport and injection characteristics.

The compound having strong hole transport and injection characteristics may be a second compound represented by formula 2.

The second compound is a compound having relatively strong hole characteristics due to the amine group substituted with at least one aryl group and/or heteroaryl group and is used in a light emitting layer with the first compound to increase charge mobility and stability and thereby to remarkably improve luminous efficiency and life-span characteristics.

L² to L⁴ of formula 2 may independently be a single bond, a substituted or unsubstituted C6 to C30 arylene group, or a substituted or unsubstituted C2 to C30 heteroarylene group,
for example, a single bond, a substituted or unsubstituted phenylene group, a substituted or unsubstituted biphenylene group, a substituted or unsubstituted naphthyl group, a substituted or unsubstituted fluorenyl group, a substituted or unsubstituted pyridinyl group, a substituted or unsubstituted pyrimidinyl group, a substituted or unsubstituted quinolinyl group, or a combination thereof.

In addition, Ar¹ to Ar³ of formula 2 may independently be a substituted or unsubstituted C6 to C30 aryl group, a substituted or unsubstituted C2 to C30 heterocyclic group, or a combination thereof,
more specifically, a substituted or unsubstituted phenyl group, a substituted or unsubstituted biphenyl group, a substituted or unsubstituted terphenyl group, a substituted or unsubstituted quaterphenyl group, a substituted or unsubstituted naphthyl group, a substituted or unsubstituted fluorenyl group, a substituted or unsubstituted carbazolyl group, a substituted or unsubstituted dibenzofuranyl group, a substituted or unsubstituted dibenzothiophenyl group, a substituted or unsubstituted anthracenyl group, a substituted or unsubstituted phenanthrenyl group, a substituted or unsubstituted triphenylene group, a substituted or unsubstituted quinolinyl group, a substituted or unsubstituted pyridinyl group, a substituted or unsubstituted pyrimidinyl group, a substituted or unsubstituted thiophenyl group, or a combination thereof.

For example, Ar¹ to Ar³ may independently be selected from substituted or unsubstituted groups of Group 4.

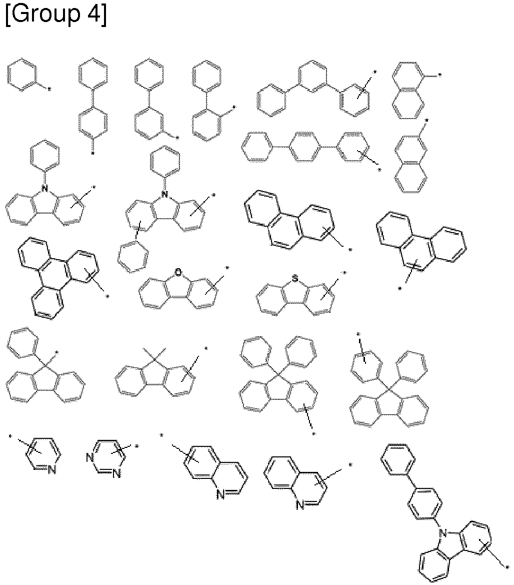

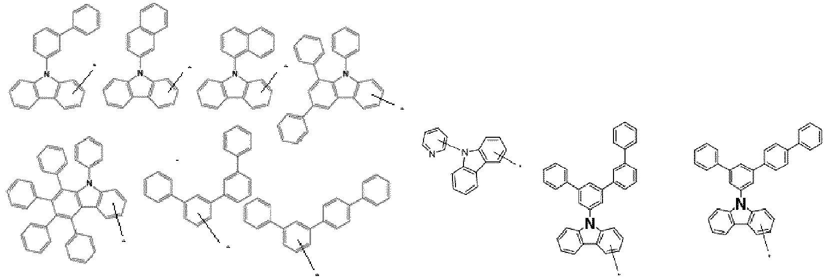

In Group 4, * is a linking point with an adjacent atom.

Ar¹ to Ar³ of formula 2 according to an example embodiment of the present invention may be further substituted with a C6 to C30 aryl group, or a C1 to C30 alkyl group or the substituents may be linked with each other to form a fused ring.

For example, when the substituents of Ar¹ to Ar³ are a triphenylmethyl group, two adjacent phenyl group to the triphenylmethyl group may be linked to form a fluorene ring.

The second compound represented by formula 2 may be, for example compounds of Group 5, but is not limited thereto.

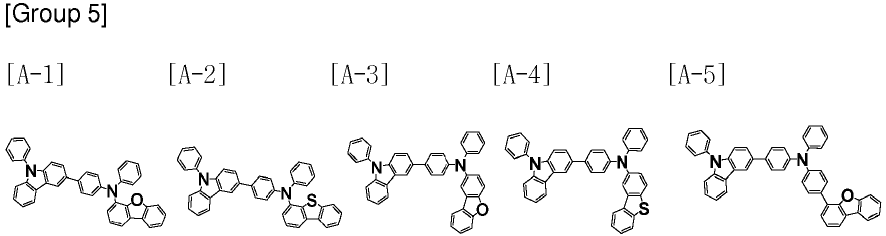

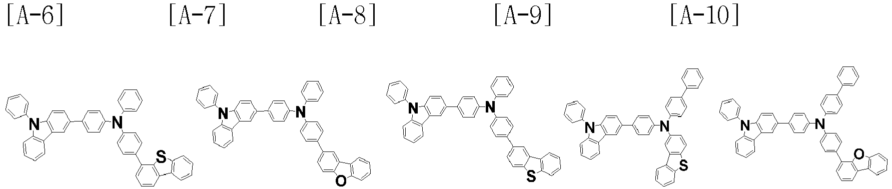

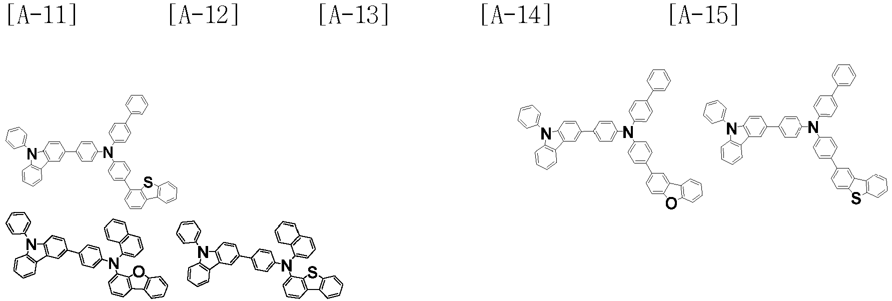

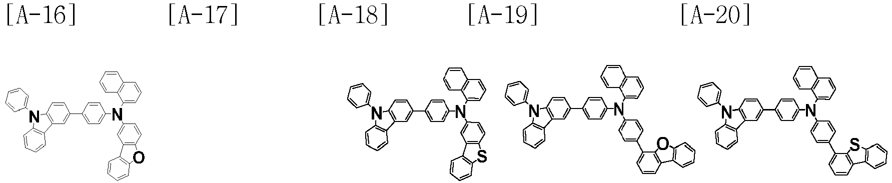

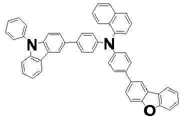

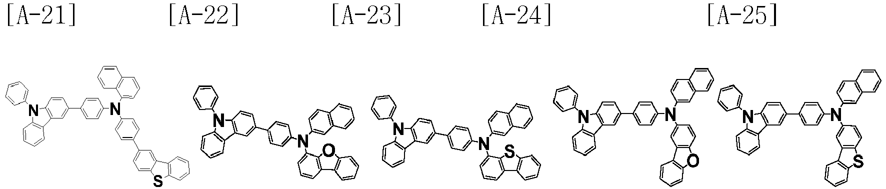

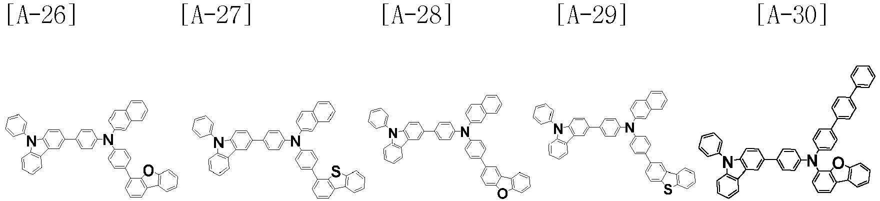

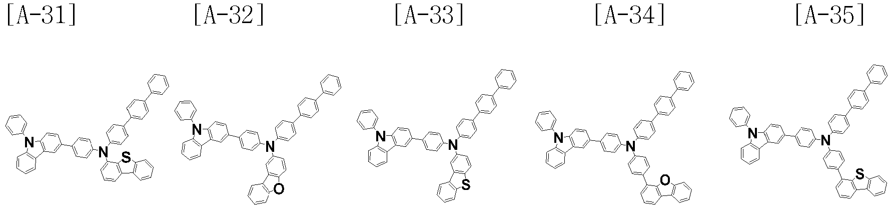

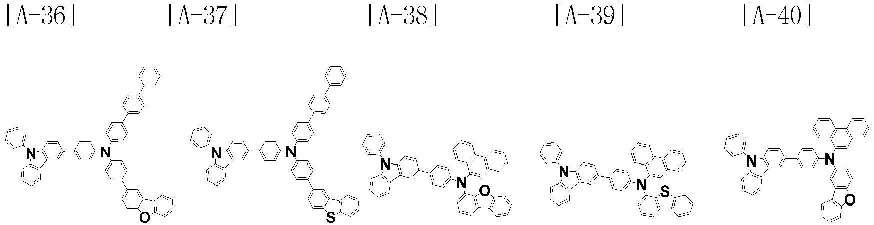

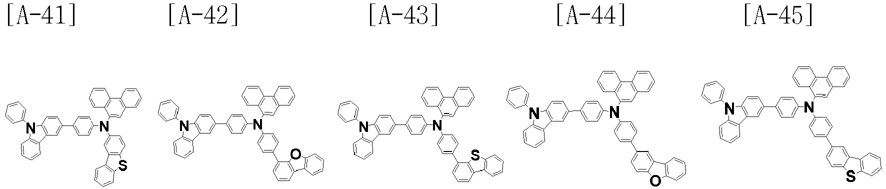

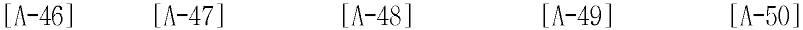

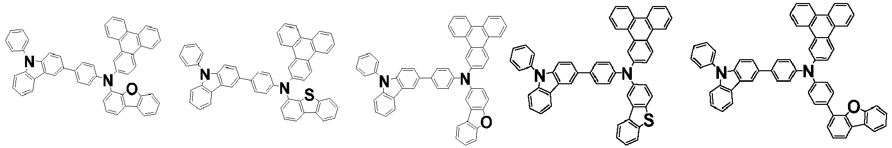

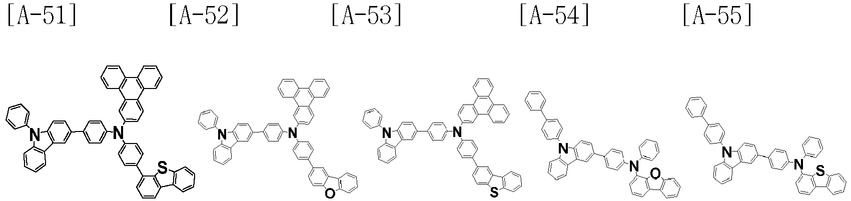

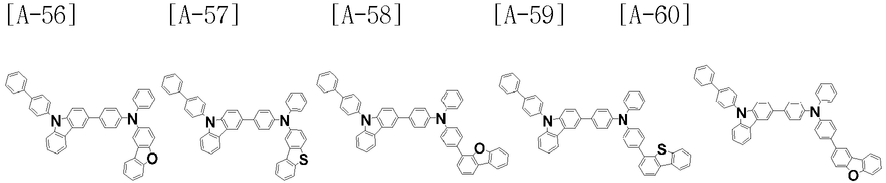

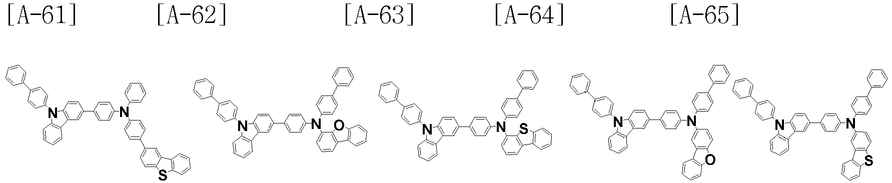

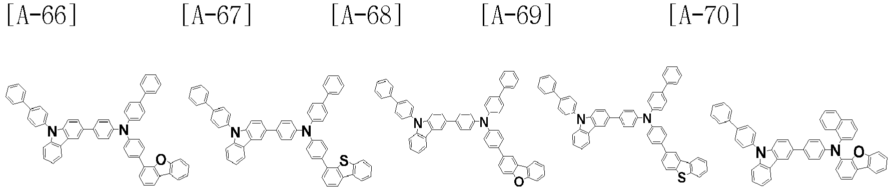

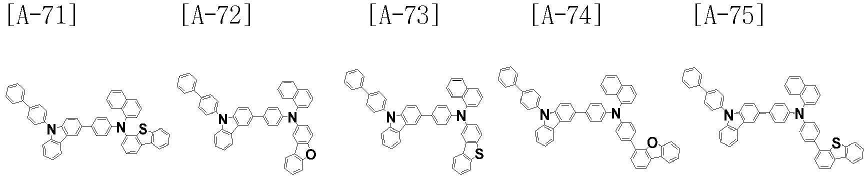

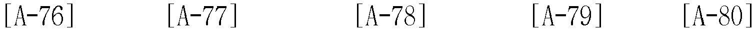

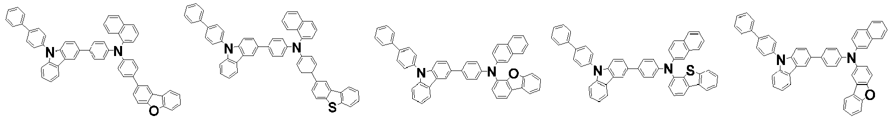

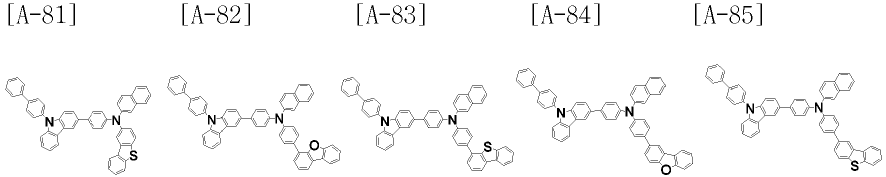

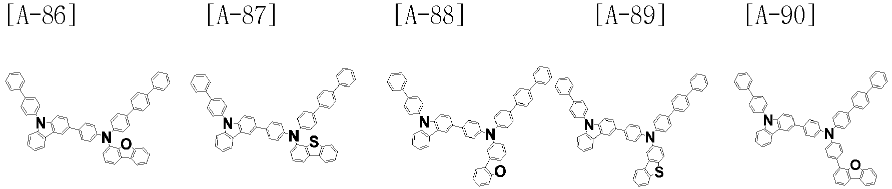

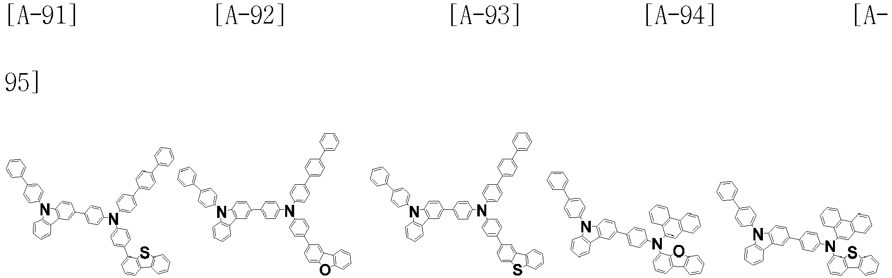

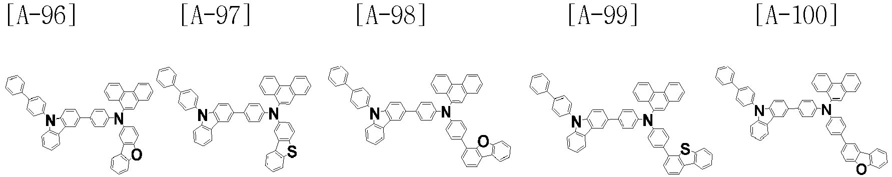

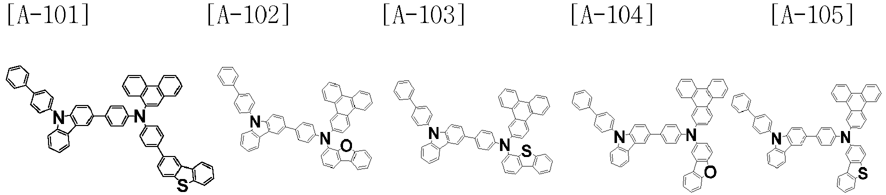

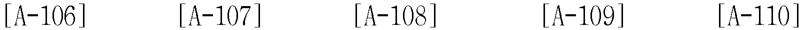

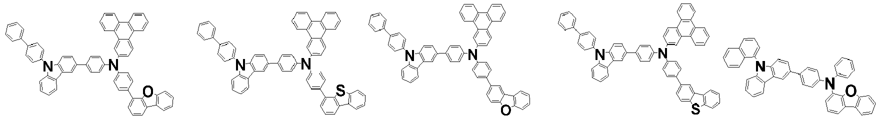

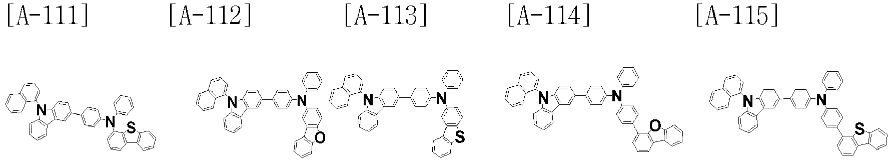

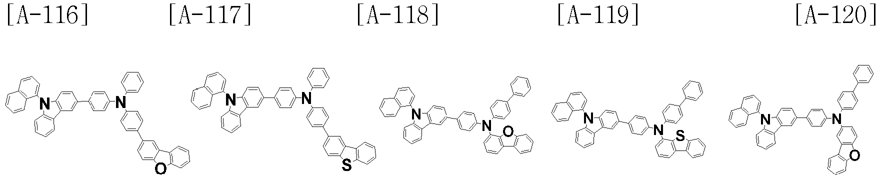

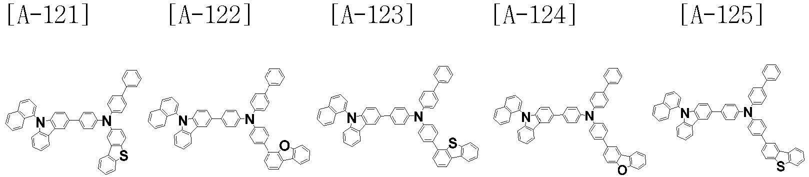

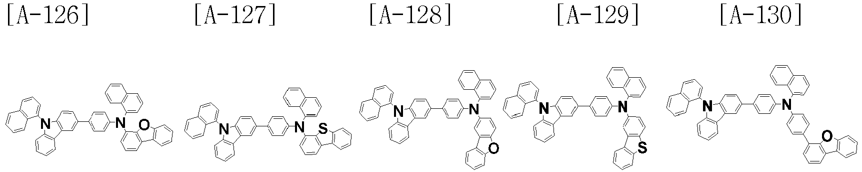

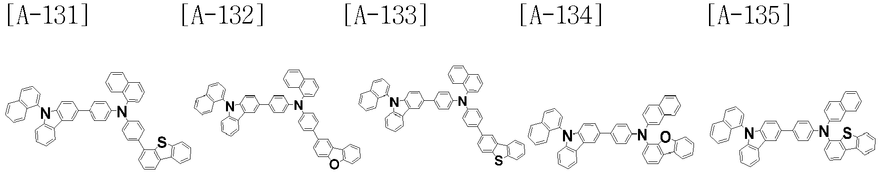

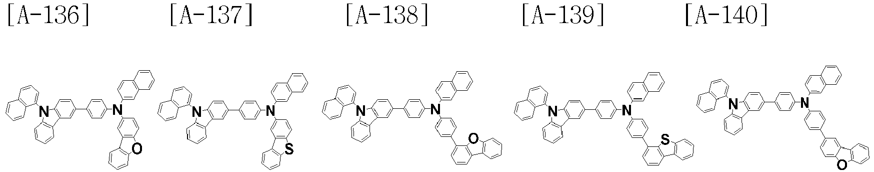

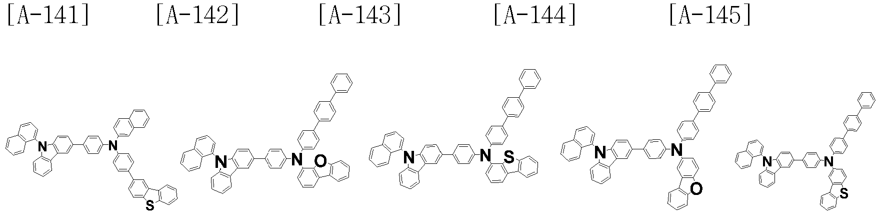

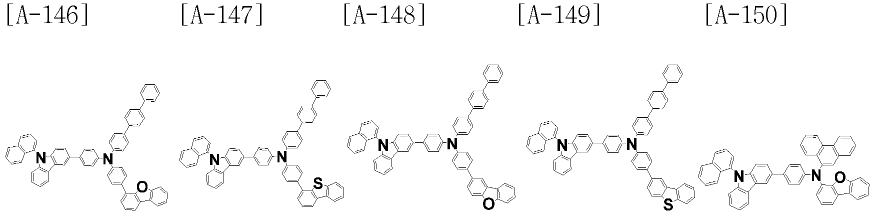

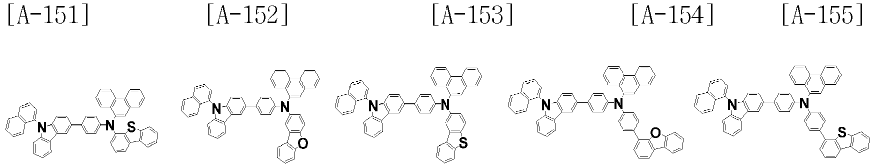

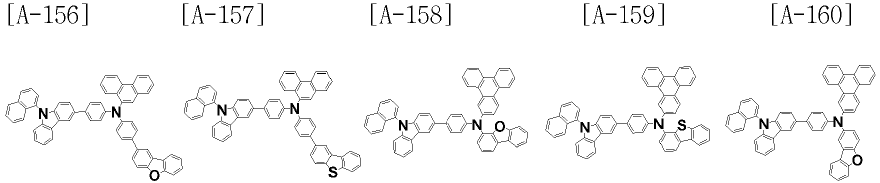

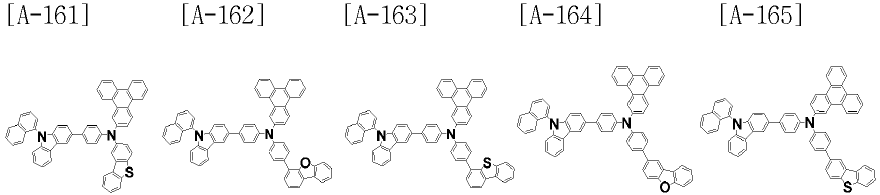

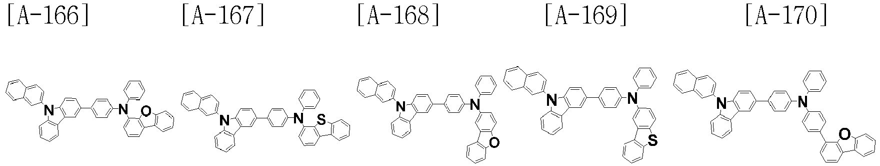

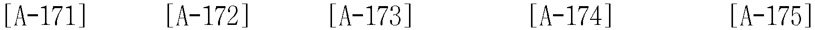

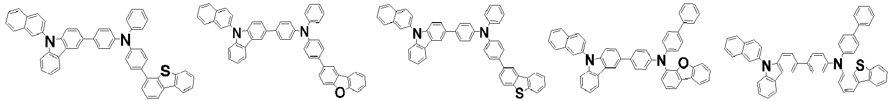

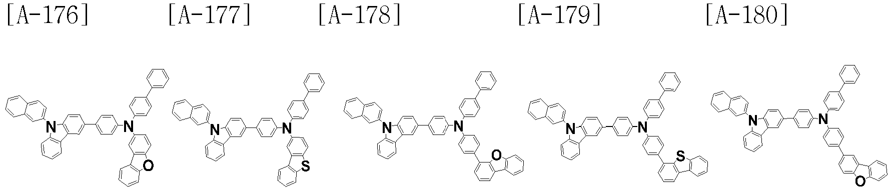

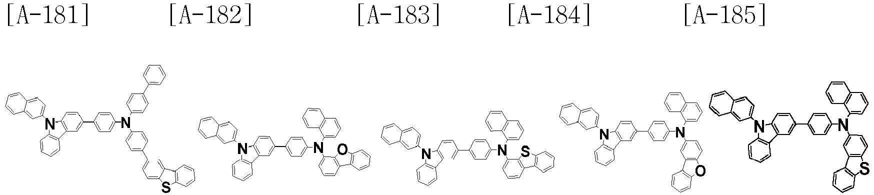

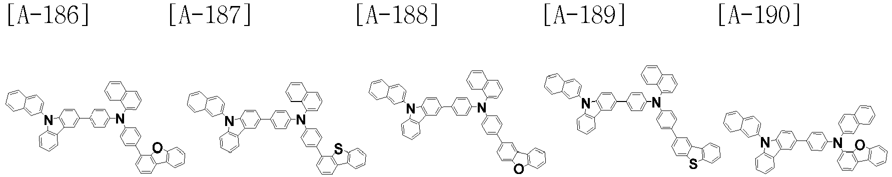

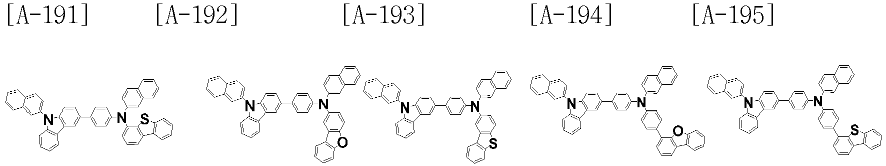

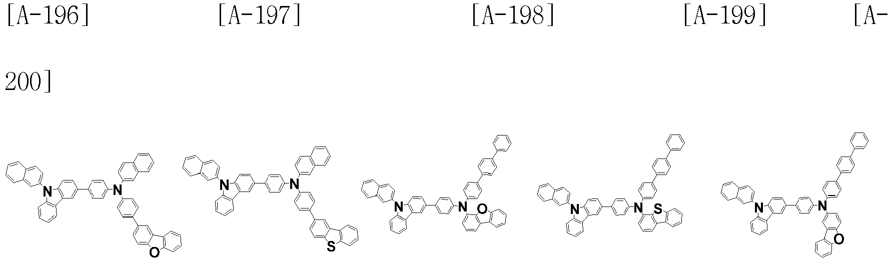

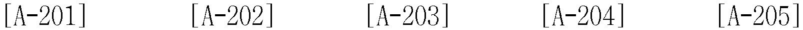

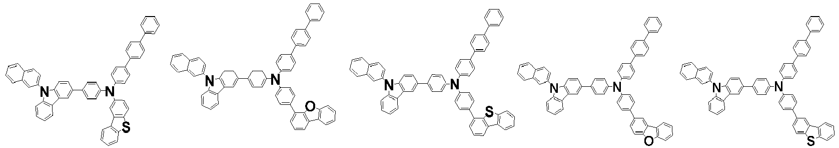

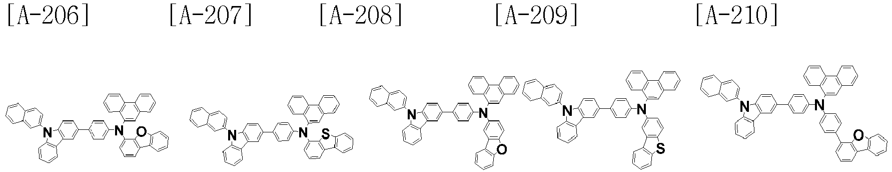

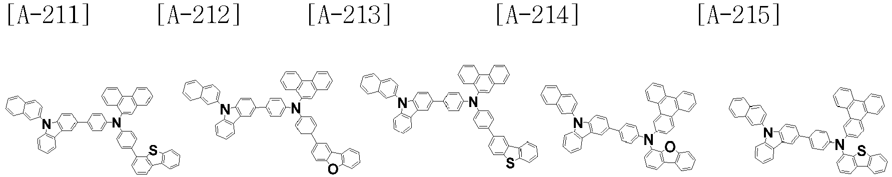

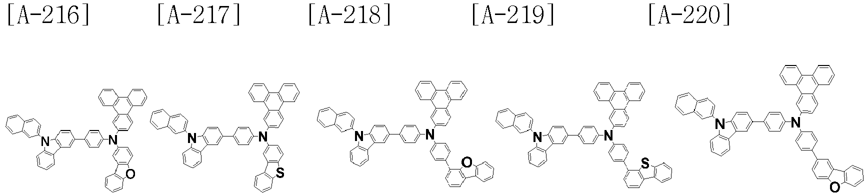

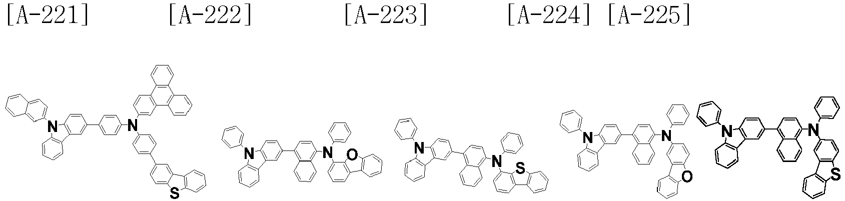

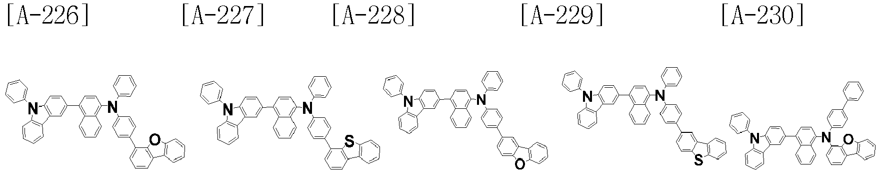

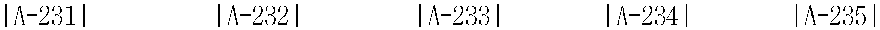

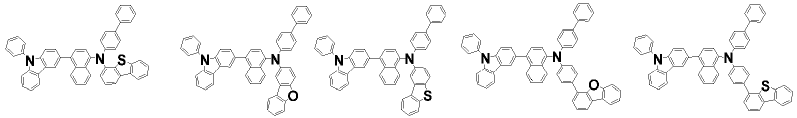

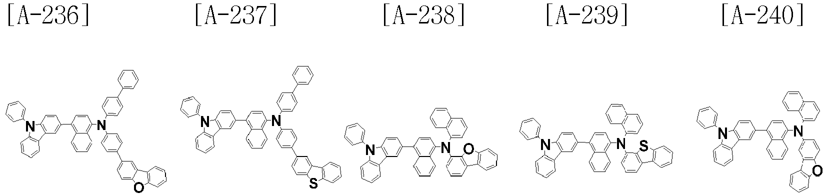

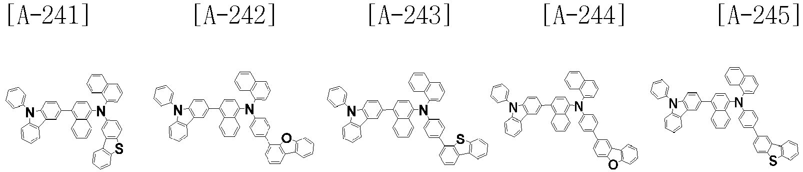

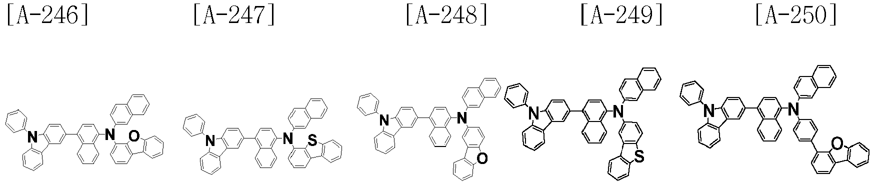

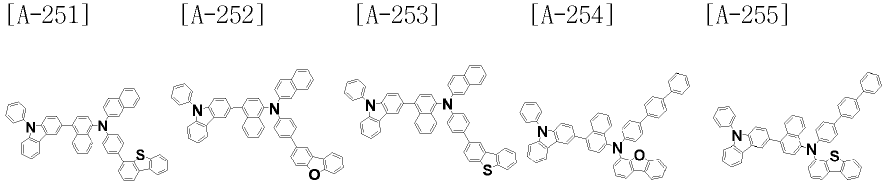

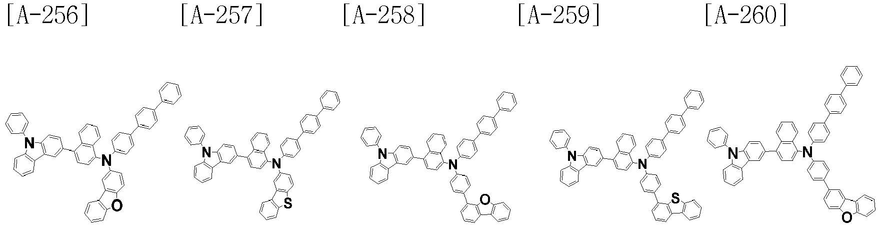

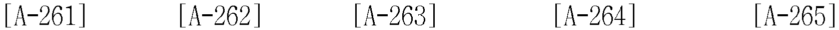

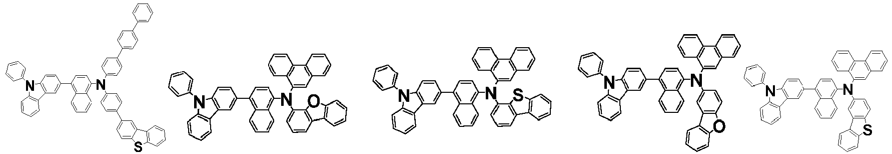

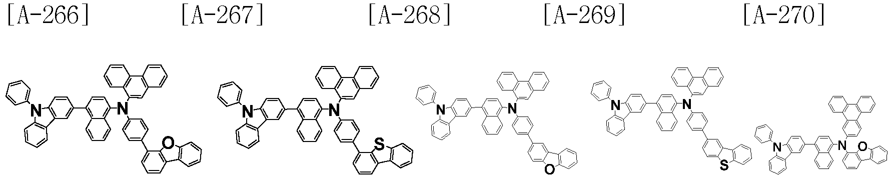

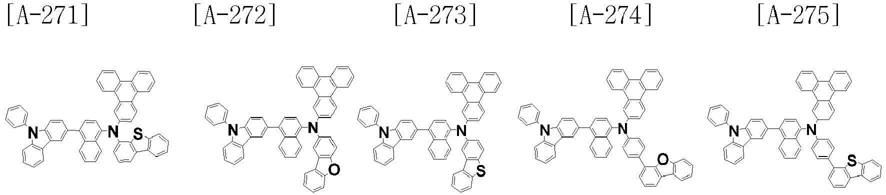

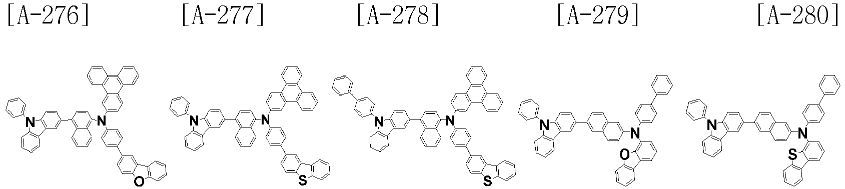

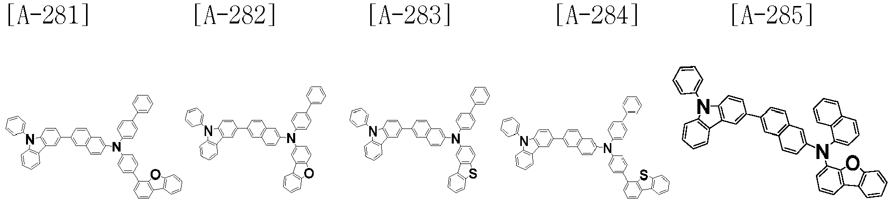

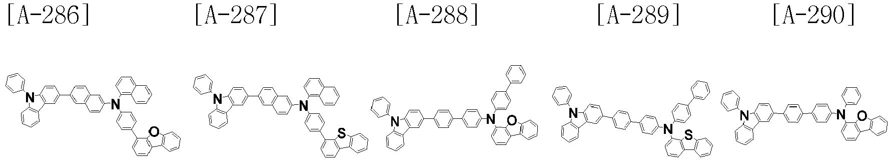

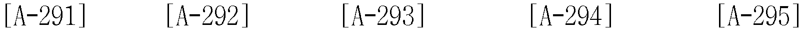

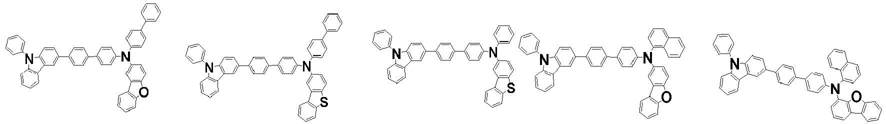

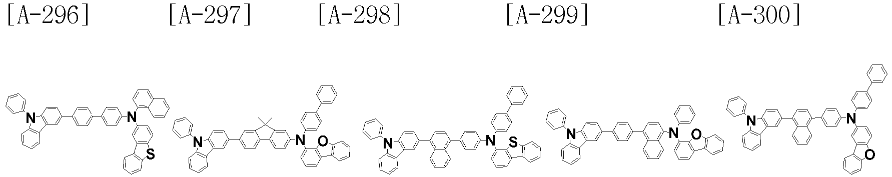

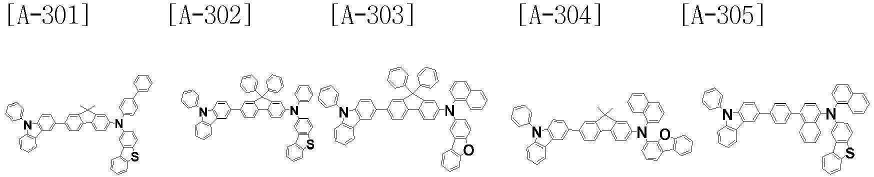

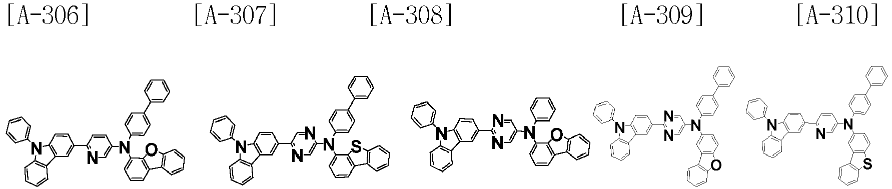

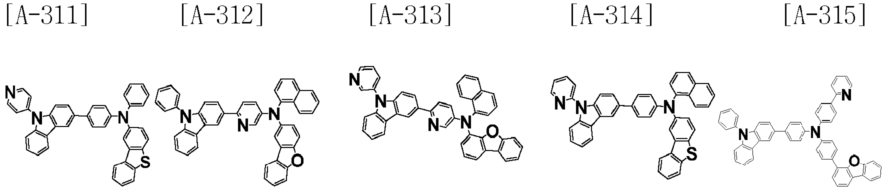

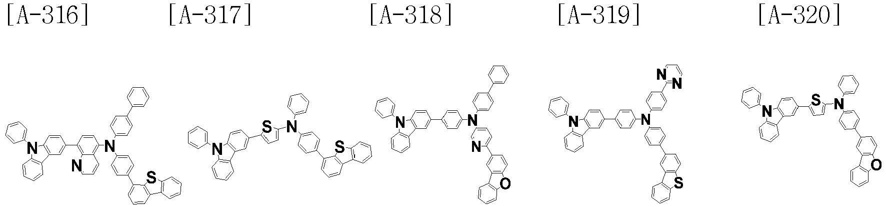

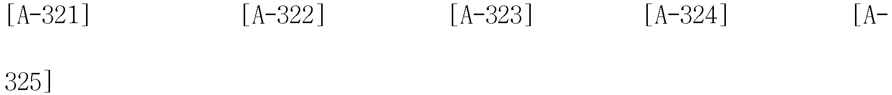

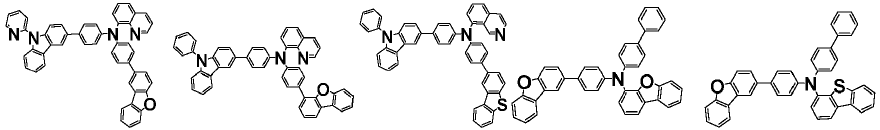

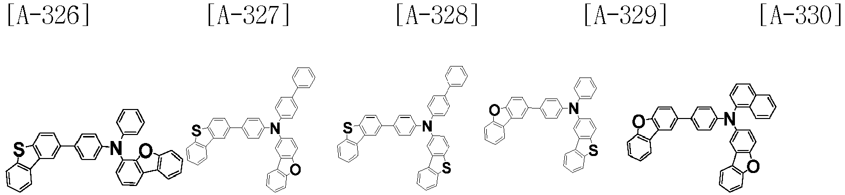

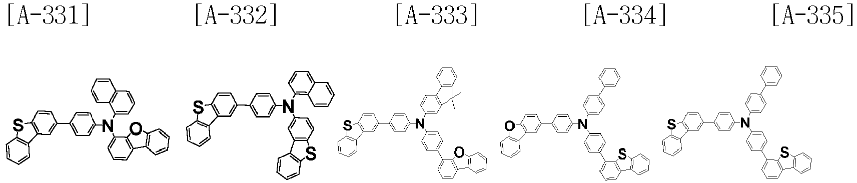

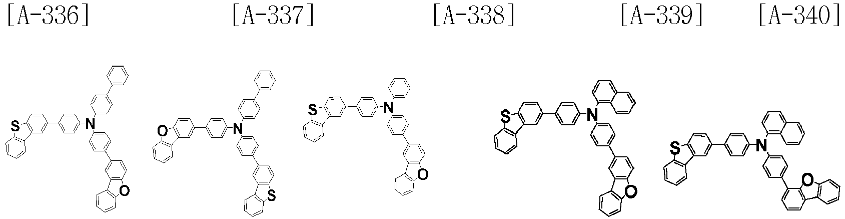

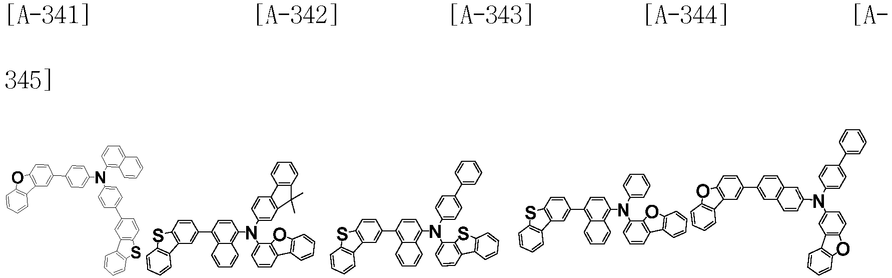

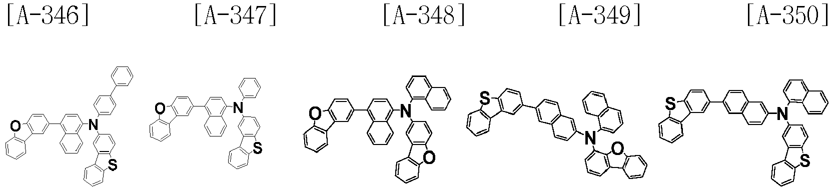

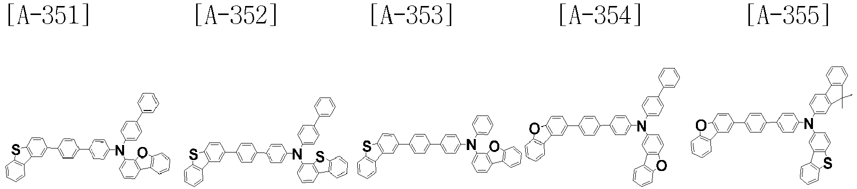

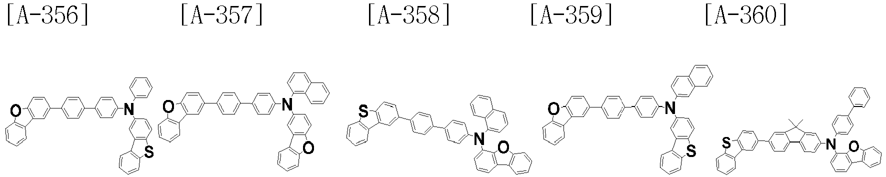

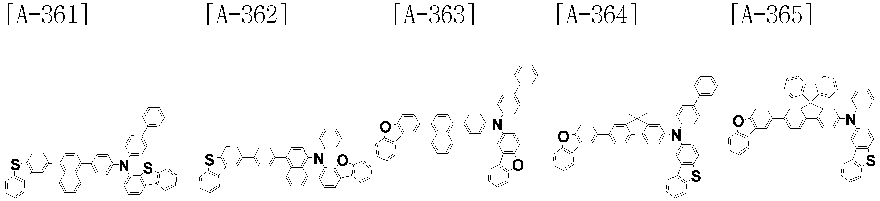

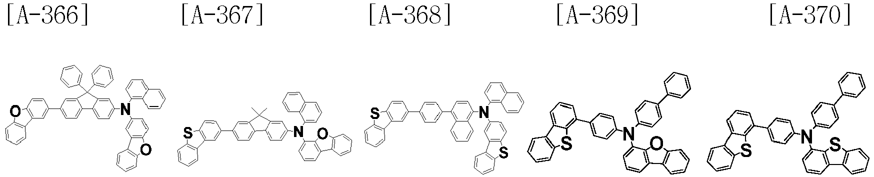

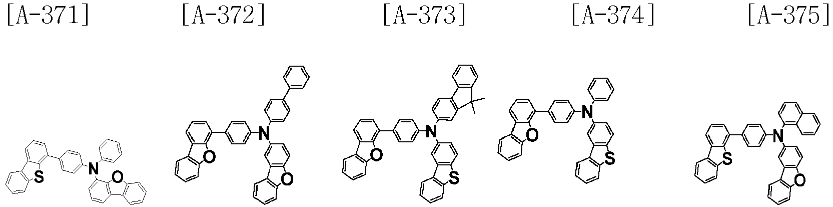

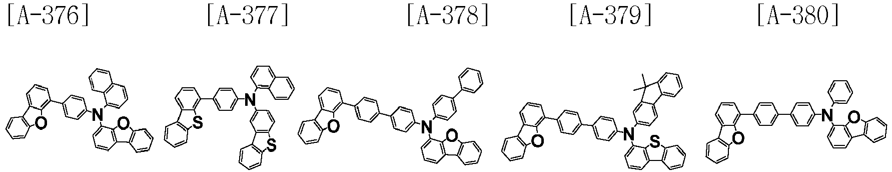

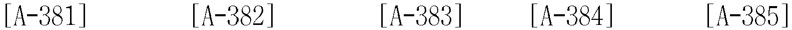

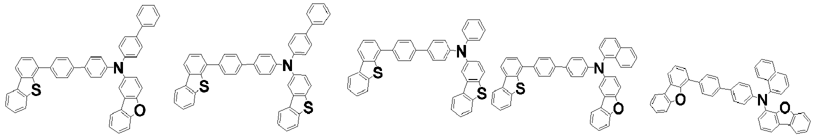

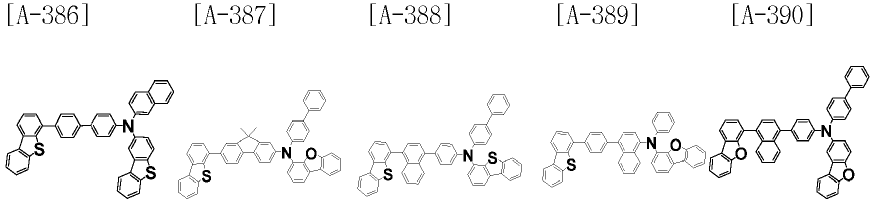

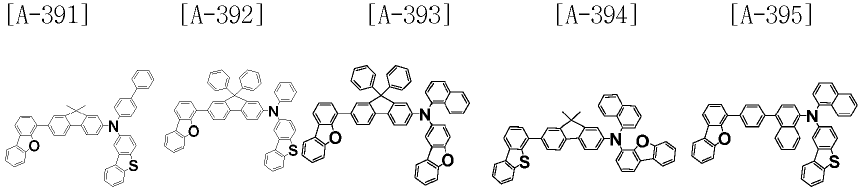

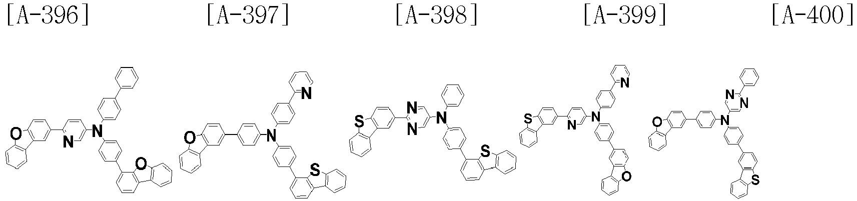

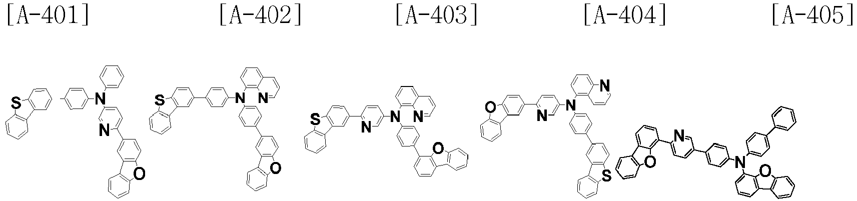

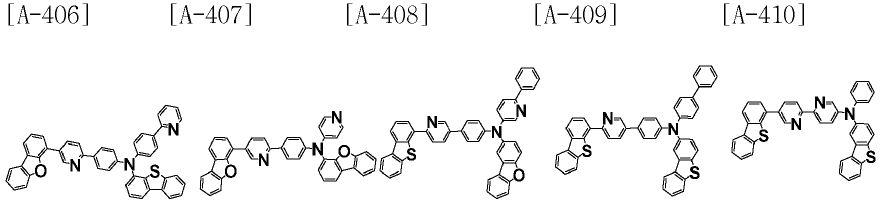

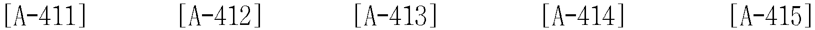

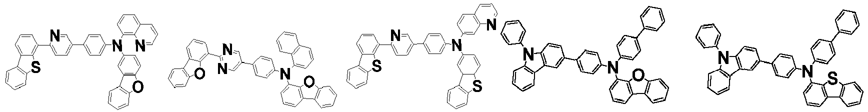

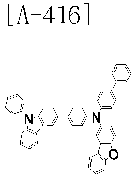

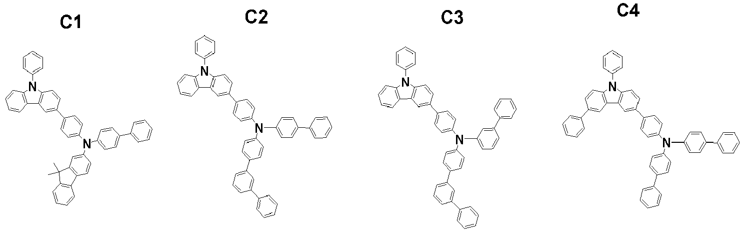

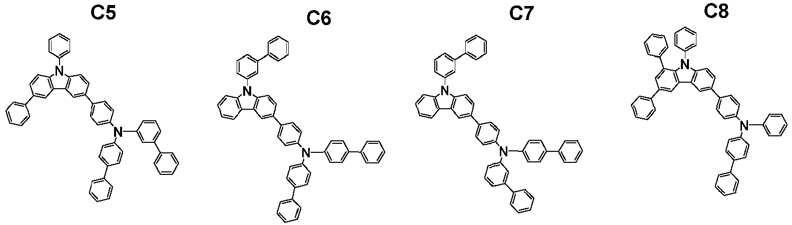

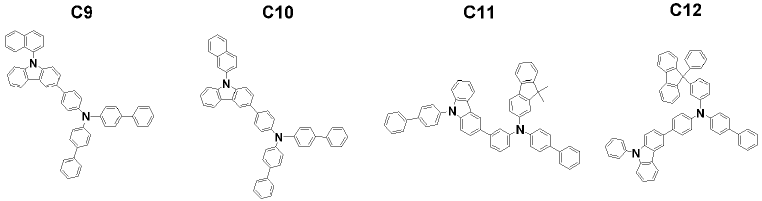

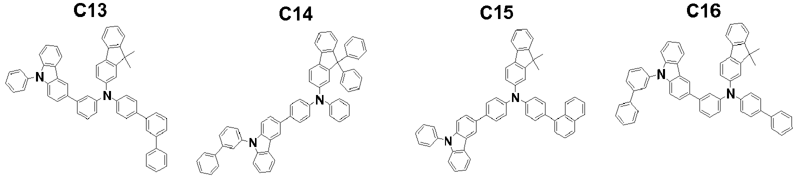

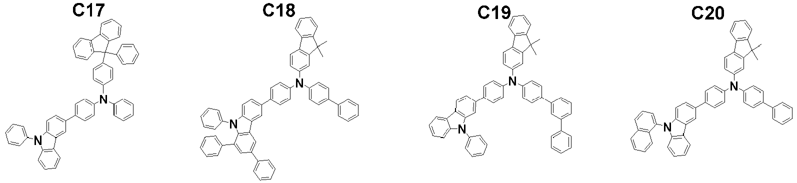

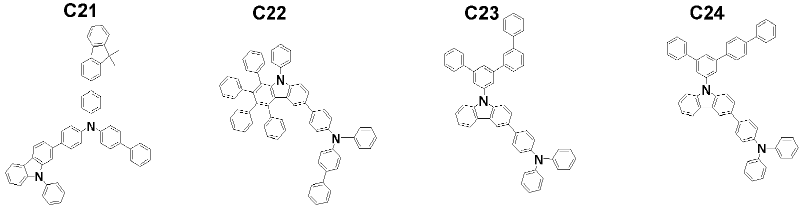

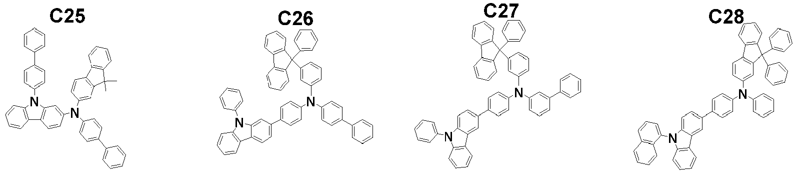

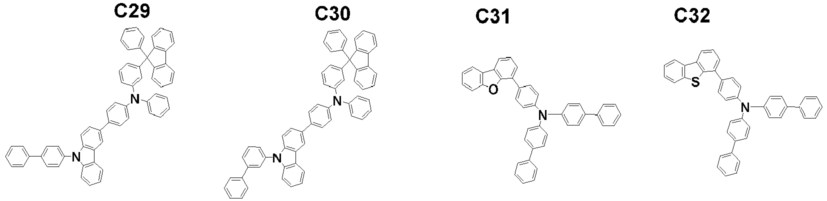

On the other hand, charge mobility may be adjusted by controlling a ratio between the second compound having hole characteristics and the first compound.

A HOMO energy level of the second compound may be -4.6 to -5.5 and a LUMO energy level thereof may be -1.7 to -0.850 eV.

In addition, the first compound and the second compound are included in a weight ratio of 1:9 to 9:1, specifically, 2:8 to 8:2, 3:7 to 7:3, 4:6 to 6:4, and 5:5. Within the ranges, bipolar characteristics may be further effectively realized, and thus efficiency and life-span may be simultaneously improved.

Specifically, in the light emitting layer 32, the first compound and the second compound may be simultaneously included as a host, for example the first compound may be represented by formula 1- Ia or formula 1-IVa and the second compound may be formula 2 wherein L² to L⁴ are independently a single bond, or a substituted or unsubstituted phenylene group, and Ar¹ to Ar³ are independently a substituted or unsubstituted C6 to C30 aryl group, or a substituted or unsubstituted C2 to C30 heterocyclic group, provided that at least one of Ar¹ to Ar³ is a substituted or unsubstituted C2 to C30 heterocyclic group.

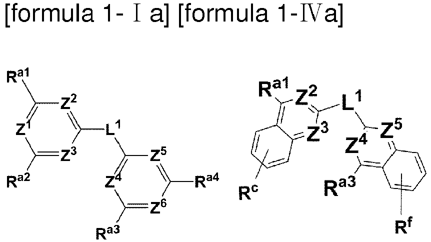

In formulas 1- Ia and 1-IVa, Z¹ to Z⁶ are independently N, or CR^{a}, at least two of Z¹ to Z³ are N, at least two of Z⁴ to Z⁶ are N, R^{a}, and R^{a1} to R^{a4} are independently hydrogen, or a substituted or unsubstituted C6 to C30 aryl group, and L¹ is a C6 to C30 arylene group that is unsubstituted or substituted with deuterium, a C1 to C30 alkyl group, or a C6 to C30 aryl group;
wherein, "substituted" is the same as described above.

More specifically, the first compound may be represented by formula 1-I a-1 or formula 1-IVa-1 and the second compound may be represented by formula 2 wherein Ar¹ to Ar³ are independently a substituted or unsubstituted phenyl group, a substituted or unsubstituted biphenyl group, a substituted or unsubstituted terphenyl group, a substituted or unsubstituted naphthyl group, a substituted or unsubstituted phenanthrenyl group, a substituted or unsubstituted carbazolyl group, a substituted or unsubstituted dibenzofuranyl group, or a substituted or unsubstituted dibenzothiophenyl group, provided that at least one of Ar¹ to Ar³ is a substituted or unsubstituted carbazolyl group, a substituted or unsubstituted dibenzofuranyl group, or a substituted or unsubstituted dibenzothiophenyl group.

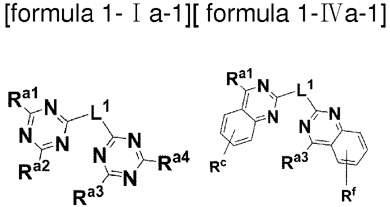

In formula 1- Ia-1 and formula 1-IVa-1, R^{a1} to R^{a4}, L¹, R^{c}, R^{f}, and "substituted" are the same as described above.

The light emitting layer 32 may further include at least one compound in addition to the first compound and the second compound as a host. For example, aryl amine compound or aryl amine carbazole compound having excellent hole characteristics may be further included.

The light emitting layer 32 may further include a dopant. The dopant is mixed with a host in a small amount to cause light emission, and may be generally a material such as a metal complex that emits light by multiple excitation into a triplet or more. The dopant may be, for example an inorganic, organic, or organic/inorganic compound, and one or more kinds thereof may be used.

The dopant may be a red, green, or blue dopant, for example phosphorescent dopant. Examples of the phosphorescent dopant may be an organic metal compound including Ir, Pt, Os, Ti, Zr, Hf, Eu, Tb, Tm, Fe, Co, Ni, Ru, Rh, Pd, or a combination thereof. The phosphorescent dopant may be, for example a compound represented by formula Z, but is not limited thereto.

[formula Z] L₂MX

In formula Z, M is a metal, and L and X are the same or different, and are a ligand to form a complex compound with M.

The M may be for example Ir, Pt, Os, Ti, Zr, Hf, Eu, Tb, Tm, Fe, Co, Ni, Ru, Rh, Pd or a combination thereof, and the L and X may be, for example a bidendate ligand.

The composition may be applied to an organic layer of an organic optoelectronic device, for example a light emitting layer. For example, the composition may be applied to a light emitting layer as a host.

The composition may be formed using a dry film formation method or a solution process. The dry film formation method may be, for example a chemical vapor deposition (CVD) method, sputtering, plasma plating, and ion plating, and two or more compounds may be simultaneously formed into a film or compound having the same deposition temperature may be mixed and formed into a film. The solution process may be, for example inkjet printing, spin coating, slit coating, bar coating and/or dip coating.

Hereinafter, an organic optoelectronic device including the composition is described.

The organic optoelectronic device may be any device to convert electrical energy into photoenergy and vice versa without particular limitation, and may be selected from an organic light emitting diode, an organic photoelectric device, an organic solar cell, an organic transistor, an organic photo conductor drum, and an organic memory device.

The organic optoelectronic device includes an anode and a cathode facing each other, and at least one organic layer interposed between the anode and the cathode, wherein the organic layer includes the composition.

Herein, an organic light emitting diode as one example of an organic optoelectronic device is described referring to drawings.

FIG. 1 is a schematic cross-sectional view of an organic light emitting diode according to an embodiment.

Referring to FIG. 1, an organic light emitting diode 100 according to an embodiment includes an anode 120 and a cathode 110 facing each other and an organic layer 105 between the anode 120 and the cathode 110.

The anode 120 may be made of a conductor having a large work function to help hole injection, and may be for example made of a metal, a metal oxide, and/or a conductive polymer. The anode 120 may be, for example a metal such as nickel, platinum, vanadium, chromium, copper, zinc, and gold or an alloy thereof; metal oxide such as zinc oxide, indium oxide, indium tin oxide (ITO), indium zinc oxide (IZO), and the like; a combination of metal and oxide such as ZnO and Al or SnO₂ and Sb; a conductive polymer such as poly(3-methylthiophene), poly(3,4-(ethylene-1,2-dioxy)thiophene) (PEDOT), polypyrrole, and polyaniline, but is not limited thereto.

The cathode 110 may be made of a conductor having a small work function to help electron injection, and may be for example made of a metal, a metal oxide and/or a conductive polymer. The cathode 110 may be for example a metal or an alloy thereof such as magnesium, calcium, sodium, potassium, titanium, indium, yttrium, lithium, gadolinium, aluminum silver, tin, lead, cesium, barium, and the like; a multi-layer structure material such as LiF/Al, LiO₂/Al, LiF/Ca, LiF/Al and BaF₂/Ca, but is not limited thereto.

The organic layer 105 includes a light emitting layer 130 including the composition.

FIG. 2 is a cross-sectional view showing an organic light emitting diode according to another embodiment.

Referring to FIG. 2, an organic light emitting diode 200 according to the present embodiment includes an anode 120 and a cathode 110 facing each other and an organic layer 105 between the anode 120 and the cathode 110 like the above embodiment.

The organic layer 105 includes a light emitting layer 130 and an auxiliary layer 140 between the light emitting layer 130 and the anode 120. The auxiliary layer 140 may help charge injection and transfer between the anode 120 and the light emitting layer 130. The auxiliary layer 140 may be, for example a hole transport layer (HTL), a hole injection layer (HIL), and/or an electron blocking layer, and may include at least one layer.

In FIGS. 1 and 2, at least one auxiliary layer between the cathode 110 and the light emitting layer 130 may be further included as an organic layer 105. The auxiliary layer may be, for example an electron transport layer (ETL), an electron injection layer (EIL), and/or an electron transport auxiliary layer.

The organic light emitting diode may be applied to an organic light emitting display device.

Hereinafter, the embodiments are illustrated in more detail with reference to examples. These examples, however, are not in any sense to be interpreted as limiting the scope of the invention.

### [Mode for Invention]

Hereinafter, a starting material and a reactant used in Synthesis Examples and Examples were purchased from Sigma-Aldrich Corporation or TCI Inc. unless there was particularly mentioned.

### Synthesis of First Compound

### Synthesis Example 1: Synthesis of Compound 1

### a) Synthesis of Intermediate 1-1

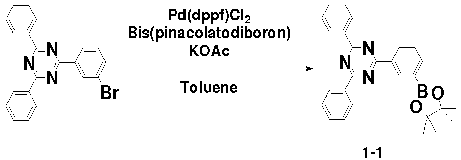

20 g (51.51 mmol) of 2-(3-bromophenyl)-4,6-diphenyl-1,3,5-triazine was dissolved in 250 mL of toluene in a 500 mL round-bottomed flask. Then, 0.05 equivalent of dichlorodiphenylphosphinoferrocene palladium, 1.2 equivalent of bis(pinacolato)diboron, and 2 equivalents of potassium acetate were added thereto, and the mixture was heated and refluxed under a nitrogen atmosphere for 18 hours. The reaction solution was cooled down, 100 mL of water was added thereto, and an organic layer was extracted therefrom. The organic layer was collected, treated with activated carbon, and filtered through silica gel, and the filtered solution was concentrated. The concentrated residue was collected and then, crystallized in 200 mL of toluene and 50 mL of acetone to obtain 19.1 g of Intermediate 1-1.

### b) Synthesis of Compound 1

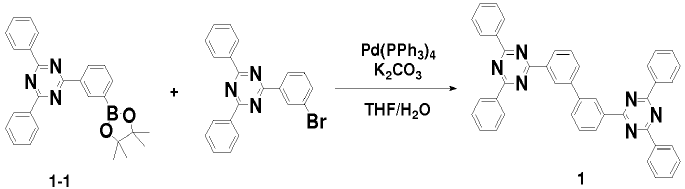

19 g (43.79 mmol) of the synthesized Intermediate 1-1 was added to 200 mL of tetrahydrofuran and 50 mL of distilled water in a 500 mL round-bottomed flask, 1 equivalent of 2-(3-bromophenyl)-4,6-diphenyl-1,3,5-triazine, 0.03 equivalent of tetrakistriphenylphosphine palladium, and 2 equivalents of potassium carbonate were added thereto, and the mixture was heated and refluxed under a nitrogen atmosphere. After 18 hours, the reaction solution was cooled down, and a solid precipitated therein was filtered and washed with 500 mL. The solid was recrystallized with 500 mL of monochlorobenzene to obtain 22.41 g of Compound 1.
LC/MS calculated for: C42H28N6 Exact Mass: 616.2375 found for: 617.24 [M+H]

### Synthesis Example 2: Synthesis of Compound 2

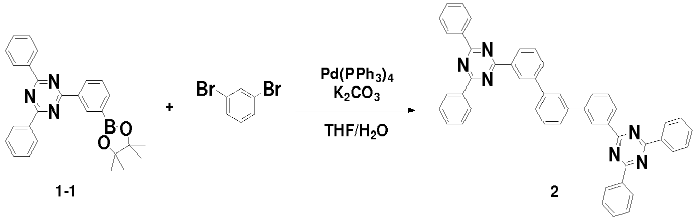

15 g (34.46 mmol) of the synthesized Intermediate 1-1, 0.5 equivalent of 1,3-dibromobenzene, 0.03 equivalent of tetrakistriphenylphosphine palladium, 2 equivalents of potassium carbonate, 200 mL of tetrahydrofuran, and 50 mL of distilled water were put in a 500 mL round-bottomed flask and then, heated and refluxed under a nitrogen atmosphere. After 20 hours, the reaction solution was cooled down, and a solid precipitated therein was filtered and washed with 500 mL of water. The solid was recrystallized with 400 mL of dichlorobenzene to obtain 9.2 g of Compound 2.
LC/MS calculated for: C48H32N6 Exact Mass: 692.2688 found for: 693.27 [M+H]

### Synthesis Example 3: Synthesis of Compound 3

### a) Synthesis of Intermediate 3-1

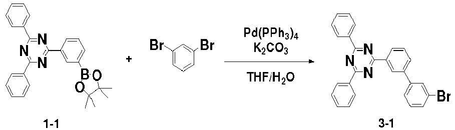

30 g (68.92 mmol) of the synthesized Intermediate 1-1, 1.2 equivalent of 1,3-dibromobenzene, 0.03 equivalent of tetrakistriphenylphosphine palladium, 2 equivalents of potassium carbonate, 300 mL of tetrahydrofuran, and 100 mL of distilled water were put in a 500 mL round-bottomed flask and then, heated and refluxed under a nitrogen atmosphere. After 18 hours, the reaction solution was cooled down, suspended in 1 L of methanol, stirred, filtered, and washed with 500 mL of water. Then, a solid therein was recrystallized with 400 mL of dichlorobenzene to obtain 32 g of Intermediate 3-1.

### b) Synthesis of Intermediate 3-2

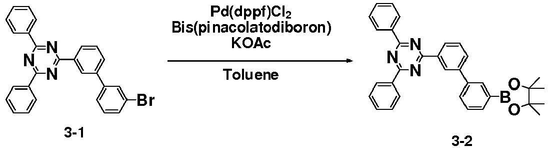

32 g (68.91 mmol) of the synthesized Intermediate 3-1 was reacted according to the same method as in a) the synthesis method of Intermediate 1-1 in Synthesis Example 1 in a 500 mL round-bottomed flask to obtain 29.96 g of Intermediate 3-2.

### c) Synthesis of Compound 3

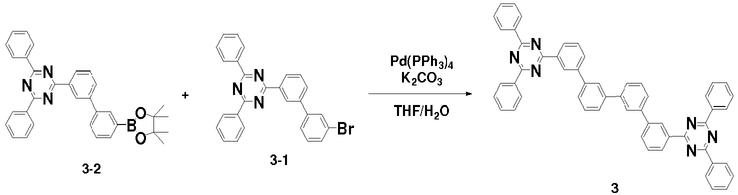

15 g (29.33 mmol) of the synthesized Intermediate 3-2, 1 equivalent of the synthesized Intermediate 3-1, 0.03 equivalent of tetrakistriphenylphosphine palladium, 2 equivalents of potassium carbonate, 200 mL of tetrahydrofuran, an 50 mL of distilled water were put in a 500 mL round-bottomed flask and then, heated and refluxed under a nitrogen atmosphere. After 18 hours, the reaction solution was cooled down, filtered, and washed with 500 mL of water. Then, a solid therein was recrystallized with 500 mL of dichlorobenzene to obtain 16.01 g of Compound 3.
LC/MS calculated for: C54H36N6 Exact Mass: 768.3001 found for: 769.3 [M+H]

### Synthesis Example 4: Synthesis of Compound 100

### a) Synthesis of Intermediate Intermediate 100-1

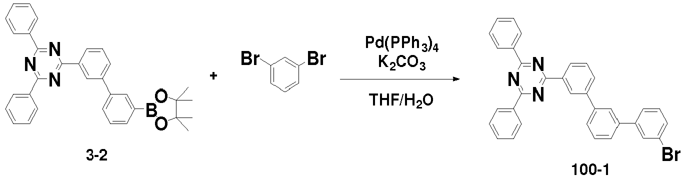

15 g (29.33 mmol) of the synthesized Intermediate 3-2 was reacted according to the same method as in a) the synthesis method of Intermediate 3-1 in Synthesis Example 3 in a 500 mL round-bottomed flask to obtain 12.84 g of Intermediate 100-1.

### b) Synthesis of Compound 100

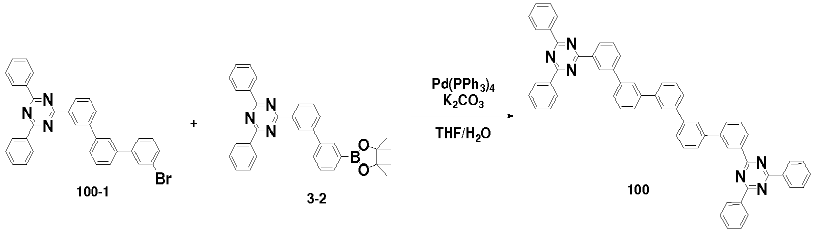

12 g (22.2 mmol) of the synthesized Intermediate 100-1, 1.2 equivalent of the synthesized Intermediate 3-2, 0.03 equivalent of tetrakistriphenylphosphine palladium, 2 equivalent of potassium carbonate, 150 mL of tetrahydrofuran, and 50 mL of distilled water were put in a 500 mL round-bottomed flask and then, heated and refluxed under a nitrogen atmosphere. After 18 hours, the reaction solution was cooled down, suspended in 1 L of methanol, stirred and filtered, and then, washed with 500 mL of water. Then, a solid therefrom is recrystallized with 500 mL of dichlorobenzene to obtain 13.3 g of Compound 100.
LC/MS calculated for: C60H40N6 Exact Mass: 844.3314 found for 845.34 [M+H]

### Synthesis Example 5: Synthesis of Compound 4

### a) Synthesis of Intermediate 4-2

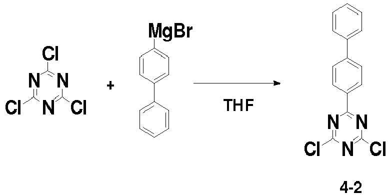

15 g (81.34 mmol) of cyaburic chloride was dissolved in 200 mL of anhydrous tetrahydrofuran in a 500 mL round-bottomed flask, 1 equivalent of a 4-biphenyl magnesium bromide solution (0.5 M tetrahydrofuran) was added thereto in a dropwise fashion under a nitrogen atmosphere at 0 ° C, and the mixture was slowly heated up to room temperature. Then, the reaction solution was stirred at room temperature for 1 hour, stirred, and then, poured into 500 mL of ice water to separate a layer. The separated organic layer was treated with anhydrous magnesium sulfate and concentrated. The concentrated residue was recrystallized with tetrahydrofuran and methanol to obtain 17.2 g of Intermediate 4-2.

### b) Synthesis of Intermediate 4-1

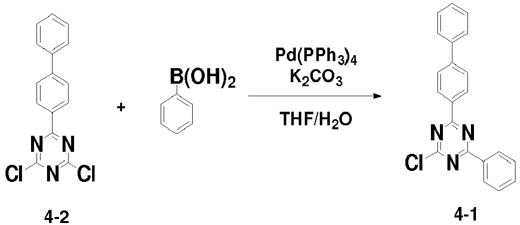

17 g (56.26 mmol) of the synthesized Intermediate 4-2 was put in a 500 mL round-bottomed flask, 1 equivalent of phenylboronic acid, 0.03 equivalent of tetrakistriphenylphosphine palladium, 2 equivalents of potassium carbonate, 150 mL of tetrahydrofuran, and 50 mL of distilled water were added thereto, and the mixture was heated and refluxed under a nitrogen atmosphere. After 18 hours, the reaction solution was cooled down, suspended in 1 L of methanol, stirred and filtered, washed with 500 mL of water, and dried to obtain 12.57 g of Intermediate 4-1.

### c) Synthesis of Compound 4

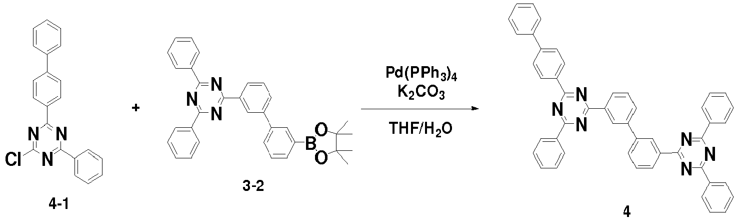

12 g (34.9 mmol) of the synthesized Intermediate 4-1 was put in a 500 mL round-bottomed flask, 1.1 equivalent of the synthesized Intermediate 3-2, 0.03 equivalent of tetrakistriphenylphosphine palladium, 2 equivalents of potassium carbonate, 150 mL of tetrahydrofuran, and 50 mL of distilled water were added thereto, and the mixture was heated and refluxed under a nitrogen atmosphere. After 18 hours, the reaction solution was cooled down, suspended in 1 L of methanol, stirred and filtered, and washed with 500 mL of water. Then, a solid produced therein was recrystallized with 500 mL of dichlorobenzene to obtain 17.8 g of Compound 4.
LC/MS calculated for: C48H32N6 Exact Mass: 692.2688 found for 692.27 [M+H]

### Synthesis Example 6: Synthesis of Compound 16

### a) Synthesis of Intermediate 16-2

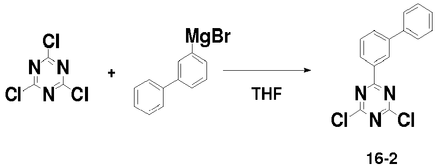

Intermediate 16-2 was synthesized according to the same method as a) the synthesis of Intermediate 4-2 of Synthesis Example 5 by using a 3-biphenyl magnesium bromide solution (0.5 M tetrahydrofuran) instead of the 4-biphenyl magnesium bromide solution (0.5 M tetrahydrofuran).

### b) Synthesis of Intermediate 16-1

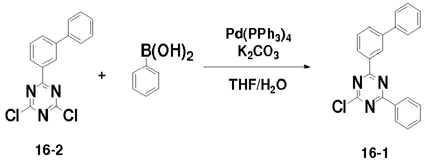

Intermediate 16-1 was synthesized according to the same method as b) the method of Synthesis Example 5 by using Intermediate 16-2 instead of Intermediate 4-2.

### c) Synthesis of Compound 16

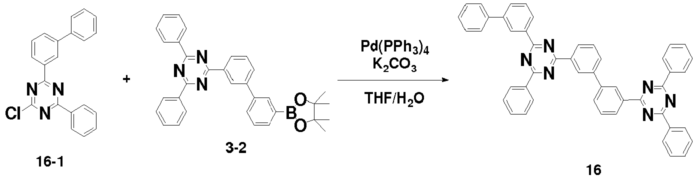

Compound 16 was synthesized according to the same method as c) the method of Synthesis Example 5.
LC/MS calculated for: C48H32N6 Exact Mass: 692.2688 found for 692.27 [M+H]

### Synthesis Example 7: Synthesis of Compound 126

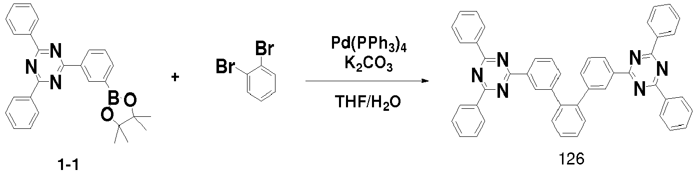

Compound 126 was synthesized according to the same method as Synthesis Example 2 by using 1,2-dibromobenzene as an intermediate.
LC/MS calculated for: C48H32N6 Exact Mass: 692.2688, found for 693.28 [M+H]

### Synthesis Example 8: Synthesis of Compound 140

### a) Synthesis of Intermediate 140-1

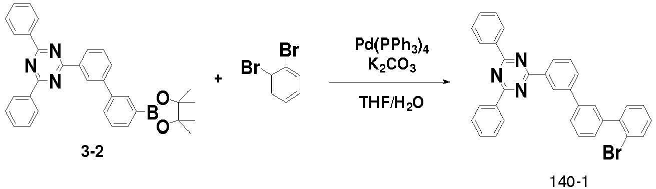

20 g (39.11 mmol) of Intermediate 3-2 was put in a 500 mL round-bottomed flask, 1.1 equivalent of 1,2-dibromobenzene, 0.03 equivalent of tetrakistriphenylphosphine palladium, 2 equivalents of potassium carbonate, 200 mL of tetrahydrofuran, and 50 mL of distilled water were added thereto, and the mixture was heated and refluxed under a nitrogen atmosphere. After 18 hours, the reaction solution was cooled down, suspended in 500 mL of methanol, stirred and filtered, and washed with 500 mL of water. Then, a solid produced therein was recrystallized with 500 mL of monochlorobenzene to obtain 18.4 g of Intermediate 140-1.

### b) Synthesis of Compound 140

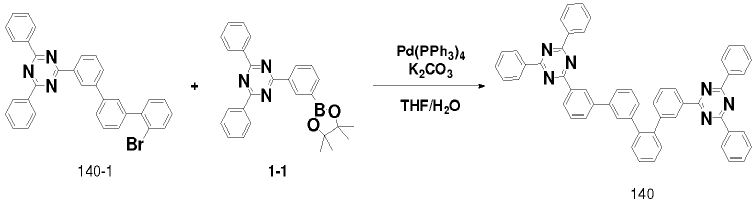

18 g (33.31 mmol) of the synthesized Intermediate 140-1 was put in a 500 mL round-bottomed flask, 1.1 equivalent of the synthesized Intermediate 1-1, 0.03 equivalent of tetrakistriphenylphosphine palladium, 2 equivalents of potassium carbonate, 200 mL of tetrahydrofuran, and 50 mL of distilled water were added thereto, and the mixture was heated and refluxed under a nitrogen atmosphere. After 18 hours, the reaction solution was cooled down, suspended in 500 mL of methanol, stirred and filtered, and washed with 500 mL of water. Then, a solid obtained therein was collected, silica gel column purified with a mixed solvent of normal hexane and ethyl acetate to obtain 19.2 g of Compound 140.
LC/MS calculated for: C54H36N6 Exact Mass: 768.3001, found for 769.3 [M+H]

### Synthesis Example 9: Synthesis of Compound 113

### a) Synthesis of Intermediate 113-3

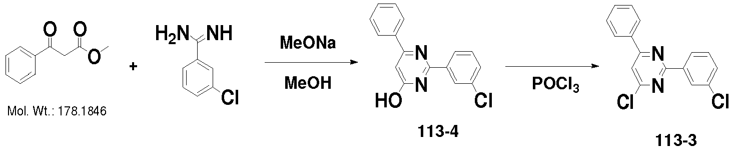

30 g (168.37 mmol) of methyl benzoyl acetate was put in a 500 mL round-bottomed flask, 1.1 equivalent of 3-chlorophenyl amidine, 1.2 equivalent of sodium methoxide, and 200 mL of methanol were added thereto, and the mixture heated and refluxed for 6 hours. The reaction solution was cooled down, a 1 N hydrochloric acid solution was added thereto, and chloridemethane was used for an extraction. The extracted solution was concentrated to obtain Intermediate 113-4, which itself is used for the following reaction.

Intermediate 113-4 was put in a 500 mL round-bottomed flask, 250 mL of phosphorylchloride was added thereto, and the mixture was heated and refluxed for 5 hours. The reaction solution was cooled down and slowly added to 1 L of ice water, and the mixture is stirred. Then, an aqueous layer is extracted with 500 mL of methane chloride, dried with anhydrous magnesium sulfate, and concentrated. The concentrated residue was purified through a silica gel column by using normal hexane and ethyl acetate to obtain 39 g of Intermediate 113-3.

### b) Synthesis of Intermediate 113-2

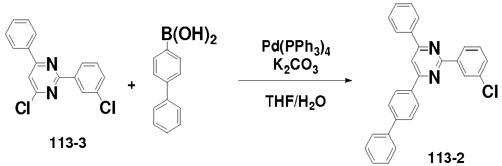

30 g (99.6 mmol) of the synthesized Intermediate 113-3 was put in a 500 mL round-bottomed flask, 1.2 equivalent of biphenyl boronic acid, 0.03 equivalent of tetrakistriphenylphosphine palladium, 2 equivalents of potassium carbonate, 250 mL of tetrahydrofuran, and 70 mL of distilled water were added thereto, and the mixture was heated and refluxed under a nitrogen atmosphere. After 18 hours, the reaction solution was cooled down, suspended in 500 mL of methanol, stirred and filtered, and washed with 500 mL of water. Then, a solid produced therein was collected and then, heated and recrystallized with 500 mL of toluene to obtain 35.9 g of Intermediate 113-2.

### c) Synthesis of Intermediate 113-1

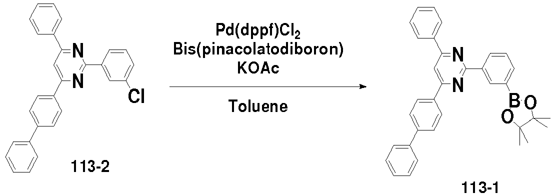

35 g (83.5 mmol) of the synthesized Intermediate 113-2 was put in a 500 mL round-bottomed flask, 250 mL of toluene, 0.05 equivalent of dichlorodiphenylphosphinoferrocene 1.2 equivalent of bis(pinacolato)diboron palladium, and 2 equivalents of potassium acetate were added thereto, and the mixture was heated and refluxed for 18 hours under a nitrogen atmosphere. The reaction solution was cooled down, and 100 mL of water was added thereto to extract an organic layer. The organic layer was collected, treated with activated carbon, filtered through silica gel, and concentrated. The concentrated residue was collected, heated and dissolved in 1 L of toluene, treated with activated carbon, filtered through silica gel. The filtered solution was cooled down and stirred to precipitate a solid. The precipitated solid was filtered to obtain 35.8 g of Intermediate 113-1.

### d) Synthesis of Compound 113

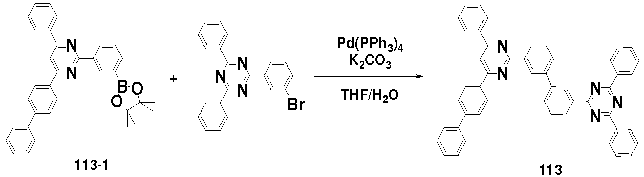

35 g (68.6 mmol) of the synthesized Intermediate 113-1, 1 equivalent of 2-(3-bromophenyl)-4,6-diphenyl-1,3,5-triazine, 0.03 equivalent of tetrakistriphenylphosphine palladium, 2 equivalents of potassium carbonate, 250 mL of tetrahydrofuran, and 70 mL of distilled water were put in a 500 mL round-bottomed flask and then, heated and refluxed under a nitrogen atmosphere. After 16 hours, the reaction solution was cooled down, suspended in 500 mL of methanol, stirred and filtered, and washed with 500 mL of water. Then, a solid produced therein was collected and then, heated and recrystallized with 500 mL of dichlorobenzene to obtain 32 g of Compound 113.
LC/MS calculated for: C49H33N5 Exact Mass: 691.2736, found for: 692.29 [M+H]

### Synthesis Example Ad-1: Synthesis of Compound 181

### a) Synthesis of Intermediate g-3

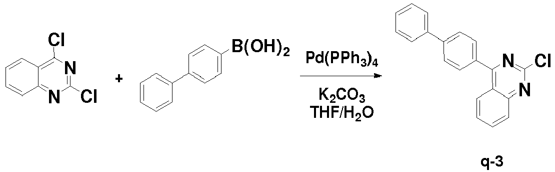

1 equivalent of 2,4-dichloro quinazoline, 1.1 equivalents of 4-biphenyl boronic acid, 0.03 equivalents of tetrakistriphenyl phosphine, and 3 equivalents of potassium carbonate were heated and refluxed in a solution of tetrahydrofuran and water (3:1) in a round-bottomed flask to be 0.25 M for 19 hours. The reaction solution was cooled down to room temperature and an organic layer was separated and concentrated. The concentrated residue was recrystallized with a mixed solution of normal hexane and dichloromethane to obtain 15 g of Intermediate q-3 (yield 75%).
LC-Mass (theoretical value: 316.08g/mol, measured value: M+1 = 317g/mol)

### b) Synthesis of Intermediate g-2

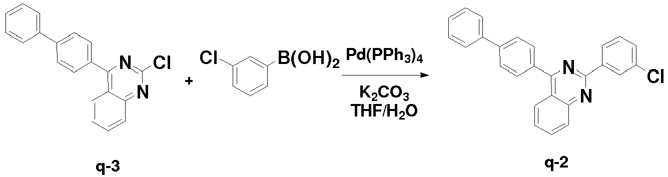

1 equivalent of the synthesized Intermediate q-3, 1.2 equivalents of 2-chlorophenyl boronic acid, 0.03 equivalent of tetrakistriphenyl phosphine, and 3 equivalents of potassium carbonate were heated and refluxed in a solution of tetrahydrofuran and water (3:1) to be 0.25 M for 18 hours. The reaction solution was cooled down to room temperature, was diluted in methanol to be 0.1 M, and then was stirred to precipitate a solid. The produced solid was filtered and recrystallized with ethyl acetate to obtain Intermediate q-2 21g (yield 89%).
LC-Mass (theoretical value: 316.08g/mol, measured value: M+1 = 317g/mol)

### c) Synthesis of Intermediate q-1

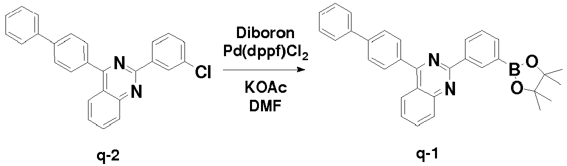

15 g of Intermediate q-1 was obtained according to the same method as the synthesis method of Intermediate 1-1 or Intermediate 3-2 except for using 1 equivalent of the synthesized Intermediate q-2 (yield 75%).
LC-Mass (theoretical value: 484.23g/mol, measured value: M+1 = 485. 27g/mol)

### d) Synthesis of Compound 181

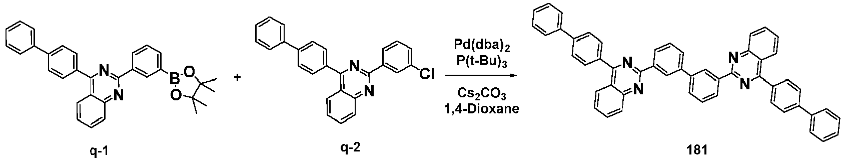

1 equivalent of the synthesized Intermediate q-1 and 1 equivalent of Intermediate q-2 were put in a round flask, 0.03 equivalent of bisdibenzylidine palladium, 0.06 equivalent of tristertiary butyl phosphine, and 2 equivalents of cecium carbonate were suspended in a 1,4-dioxane solvent to be 0.25 M, and and the mixture was heated and refluxed under a nitrogen atmosphere for 20 hours. The solid produced during the reaction was filtered and washed with water and dried. The dried solid was heated and recrystallized in 0.1 M of dichlorobenzene to obtain Compound 181 11g (yield 72%)
LC-Mass (theoretical value: 714.28g/mol, measured value: M+1 = 715.31 g/mol)

### Synthesis Example Ad-2: Synthesis of Compound 180

Compound 180 was synthesized using "phenyl boronic acid" instead of "4-biphenyl boronic acid" in a) the synthesis of Intermediate q-3 of Synthesis Example Ad-1 and using the synthesis method of Synthesis Example Ad-1.
LC-Mass (theoretical value: 638.76g/mol, measured value: M+1 = 639.80g/mol)

### Synthesis Example Ad-3: Synthesis of Compound 183

Compound 183 was synthesized using "phenyl boronic acid" instead of "4-biphenyl boronic acid" in a) the synthesis of Intermediate q-3 of Synthesis Example Ad-1 and using the synthesis method of Synthesis Example Ad-1.
LC-Mass (theoretical value: 562.66g/mol, measured value: M+1 = 563.69g/mol)

### Synthesis Example Ad-4: Synthesis of Compound 190

Compound 190 was synthesized using "phenyl boronic acid" instead of "4-biphenyl boronic acid" in a) the synthesis of Intermediate q-3 of Synthesis Example Ad-1, using "2-chloro-2'-biphenyl boronic acid" instead of "2-chlorophenyl boronic acid" in b) step, and using the synthesis method of Synthesis Example Ad-1.
LC-Mass (theoretical value: 638.76g/mol, measured value: M+1 = 639.77g/mol)

### Synthesis of Second Compound

### Synthesis of Intermediate M-1

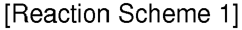

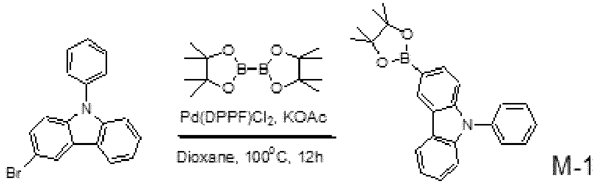

50 g (155.18 mmol) of 3-bromo-9-phenyl-9H-carbazole, 3.41 g (4.65 mmol) of Pd(dppf)Cl₂, 51.32g (201.8 mmol) of bis(pinacolato)diboron, and 45.8 g (465.5 mmol) of potassium acetate were dissolved in 520 ml of 1,4-dioxane. The reactant was refluxed and stirred for 12 hours under a nitrogen atmosphere and then extracted for 3 times with dichloromethane and distilled water. The extracted solution was dried with magnesium sulfite and filtered, and the filtrate was concentrated under a reduced pressure. The product was purified with n-hexane/dichloromethane (7:3 volume ratio) through a silica gel column chromatography to obtain 43 g (yield 75%) of white solid Intermediate M-1 as a target compound.
LC-Mass (theoretical value: 369.19g/mol, measured value: M+1 = 370g/mol)

### Synthesis of Intermediate M-2

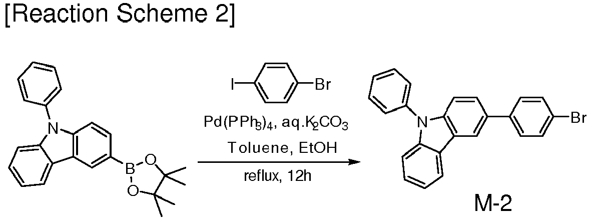

40 g (108.3 mmol) of Intermediate M-1, 30.6g (108.3 mmol) of 1-bromo-4-iodobenzene and 1.25 g (1.08 mmol) of tetrakistriphenylphosphine palladium were added in a flask and dissolved in 270 ml of toluene and 135 ml of ethanol under a nitrogen atmosphere.

Then, 135 ml of an aqueous solution including 31.9 g (58.9 mmol) of potassium carbonate was added into the reactants and then refluxed and stirred for 12 hours. After the reaction, the reactants were extracted with ethylacetate, the extract was dried with magnesium sulfite and filtered, and then, the filtrate was concentrated under reduced pressure. The product was purified with n-hexane/dichloromethane (7:3 volume ratio) through a silica gel column chromatography and then 35g of white solid Intermediate M-2 was obtained as a target compound (yield 81%).
LC-Mass (theoretical value: 398.29g/mol, measured value: M+1 = 399g/mol)

### Synthesis of Intermediate M-3

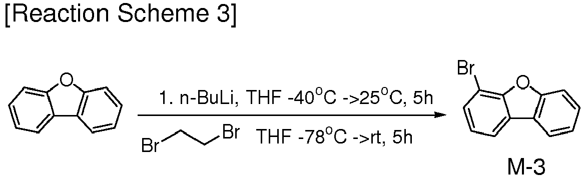

10 g (59.5 mmol) of a dibenzofuran was added in a two neck round-bottomed flask that was dried under vacuum and 119 mL of anhydrous tetrahydrofuran was added under a nitrogen atmosphere followed by dissolving, and then, being cooling down to -40 °C and stirred.

Then, 26 mL of 2.5 M n-butyl lithium (in hexane, 65.5 mmol) was slowly added thereto and the resultant was stirred for 5 hours under room temperature under a nitrogen atmosphere. The reaction solution was cooled down to - 78° C and 22.4 g (119 mmol) of 1,2-dibromoethane dissolved in 10mL anhydrous tetrahydrofuran was slowly added and then stirred for 5 hours at room temperature.

After the reaction is completed, the solution was concentrated under a reduced pressure to remove the solvent and was extracted with distilled water and dichloromethane, the extract solution was dried with magnesium sulfite and filtered, and the filtrate was concentrated under a reduced pressure. The reaction solution was recrystallized in n-hexane and then 11 g of white solid Intermediate M-3 was obtained as a target compound (yield 75%).
GC-Mass (theoretical value: 245.97g/mol, measured value: 246g/mol)

### Synthesis of Intermediate M-4

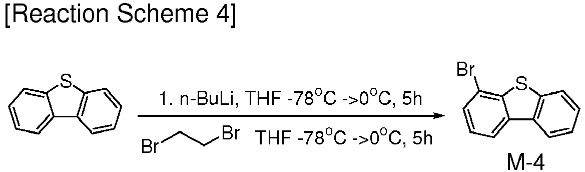

11 g of white solid Intermediate M-4 as a target compound was obtained using 10 g (54.3 mmol) of dibenzothiophene instead of the dibenzofuran in the synthesis method of Intermediate M-3 in [Reaction Scheme 4] (yield 77%).
GC-Mass (theoretical value: 261.95g/mol, measured value: 262g/mol)

### Synthesis of Intermediate M-5

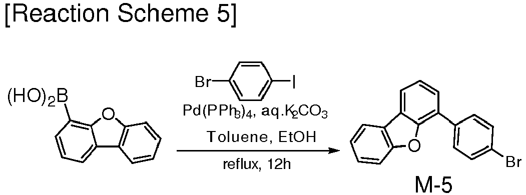

27 g of white solid Intermediate M-5 as a target compound was obtained using 20 g (94.4 mmol) of 4-dibenzofuranboronic acid instead of Intermediate M-1 in the synthesis method of Intermediate M-2 in [Reaction Scheme 5] (yield 89%).
LC-Mass (theoretical value: 322.00g/mol, measured value: M+1 = 323g/mol)

### Synthesis of Intermediate M-6

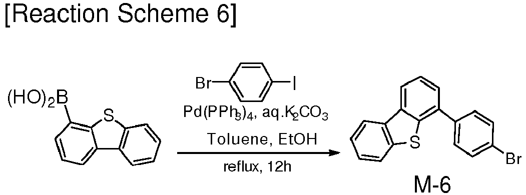

25 g of white solid Intermediate M-6 as a target compound was obtained using 20 g (87.69 mmol) of 4-dibenzothiopheneboronic acid instead of Intermediate M-1 in the synthesis method of Intermediate M-2 in [Reaction Scheme 6] (yield 83%).
LC-Mass (theoretical value: 337.98g/mol, measured value: M+1 = 338g/mol)

### Synthesis of Intermediate M-7

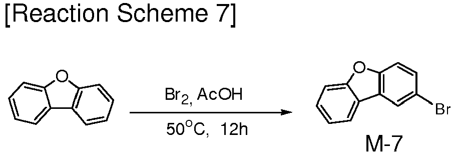

30 g (178.4 mmol) of a dibenzofuran was added in a round-bottomed flask and dissolved in 270 g of acetic acid, 29 g (181.5 mmol) of bromine dissolved in 6 g of acetic acid was slowly added thereto at 50° C for 4 hours. The reaction solution was further stirred at 50 °C for 8 hours and cooled down, and then the solution was added in distilled water. The orange solid was dissolved in dichloromethane and washed with a sodiumthiosulfite aqueous solution, the organic layer was dried magnesium sulfite and filtered, and the filtrate was concentrate under reduced pressure. The product was recrystallized in dichloromethane/n-hexane and then 10.1 g of white solid intermediate M-7 was obtained as a target compound (yield 23%).
GC-Mass (theoretical value: 245.97g/mol, measured value: 246g/mol)

### Synthesis of Intermediate M-8

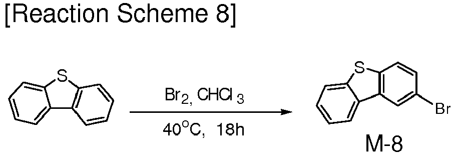

30 g (162.8 mmol) of a dibenzothiophene was added in a round-bottomed flask and dissolved in 2L of chloroform, then, 27.3 g (170.9 mmol) of bromine dissolved was slowly added thereto for 6 hours. The reaction solution was further stirred at 40° C for 12 hours and cooled down, and then the solution was added in a sodium thiosulfite aqueous solution. The organic layer was dried with magnesium sulfite and filtered and the filtrate was concentrated under a reduced pressure. The product was recrystallized with ethylacetate/n-hexane and then 15.4 g of white solid Intermediate M-8 was obtained as a target compound (yield 36%).
GC-Mass (theoretical value: 261.95g/mol, measured value: 262g/mol)

### Synthesis of Intermediate M-9

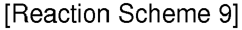

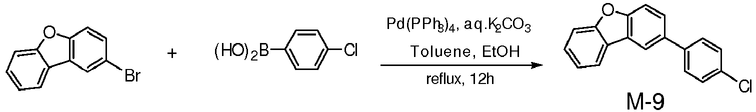

20 g (127.9 mmol) of 4-chlorophenylboronic acid, 30.0 g (121.5 mmol) of Intermediate M-7, and 1.48 g (1.28 mmol) of tetrakistriphenylphosphine palladium were dissolved in 320 ml of toluene and 160 ml of ethanol under a nitrogen atmosphere, and 160 ml of an aqueous solution including 37.7 g (255.8 mmol) of potassium carbonate was added thereto and then refluxed and stirred for 12 hours. After the reaction is completed, the reaction solutuon was extracted with ethylacetate, the extract was dried with magnesium sulfite and filtered, and then the filtrate was concentrated under a reduced pressure. The product was purified with n-hexane/dichloromethane (9:1 volume ratio) through a silica gel column chromatography and then 28.1 g of white solid Intermediate M-9 was obtained as a target compound (yield 83%).
LC-Mass (theoretical value: 278.05g/mol, measured value: M+1 = 279g/mol)

### Synthesis of Intermediate M-10

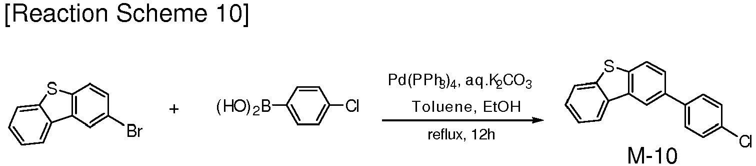

30.4 g of white solid Intermediate M-10 as a target compound was obtained using 32.0g (121.5mmol) of Intermediate M-8 instead of Intermediate M-7 in the synthesis method of Intermediate M-9 in [Reaction Scheme 10] (yield 85%).
LC-Mass (theoretical value: 294.03 g/mol, measured value: M+1 = 295 g/mol)

### Synthesis of Intermediate M-11

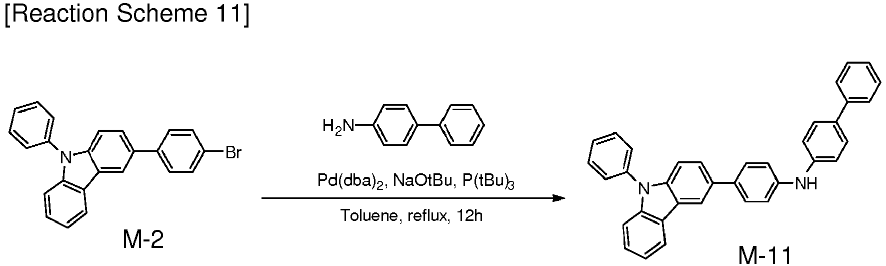

30 g (75.3 mmol) of intermediate M-2, 14.0 g (82.83 mmol) of 4-aminobiphenyl, 10.9 g (113.0 mmol) of sodium t-butoxide, and 0.46 g (2.26 mmol) of tri-tetra-butylphosphine were dissolved in 75 0ml of toluene, and 0.43 g (0.753 mmol) of Pd(dba)₂ was added, and then refluxed and stirred for 12 hours under a nitrogen atmosphere. After the reaction, the reactant was extracted with ethylacetate and distilled water, an organic layer was dried with magnesium sulfite and filtered, and the filtrate was concentrate under a reduced pressure. The product was purified with n-hexane/dichloromethane (7:3 volume ratio) through a silica gel column chromatography and then 27.5 g of white solid intermediate M-11 was obtained as a target compound (yield 75%).
LC-Mass (theoretical value: 486.21g/mol, measured value: M+1 = 487g/mol)

### Synthesis of Intermediate M-12

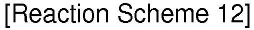

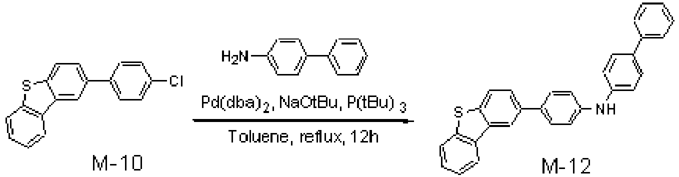

5.23 g of white solid Intermediate M-12 as a target compound was obtained using 5 g (17.0 mmol) of Intermediate M-10 instead of Intermediate M-2 in the synthesis method of Intermediate M-11 in [Reaction Scheme 12] (yield 72%).
LC-Mass (theoretical value: 427.14g/mol, measured value: M+1 = 428g/mol)

### Synthesis of Intermediate M-13

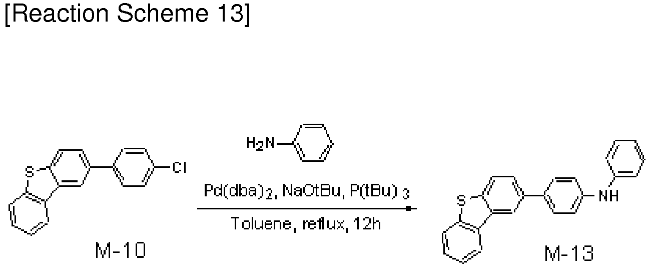

4.66 g of white solid Intermediate M-13 as a target compound was obtained using 1.66 g (17.85 mmol) of aniline instead of 4-aminobiphenyl in the synthesis method of Intermediate M-12 in [Reaction Scheme 13] (yield 78%).
LC-Mass (theoretical value: 351.11g/mol, measured value: M+1 = 352g/mol)

### Synthesis of Intermediate M-14

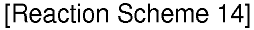

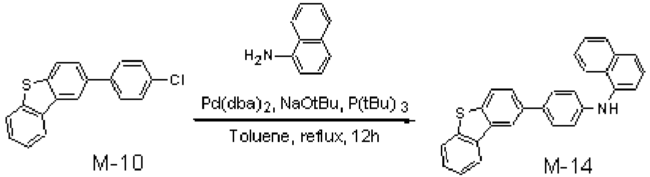

4.98 g of white solid Intermediate M-14 as a target compound was obtained using 2.56 g (17.85 mmol) of 1-aminonaphthalene instead of 4-aminobiphenyl in the synthesis method of Intermediate M-12 in [Reaction Scheme 14] (yield 73%).
LC-Mass (theoretical value: 401.12g/mol, measured value: M+1 = 402g/mol)

### Synthesis of Intermediate M-15

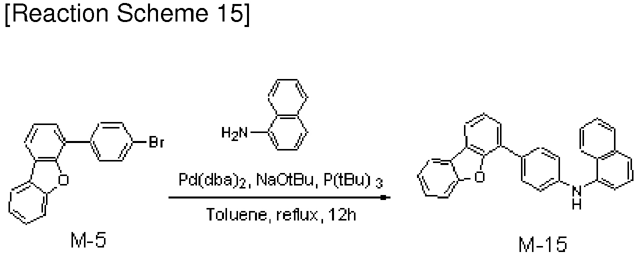

5.05 g of white solid Intermediate M-15 as a target compound was obtained using 5.49 g (17.0 mmol) of Intermediate M-5 instead of Intermediate M-10 in the synthesis method of Intermediate M-14 in [Reaction Scheme 15] (yield 77%).
LC-Mass (theoretical value: 385.15g/mol, measured value: M+1 = 386g/mol)

### Synthesis of Intermediate M-16

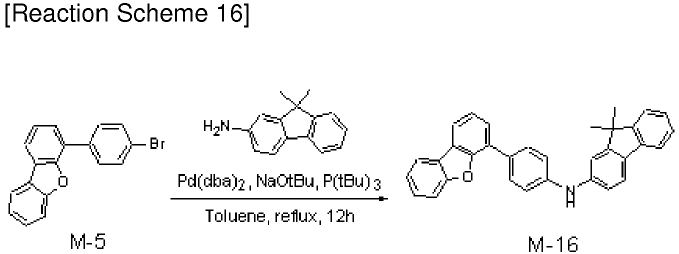

6.0 g of white solid Intermediate M-16 as a target compound was obtained using 3.74 g (17.85 mmol) of (9,9-dimethyl-9H-fluoren-2-yl)amine instead of 1-aminonaphthalene in the synthesis method of Intermediate M-15 in [Reaction Scheme 16] (yield 78%).
LC-Mass (theoretical value: 451.19g/mol, measured value: M+1 = 452g/mol)

### Synthesis of Intermediate M-17

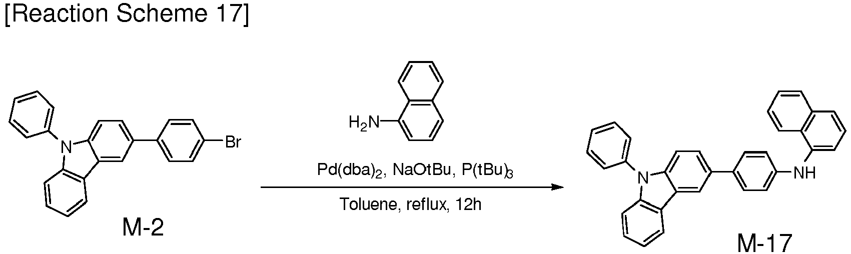

25.7 g of white solid Intermediate M-17 as a target compound was obtained using 11.9 g (82.83 mmol) of 1-aminonaphthalene instead of 4-aminobiphenyl in the synthesis method of Intermediate M-11 in [Reaction Scheme 17] (yield 74%).
LC-Mass (theoretical value: 460.19g/mol, measured value: M+1 = 461g/mol)

### Synthesis of Intermediate C-10-3

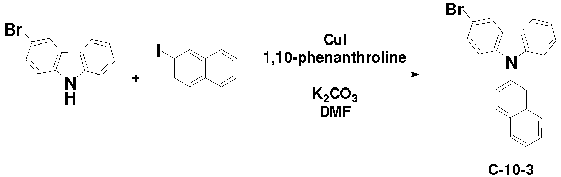

30 g (121.9 mmol) of 3-bromo carbazole, 1.5 equivalents of 2-iodo naphthalene, 0.05 equivalents of a copper catalyst, 0.1 equivalents of 1,10-phenanthroline, and 2 equivalents of potassium acetate was put in 250 mL of dimethyl formamide in a 500 mL round-bottomed flask and then, heated and refluxed for 18 hours. The reaction solution is cooled down, was added to 1 L of water and then solidified and stirred. The solid was filtered and recrystallized with 500 mL of ethyl acetate to obtain 34 g of Intermediate C-10-3.
LC/MS calculated for: C22H14BrN Exact Mass: 371.0310, found for: 372.05 [M+H]

### Synthesis of Intermediate C-10-2

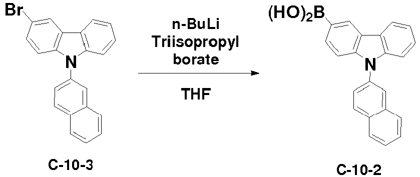

34 g (91.33 mmol) of the synthesized Intermediate C-10-3 was dissolved 200 mL of anhydrous tetrahydrofuran in a 500 mL round-bottomed flask, and a temperature was down to -78° C under a nitrogen atmosphere. 1.3 equivalents of a butyl lithium solution was added in a dropwise fashion and was stirred for 30 minutes, 1.5 equivalents of triisopropylborate was added in a dropwise fashion and was stirred for 1 hour, and a temperature is upto room temperature and the resultant was stirred for 2 hours. 100 mL of water was slowly added to the reaction solution and layer separated to separate an organic layer. The aqueous layer was extracted with 100 mL of ethyl acetate, and the organic layer was collected, washed with 100 mL of salt-saturated water, was dried with anhydrous magnesium sulfate, and then filtered and concentrated. The concentrated residue was vacuum-dried to obtain 33 g of Intermediate C-10-2 which was used without additional purification in the next reaction.

### Synthesis of Intermediate C-10-1

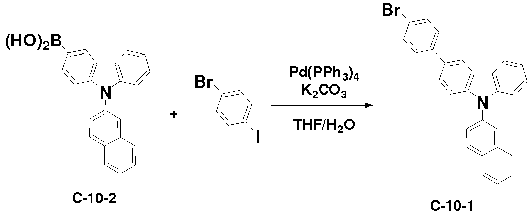

33 g of the synthesized Intermediate C-10-2 was put in a 500 mL round-bottomed flask, 1.5 equivalents of 4-bromo-iodo benzene, 0.05 equivalents of tetrakistriphenyl phosphine palladium, 2.5 equivalents of potassium carbonate were suspended in a mixed solution of 200 mL of tetrahydrofuran and 70 mL of water and heated and refluxed under a nitrogen atmosphere. The reaction solution was cooled down, the aqueous layer was removed, and then the organic layer was collected and concentrated. The concentrated residue was purified with a mixed solution of normal hexane and ethyl acetate with a silica gel column to obtain 37 g of Intermediate C-10-1.
LC/MS calculated for: C28H18BrN, Exact Mass: 447.0623, found for: 447.10

### Synthesis Example 10: Synthesis of Compound A-414

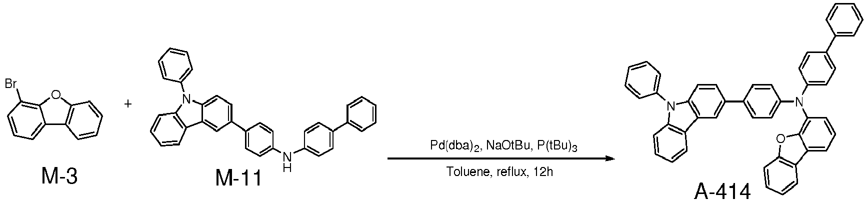

5 g (20.2 mmol) of Intermediate M-3, 9.85 g (20.2 mmol) of Intermediate M-11, 2.91 g (30.3 mmol) of sodium t-butoxide, and 0.12 g (2.26 mmol) of tri-tetra-butylphosphine were dissolved in 200 ml of toluene, 0.12 g (0.202 mmol) of Pd(dba)₂ was added, and then refluxed and stirred for 12 hours under a nitrogen atmosphere. After the reaction, the reactant was extracted with ethylacetate and distilled water, an organic layer was dried with magnesium sulfite and filtered, and the filtrate was concentrate under a reduced pressure. The product was purified with n-hexane/dichloromethane (7:3 volume ratio) through a silica gel column chromatography and then 12 g of white solid A-414 was obtained as a target compound (yield 91%).
LC-Mass (theoretical value: 652.25g/mol, measured value: M+1 = 653g/mol)

### Synthesis Example 11: Synthesis of Compound A-415

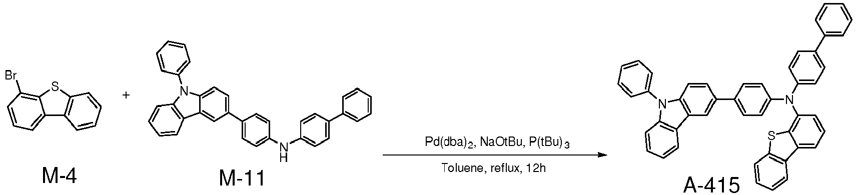

11.8 g of white solid A-415 as a target compound was obtained using 5.3 g (20.2 mmol) of Intermediate M-4 instead of Intermediate M-3 in Synthesis Example 10 (yield 87%).
LC-Mass (theoretical value: 668.23g/mol, measured value: M+1 = 669g/mol)

### Synthesis Example 12: Synthesis of Compound A-9

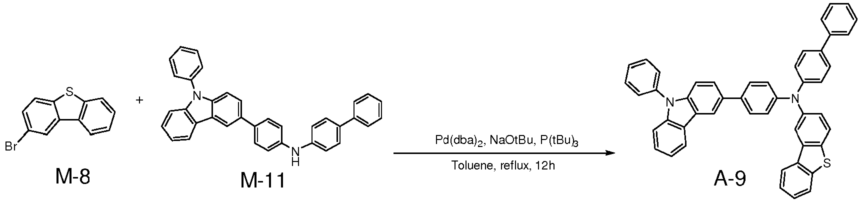

11.8 g of white solid A-9 as a target compound was obtained using 5.3 g (20.2 mmol) of Intermediate M-8 instead of Intermediate M-3 in Synthesis Example 10 (yield 87%).
LC-Mass (theoretical value: 668.23g/mol, measured value: M+1 = 669g/mol)

### Synthesis Example 13: Synthesis of Compound A-10

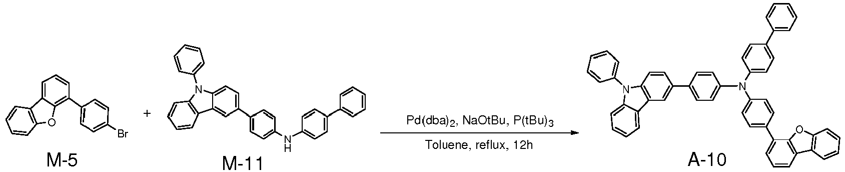

12.4 g of white solid A-10 as a target compound was obtained using 6.5 g (20.2 mmol) of Intermediate M-5 instead of Intermediate M-3 in Synthesis Example 10 (yield 84%).
LC-Mass (theoretical value: 728.28g/mol, measured value: M+1 = 729g/mol)

### Synthesis Example 14: Synthesis of Compound A-11

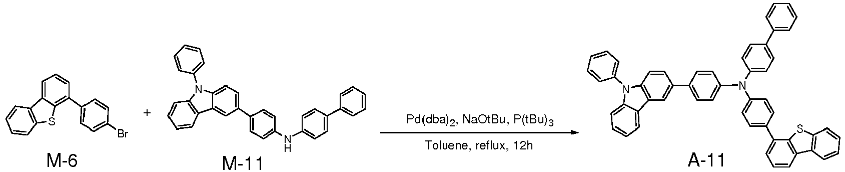

13.2 g of white solid A-11 as a target compound was obtained using 6.85 g (20.2 mmol) of Intermediate M-6 instead of Intermediate M-3 in Synthesis Example 10 (yield 88%).
LC-Mass (theoretical value: 744.26g/mol, measured value: M+1 = 745g/mol)

### Synthesis Example 15: Synthesis of Compound A-18

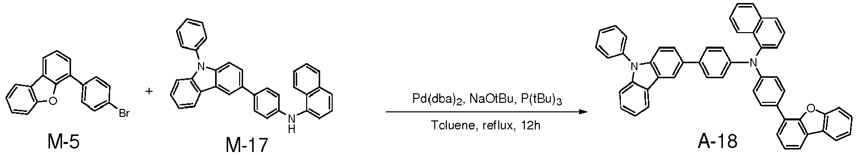

6.53 g (20.2 mmol) of Intermediate M-5 and 9.30 g (20.2 mmol) of Intermediate M-17, 2.91 g (30.3 mmol) of sodium t-butoxide, and 0.12 g (2.26 mmol) of tri-tetra-butylphosphine were dissolved in 200 ml of toluene, 0.12 g (0.202 mmol) of Pd(dba)₂ was added, and then refluxed and stirred for 12 hours under a nitrogen atmosphere. After the reaction, the reactant was extracted with ethyl acetate and distilled water, an organic layer was dried with magnesium sulfite and filtered, and the filtrate was concentrate under a reduced pressure. The product was purified with n-hexane/dichloromethane (7:3 volume ratio) through a silica gel column chromatography and then 12.5 g of white solid A-18 was obtained as a target compound (yield 88%).
LC-Mass (theoretical value: 702.27g/mol, measured value: M+1 = 703g/mol)

### Synthesis Example 16: Synthesis of Compound A-19

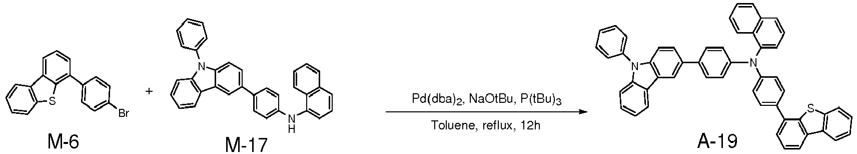

12.3 g of white solid A-19 as a target compound was obtained using 6.85 g (20.2 mmol) of Intermediate M-6 instead of Intermediate M-5 in Synthesis Example 15 (yield 85%).
LC-Mass (theoretical value: 718.24g/mol, measured value: M+1 = 719g/mol)

### Synthesis Example 17: Synthesis of Compound A-327

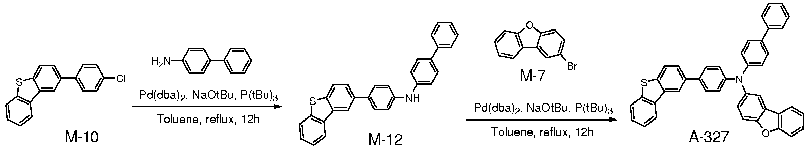

5.2 g (12.2 mmol) of Intermediate M-12 and 3.0 g (12.2 mmol) of Intermediate M-7, 1.76 g (18.3 mmol) of sodium t-butoxide, and 0.074 g (0.37 mmol) of tri-tetra-butylphosphine were dissolved in 120 ml of toluene, 0.070 g (0.122 mmol) of Pd(dba)₂ was added, and then refluxed and stirred for 12 hours under a nitrogen atmosphere. After the reaction, the reactant was extracted with ethylacetate and distilled water, an organic layer was dried with magnesium sulfite and filtered, and the filtrate was concentrate under a reduced pressure. The product was purified with n-hexane/dichloromethane (7:3 volume ratio) through a silica gel column chromatography and then 6.2 g of white solid A-327 was obtained as a target compound (yield 86%).
LC-Mass (theoretical value: 593.18g/mol, measured value: M+1 = 594g/mol)

### Synthesis Example 18: Synthesis of Compound A-335

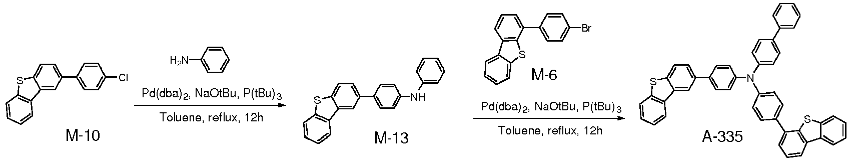

4.3 g (12.2 mmol) of Intermediate M-13 and 4.14 g (12.2 mmol) of Intermediate M-6, 1.76 g (18.3 mmol) of sodium t-butoxide, and 0.074 g (0.37 mmol) of tri-tetra-butylphosphine were dissolved in 120 ml of toluene, 0.070 g (0.122 mmol) of Pd(dba)₂ was added, and then refluxed and stirred for 12 hours under a nitrogen atmosphere. After the reaction, the reactant was extracted with ethylacetate and distilled water, an organic layer was dried with magnesium sulfite and filtered, and the filtrate was concentrate under a reduced pressure. The product was purified with n-hexane/dichloromethane (7:3 volume ratio) through a silica gel column chromatography and then 6.8 g of white solid A-335 was obtained as a target compound (yield 91%).
LC-Mass (theoretical value: 609.16g/mol, measured value: M+1 = 610g/mol)

### Synthesis Example 19: Synthesis of Compound A-340

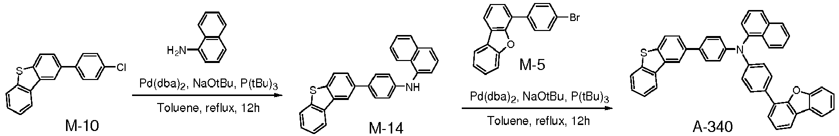

4.9 g (12.2 mmol) of Intermediate M-14 and 3.94 g (12.2 mmol) of Intermediate M-5, 1.76 g (18.3 mmol) of sodium t-butoxide, and 0.074 g (0.37 mmol) of tri-tetra-butylphosphine were dissolved in 120 ml of toluene, 0.070 g (0.122 mmol) of Pd(dba)₂ was added, and then refluxed and stirred for 12 hours under a nitrogen atmosphere. After the reaction, the reactant was extracted with ethylacetate and distilled water, an organic layer was dried with magnesium sulfite and filtered, and the filtrate was concentrate under a reduced pressure. The product was purified with n-hexane/dichloromethane (7:3 volume ratio) through a silica gel column chromatography and then 7.2 g of white solid A-340 was obtained as a target compound (yield 92%).
LC-Mass (theoretical value: 643.20g/mol, measured value: M+1 = 644g/mol)

### Synthesis Example 20: Synthesis of Compound A-373

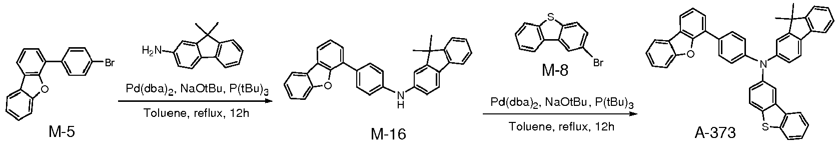

5.51 g (12.2 mmol) of Intermediate M-16 and 3.21 g (12.2 mmol) of Intermediate M-8, 1.76 g (18.3 mmol) of sodium t-butoxide, and 0.074 g (0.37 mmol) of tri-tetra-butylphosphine were dissolved in 120 ml of toluene, 0.070 g (0.122 mmol) of Pd(dba)₂ was added, and then refluxed and stirred for 12 hours under a nitrogen atmosphere. After the reaction, the reactant was extracted with ethylacetate and distilled water, an organic layer was dried with magnesium sulfite and filtered, and the filtrate was concentrate under a reduced pressure. The product was purified with n-hexane/dichloromethane (7:3 volume ratio) through a silica gel column chromatography and then 7.0 g of white solid A-373 was obtained as a target compound (yield 91%).
LC-Mass (theoretical value: 633.21 g/mol, measured value: M+1 = 634g/mol)

### Synthesis Example 21: Synthesis of Compound A-376

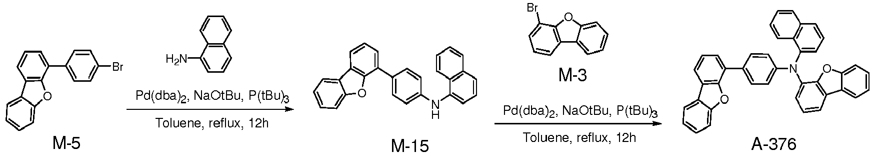

4.7 g (12.2 mmol) of Intermediate M-15 and 3.01 g (12.2 mmol) of Intermediate M-3, 1.76 g (18.3 mmol) of sodium t-butoxide, and 0.074 g (0.37 mmol) of tri-tetra-butylphosphine were dissolved in 120 ml of toluene, 0.070 g (0.122 mmol) of Pd(dba)₂ was added, and then refluxed and stirred for 12 hours under a nitrogen atmosphere. After the reaction, the reactant was extracted with ethylacetate and distilled water, an organic layer was dried with magnesium sulfite and filtered, and the filtrate was concentrate under a reduced pressure. The product was purified with n-hexane/dichloromethane (7:3 volume ratio) through a silica gel column chromatography and then 6.2 g of white solid A-376 was obtained as a target compound (yield 92%).
LC-Mass (theoretical value: 551.19g/mol, measured value: M+1 = 552g/mol)

### Synthesis Example 22: Synthesis of Compound C10

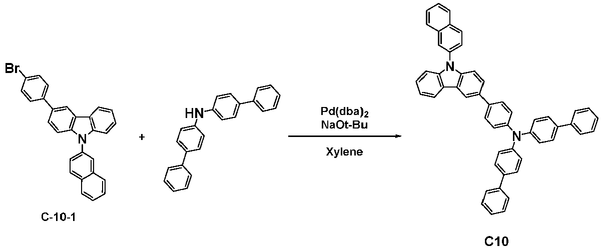

37 g (82.52 mmol), 1.2 equivalents of dibiphenyl amine, 0.05 equivalents of dibenzylidine acetone bispalladium, and 1.5 equivalents of sodium tertiary butoxide were put in a 500 mL round-bottomed flask, 250 mL of xylene was added, and then refluxed and stirred for 18 hours under a nitrogen atmosphere. The reaction solution is cooled down, was diluted in 1 L of methanol, and then was stirred. The produced solids were filtered. The solides were washed with 300 mL of water and 300 mL of methanol, solids are collected, and recrystallized with 50 mL of methylene chloride and 300 mL of hexane to obtain 43 g of Compound C10.
LC/MS calculated for: C52H36N2 Exact Mass: 688.2878, found for: 688.29

### Synthesis Example 23: Synthesis of Compound C31

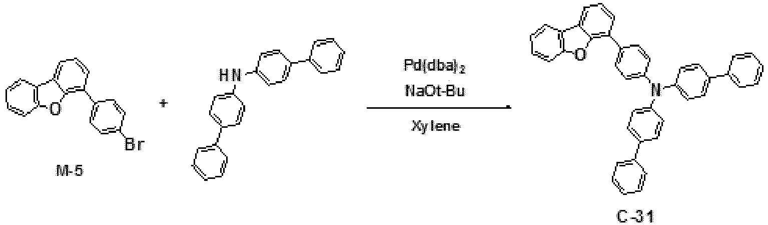

10 g (30.9 mmol) of Intermediate M-5 and 9.9 g (30.9 mmol) of bis(4-biphenyl)amine, and 4.5 g (46.35 mmol) of sodium t-butoxide were put in a round-bottomed flask and 155 ml of toluene was added, and they were dissolved therein. 0.178 g (0.31 mmol) of Pd(dba)2 and 0.125 g (0.62 mmol) of tri-tertiary-butylphosphine were sequentially added thereto, and then refluxed and stirred for 4 hours under a nitrogen atmosphere. After the reaction is completed, it was extracted with toluene and distilled water, the organic layer was dried with magnesium sulfate and filtered, and the filterate was concentrated under reduced pressure. The product was purified with n-hexane/dichloromethane (8:2 volume ratio) through a silica gel column chromatography and then 16 g of Compound C31 was obtained as a target compound (yield 92%).
LC-Mass (theoretical value: 563.22g/mol, measured value: M+ = 563.28g/mol)

### Manufacture of Organic Light Emitting Diode I -Green Diode

### Example 1

A glass substrate coated with ITO (indium tin oxide) as a 1500 Å-thick thin film was ultrasonic wave-washed with distilled water. After washing with the distilled water, the glass substrate was ultrasonic wave-washed with a solvent such as isopropyl alcohol, acetone, methanol, and the like and dried and then, moved to a plasma cleaner, cleaned by using oxygen plasma for 10 minutes, and moved to a vacuum depositor. This obtained ITO transparent electrode was used as an anode, a 700 Å-thick hole injection layer was formed on the ITO substrate by vacuum depositing Compound A, and a hole transport layer was formed on the injection layer by depositing Compound B to be 50 Å thick and Compound C to be 1020 Å thick. On the hole transport layer (HTL), a 400 Å-thick light emitting layer was formed by vacuum-depositing Compound 1 of Synthesis Example 1 and Compound A-414 of Synthesis Example 10 simultaneously as a host and 10 wt% of tris(2-phenylpyridine)iridium(III) [Ir(ppy)₃] as a dopant. Herein, Compound 1 and Compound A-414 were used in a weight ratio of 3:7, but their ratio in the following Examples was separately provided. Subsequently, on the light emitting layer, a 300 Å-thick electron transport layer was formed by simultaneously vacuum-depositing the compound D and Liq in a ratio of 1:1, and on the electron transport layer, Liq and Al were sequentially vacuum-deposited to be 15 Å thick and 1200 Å thick, manufacturing an organic light emitting diode.

The organic light emitting diode had a structure of 5-layered organic thin films specifically as follows.
ITO/Compound A (700 Å)/Compound B (50 Å)/Compound C (1020 Å)/EML[Compound 1:A-414:Ir(ppy)₃ = 27wt%:63wt%:10wt%] (400 Å)/Compound D:Liq (300 Å)/Liq (15 Å)/AI (1200 Å).
Compound A: N4,N4'-diphenyl-N4,N4'-bis(9-phenyl-9H-carbazol-3-yl)biphenyl-4,4'-diamine
Compound B: 1,4,5,8,9,11-hexaazatriphenylene-hexacarbonitrile (HAT-CN),
Compound C:N-(biphenyl-4-yl)-9,9-dimethyl-N-(4-(9-phenyl-9H-carbazol-3-yl)phenyl)-9H-fluoren-2-amine
Compound D: 8-(4-(4,6-di(naphthalen-2-yl)-1,3,5-triazin-2-yl)phenyl)quinoline

### Example 2 to Example 16

Each organic light emitting diode according to Example 2 to Example 6 were manufactured according to the same method as Example 1 by using the first hosts and the second hosts as shown in Table 1 in each corresponding ratio.

### Comparative Example 1 to Comparative Example 10

Each organic light emitting diode according to Comparative Examples 1 to 10 was manufactured according to the same method as Example 1 by using the first hosts as single hosts as shown in Table 1.

### Comparative Example 11

An organic light emitting diode was manufactured according to the same method as Example 1 by using Compound A-414 and Comparative Example Compound I in a ratio of 5:5 as a host.

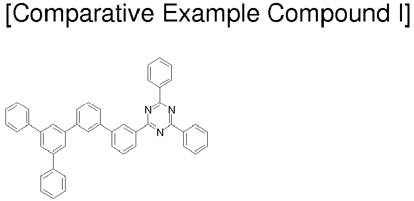

### Comparative Example 12

An organic light emitting diode was manufactured according to the same method as Example 1 by using Compound 1 and mCP (1,3-bis(N-carbazolyl)benzene) in a ratio of 5:5 as a host.

### Manufacture of Organic Light Emitting Diode II-Red Diode

### Example 17

An organic organic light emitting diode according to Example 17 was manufactured according to the same method as Example 1, except for using a mixture of Compound 181 of Synthesis Example Ad-1 and Compound C31 of Synthesis Example 23 in a weight ratio of 3:7 as a host and doping a dopant, 5 wt% of [Ir(piq)₂acac] to form a light emitting layer.

### Example 18 to Example 20

Each organic light emitting diode according to Example 18 to Example 20 were manufactured according to the same method as Example 17 by using the first hosts and the second hosts as shown in Table 2 in each corresponding ratio.

### Comparative Example 13 to Comparative Example 15

Each organic light emitting diode according to Comparative Examples 13 to 15 was manufactured according to the same method as Example 17 by using the first hosts as single hosts as shown in Table 2.

### Evaluation 1: Luminous Efficiency and Life-span Increase Effect

Luminous efficiency and life-span characteristics of the organic light emitting diodes according to Examples 1 to 20 and Comparative Examples 1 to 15 were evaluated. Specific measurement methods are as follows, and the results are shown in Tables 1 and 2.

### (1) Measurement of Current Density Change Depending on Voltage Change

The obtained organic light emitting diodes were measured regarding a current value flowing in the unit device, while increasing the voltage from 0 V to 10 V using a current-voltage meter (Keithley 2400), and the measured current value was divided by area to provide the results.

### (2) Measurement of Luminance Change Depending on Voltage Change

Luminance was measured by using a luminance meter (Minolta Cs-1000A), while the voltage of the organic light emitting diodes was increased from 0 V to 10 V.

### (3) Measurement of Luminous Efficiency

Current efficiency (cd/A) at the same current density (10 mA/cm²) were calculated by using the luminance, current density, and voltages (V) from the items (1) and (2).

### (4) Measurement of Life-span

T90 life-spans of the organic light emitting diodes according to Examples 1 to 12 and Comparative Examples 1 to 14 were measured as a time when their luminance decreased down to 90 % relative to the initial luminance (cd/m2) after emitting light with 5000 cd/m² as the initial luminance (cd/m²) and measuring their luminance decrease depending on a time with a Polanonix life-span measurement system.

### (5) Measurement of Driving Voltage

Driving voltage of each organic light emitting diode was measured at 15 mA/cm² by using a current-voltage meter (Keithley 2400), and the results are provided in Tables 1 and 2.

**[Table 1]**

| Green Diode Data | | | | | | | |
|---|---|---|---|---|---|---|---|
| | First host | Second host | Ratio of First host + Second host | Col or | Efficie ncy (Cd/A) | Life-span T90 | Driving voltage (Vd) |
| Example 1 | 1 | A-414 | 3:7 | gre en | 54 | 235 | 3.9 |
| Example 2 | 2 | A-414 | 3:7 | gre en | 49 | 244 | 4.1 |
| Example 3 | 2 | C31 | 3:7 | gre en | 61 | 250 | 3.6 |
| Example 4 | 3 | A-415 | 3:7 | gre en | 52 | 261 | 4.3 |
| Example 5 | 100 | A-9 | 3:7 | gre en | 51 | 280 | 4.2 |
| Example 6 | 4 | A-10 | 2:8 | gre en | 48 | 291 | 3.9 |
| Example 7 | 4 | C31 | 2:8 | gre en | 49 | 311 | 3.7 |
| Example 8 | 16 | A-11 | 2:8 | gre en | 53 | 231 | 3.8 |
| Example 9 | 16 | C31 | 2:8 | gre en | 62 | 352 | 3.6 |
| Example 10 | 16 | C10 | 2:8 | gre en | 55 | 309 | 3.7 |
| Example 11 | 126 | A-18 | 2:8 | gre en | 49 | 278 | 4.2 |
| Example 12 | 126 | C31 | 2:8 | gre en | 55 | 334 | 3.7 |
| Example 13 | 140 | A-19 | 3:7 | gre en | 48 | 257 | 4.3 |
| Example | 140 | C10 | 3:7 | gre | 51 | 319 | 3.7 |
| 14 | | | | en | | | |
| Example 15 | 113 | A-327 | 3:7 | gre en | 47 | 281 | 4.1 |
| Example 16 | 113 | C31 | 3:7 | gre en | 61 | 376 | 3.8 |
| Compar ative Example 1 | 1 | - | - | gre en | 35 | 71 | 4.8 |
| Compar ative Example 2 | 2 | - | - | gre en | 35 | 80 | 4.9 |
| Compar ative Example 3 | 4 | - | - | gre en | 37 | 75 | 5.1 |
| Compar ative Example 4 | 16 | - | - | gre en | 40 | 77 | 4.7 |
| Compar ative | 126 | - | - | gre en | 33.6 | 10 | 4.5 |
| Example 5 | | | | | | | |
| Compar ative Example 6 | 113 | - | - | gre en | 20.6 | 32 | 4.7 |
| Compar ative Example 7 | A-414 | - | - | gre en | 18 | 15 | 6.2 |
| Compar ative Example 8 | A-415 | - | - | gre en | 19 | 18 | 6.4 |
| Compar ative Example 9 | C31 | - | - | gre en | 21 | 19 | 5.9 |
| Compar ative Example 10 | C10 | - | - | gre en | 22 | 23 | 6.8 |
| Compar | Comparati | A-414 | 5:5 | gre | 38 | 54 | 5.1 |
| ative Example 11 | ve Example compound I | | | en | | | |
| Compar ative Example 12 | 1 | mCP | 5:5 | gre en | 40 | 75 | 4.8 |

**[Table 2]**

| Red Diode Data | | | | | | | |
|---|---|---|---|---|---|---|---|
| | First host | Second host | Ratio of First host + Second host | Col or | Efficie ncy (Cd/A) | Life-span T90 | Driving voltage (Vd) |
| Example 17 | 181 | C31 | 3:7 | red | 23 | 450 | 3.6 |
| Example 18 | 180 | A-414 | 3:7 | red | 21 | 400 | 3.6 |
| Example 19 | 183 | C31 | 3:7 | red | 22 | 380 | 3.94 |
| Example 20 | 190 | C31 | 3:7 | red | 20 | 350 | 3.9 |
| Compar ative Example 13 | 181 | - | 100 | red | 12 | 25 | 6.6 |
| Compar ative Example 14 | C31 | - | 100 | red | 11 | 33 | 7.9 |
| Compar ative Example 15 | A-414 | - | 100 | red | 9 | 30 | 8.1 |

Referring to Tables 1 and 2, the host combination of the present invention showed remarkably improved luminous efficiency, life-span, and driving voltage compared with a single host.

The scope of the present invention is defined by the appended claims.

## Claims

1. A composition for an organic optoelectronic device, comprising
at least one first compound represented by formula 1; and
at least one second compound represented by formula 2: 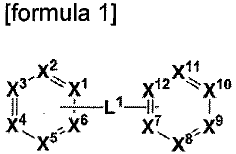 wherein, in formula 1,
X¹ to X¹² are independently N, C, or CR^{a},
at least one of X¹ to X⁶ is N,
at least one of X⁷ to X¹² is N,
R^{a'}s are independently hydrogen, deuterium, a substituted or unsubstituted C1 to C30 alkyl group, a substituted or unsubstituted C1 to C30 alkenyl group, a substituted or unsubstituted C1 to C30 alkynyl group, a substituted or unsubstituted C6 to C30 aryl group, a substituted or unsubstituted C6 to C30 aryloxy group, a substituted or unsubstituted C6 to C30 arylthio group, a substituted or unsubstituted C2 to C30 heteroaryl group, a hydroxyl group, a thiol group, or a combination thereof,
R^{a'}s are independently present or adjacent R^{a'}s are linked with each other to provide a ring, and
L¹ is a C6 to C30 arylene group that is unsubstituted or substituted with deuterium, a C1 to C40 silyl group, a C1 to C30 alkyl group, a C3 to C30 cycloalkyl group, a C2 to C30 heterocycloalkyl group, or a C6 to C30 aryl group; 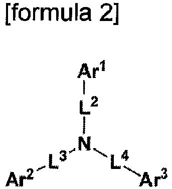
wherein, in formula 2,
L² to L⁴ are independently a single bond, a substituted or unsubstituted C6 to C30 arylene group, or a substituted or unsubstituted C2 to C30 heteroarylene group,
Ar¹ to Ar³ are independently, a substituted or unsubstituted C6 to C30 aryl group, a substituted or unsubstituted C2 to C30 heterocyclic group, or a combination thereof, and
when specific definition is not otherwise provided, "substituted" of formulas 1 and 2 refers to replacement of at least one hydrogen by deuterium, a halogen, a hydroxyl group, an amino group, a C1 to C30 amine group, a C6 to C30 arylamine group, a nitro group, a C1 to C40 silyl group, a C1 to C30 alkyl group, a C3 to C30 cycloalkyl group, a C2 to C30 heterocycloalkyl group, a C6 to C30 aryl group, a C2 to C30 heterocyclic group, a C1 to C20 alkoxy group, a C1 to C10 trifluoroalkyl group, or a cyano group;
and the first compound and the second compound are included in a weight ratio of 1:9 to 9:1.

2. The composition for an organic optoelectronic device of claim 1, wherein formula 1 is represented by one of formula 1- I to formula 1-IV: 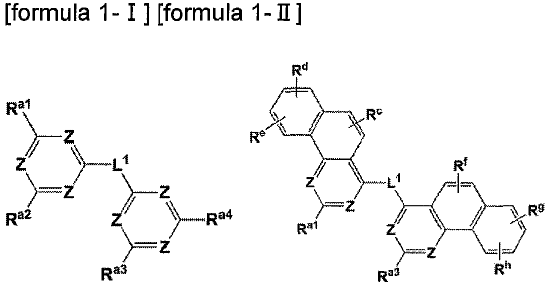 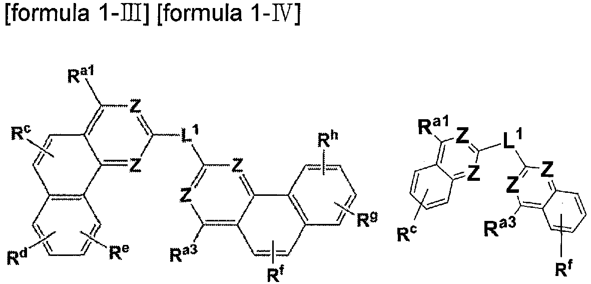 wherein, in formulas 1- I to 1-IV,
Z's are independently N, or CR^{a},
in each ring including Z, at least one Z is N,
R^{a}, R^{a1} to R^{a4}, R^{c}, R^{d}, R^{e}, R^{f}, R^{g}, and R^{h} are independently hydrogen, deuterium, a substituted or unsubstituted C1 to C30 alkyl group, a substituted or unsubstituted C6 to C30 aryl group, a substituted or unsubstituted C2 to C30 heteroaryl group, or a combination thereof,
L¹ is a C6 to C30 arylene group that is unsubstituted or substituted with deuterium, a C1 to C40 silyl group, a C1 to C30 alkyl group, a C3 to C30 cycloalkyl group, a C2 to C30 heterocycloalkyl group, or a C6 to C30 aryl group, and
when specific definition is not otherwise provided, "substituted" is the same as defined in claim 1.

3. The composition for an organic optoelectronic device of claim 1, wherein
L¹ is a phenylene group that is unsubstituted or substituted with deuterium, a C1 to C40 silyl group, a C1 to C30 alkyl group, or a C6 to C30 aryl group;
a biphenylene group that is unsubstituted or substituted with deuterium, a C1 to C40 silyl group, a C1 to C30 alkyl group, or a C6 to C30 aryl group;
a terphenylene group that is unsubstituted or substituted with deuterium, a C1 to C40 silyl group, a C1 to C30 alkyl group, or a C6 to C30 aryl group; or
a quaterphenylene group that is unsubstituted or substituted with deuterium, a C1 to C40 silyl group, a C1 to C30 alkyl group, or a C6 to C30 aryl group.

4. The composition for an organic optoelectronic device of claim 1, wherein L¹ is a substituted or unsubstituted linker selected from groups of Group 2: 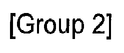 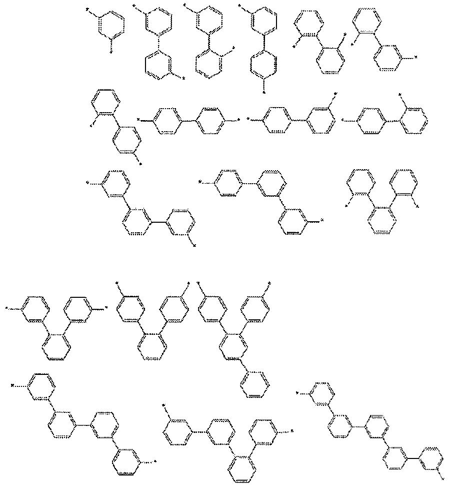 wherein, in Group 2, * is a linking point with an adjacent atom.

5. The composition for an organic optoelectronic device of claim 1, wherein X¹ to X¹² of formula 1 are independently N, C, or CR^{a},
three of X¹ to X⁶ are N,
three of X⁷ to X¹² are N,
R^{a'}s are independently hydrogen, deuterium, a substituted or unsubstituted C1 to C30 alkyl group, a substituted or unsubstituted C6 to C30 aryl group, a substituted or unsubstituted C2 to C30 heteroaryl group, or a combination thereof, and
L¹ is a C6 to C30 arylene group that is unsubstituted or substituted with deuterium, a C1 to C30 alkyl group, or a C6 to C30 aryl group,
wherein when specific definition is not otherwise provided, "substituted" refers to replacement of at least one hydrogen by deuterium, a halogen, a hydroxyl group, a C1 to C40 silyl group, a C1 to C30 alkyl group, a C3 to C30 cycloalkyl group, a C2 to C30 heterocycloalkyl group, a C6 to C30 aryl group, or a C2 to C30 heterocyclic group.

6. The composition for an organic optoelectronic device of claim 5, wherein
R^{a'}s are a substituted or unsubstituted C6 to C30 aryl group, and
the C6 to C30 aryl group is a substituted or unsubstituted phenyl group, a substituted or unsubstituted biphenyl group, a substituted or unsubstituted terphenyl group, a substituted or unsubstituted quaterphenyl group, a substituted or unsubstituted naphthyl group, a substituted or unsubstituted anthracenyl group, a substituted or unsubstituted fluorenyl group, a substituted or unsubstituted triphenylene group, or a substituted or unsubstituted phenanthrenyl group.

7. The composition for an organic optoelectronic device of claim 1, wherein
L² to L⁴ of formula 2 are independently a single bond, a substituted or unsubstituted phenylene group, a substituted or unsubstituted biphenylene group, a substituted or unsubstituted naphthyl group, a substituted or unsubstituted fluorenyl group, a substituted or unsubstituted pyridinyl group, a substituted or unsubstituted pyrimidinyl group, a substituted or unsubstituted quinolinyl group, or a combination thereof,
Ar¹ to Ar³ are independently a substituted or unsubstituted phenyl group, a substituted or unsubstituted biphenyl group, a substituted or unsubstituted terphenyl group, a substituted or unsubstituted quaterphenyl group, a substituted or unsubstituted naphthyl group, a substituted or unsubstituted fluorenyl group, a substituted or unsubstituted carbazolyl group, a substituted or unsubstituted dibenzofuranyl group, a substituted or unsubstituted dibenzothiophenyl group, a substituted or unsubstituted anthracenyl group, a substituted or unsubstituted phenanthrenyl group, a substituted or unsubstituted triphenylene group, a substituted or unsubstituted quinolinyl group, a substituted or unsubstituted pyridinyl group, a substituted or unsubstituted pyrimidinyl group, a substituted or unsubstituted thiophenyl group, or a combination thereof.

8. The composition for an organic optoelectronic device of claim 7, wherein Ar¹ to Ar³ of formula 2 are selected from substituted or unsubstituted groups of Group 4: 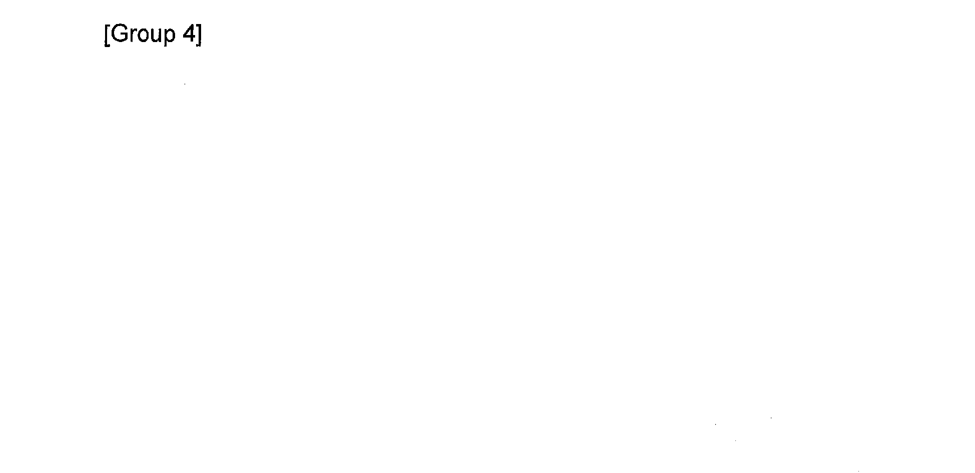 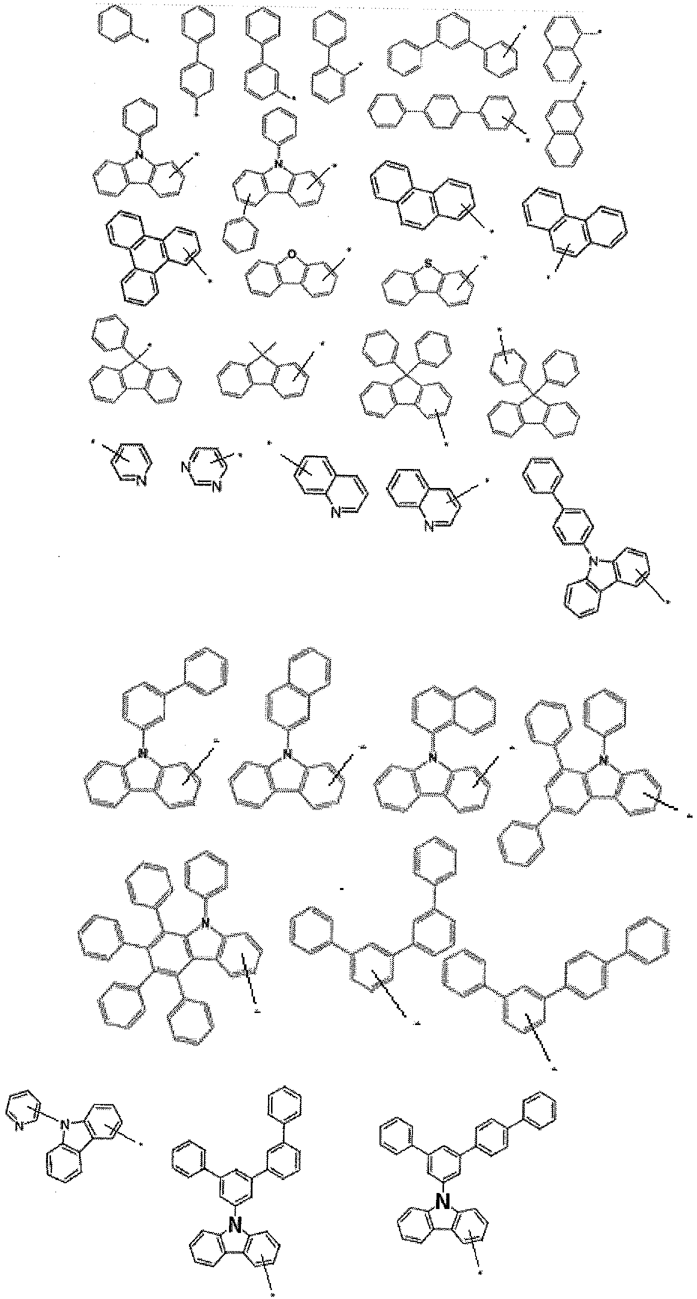 wherein, in Group 4, * is a linking point withan adjacent atom.

9. The composition for an organic optoelectronic device of claim 1, wherein
the first compound is represented by formulas 1- I a to 1-IVa, and
the second compound is formula 2 wherein L² to L⁴ are independently a single bond, or a substituted or unsubstituted phenylene group, and Ar¹ to Ar³ are independently a substituted or unsubstituted C6 to C30 aryl group, or a substituted or unsubstituted C2 to C30 heterocyclic group, provided that at least one of Ar¹ to Ar³ is a substituted or unsubstituted C2 to C30 heterocyclic group: 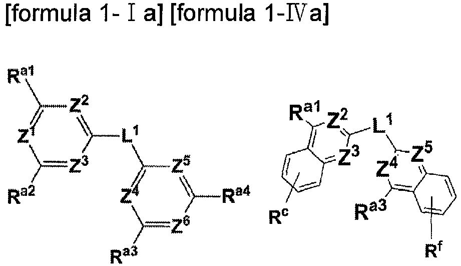 wherein, in formulas 1- I a and 1-IVa,
Z¹ to Z⁶ are independently N, or CR^{a},
at least two of Z¹ to Z³ are N,
at least two of Z⁴ to Z⁶ are N,
R^{a'}s, R^{a1} to R^{a4} R^{c} and R^{f} are independently hydrogen, or a substituted or unsubstituted C6 to C30 aryl group,
L¹ is a C6 to C30 arylene group that is substituted or unsubstituted with deuterium, a C1 to C30 alkyl group, or a C6 to C30 aryl group, and
when specific definition is not otherwise provided, "substituted" is the same as defined in claim 1.

10. The composition for an organic optoelectronic device of claim 1, wherein the composition further includes a phosphorescent dopant.

11. An organic optoelectronic device, comprising
an anode (120) and a cathode (110) facing each other, and
at least one organic layer (105) disposed between the anode and the cathode,
wherein the organic layer includes the composition for an organic optoelectronic device of any one of claim 1 to claim 10.

12. The organic optoelectronic device of claim 11, wherein
the organic layer (105) includes a light emitting layer (130),
the light emitting layer includes the composition for an organic optoelectronic device.

13. A display device comprising the organic optoelectronic device of claim 11.

## Patentansprüche

1. Zusammensetzung für eine organische optoelektronische Vorrichtung, mit:
mindestens einer durch die Formel 1 dargestellten ersten Verbindung; und
mindestens einer durch die Formel 2 dargestellten zweiten Verbindung: 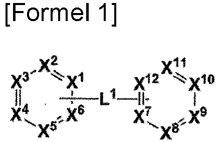 wobei in Formel 1
X¹ bis X¹² unabhängig voneinander N, C oder CR^{a} sind,
mindestens eine der Komponenten X¹ bis X⁶ N ist,
mindestens eine der Komponenten X⁷ bis X¹² N ist,
die Komponenten R^{a} unabhängig voneinander Wasserstoff, Deuterium, eine substituierte oder unsubstituierte C1- bis C30-Alkylgruppe, eine substituierte oder unsubstituierte C1- bis C30-Alkenylgruppe, eine substituierte oder unsubstituierte C1- bis C30-Alkinylgruppe, eine substituierte oder unsubstituierte C6- bis C30-Arylgruppe, eine substituierte oder unsubstituierte C6- bis C30-Aryloxygruppe, eine substituierte oder unsubstituierte C6-bis C30-Arylthiogruppe, eine substituierte oder unsubstituierte C2- bis C30-Heteroarylgruppe, eine Hydroxylgruppe, eine Thiolgruppe oder eine Kombination davon sind,
die Komponenten R^{a} unabhängig voneinander vorhanden sind oder benachbarte Komponenten R^{a} miteinander verbunden sind, um einen Ring zu bilden, und
L¹ eine C6- bis C30-Arylengruppe ist, die unsubstituiert oder mit Deuterium, einer C1- bis C40-Silylgruppe, einer C1- bis C30-Alkylgruppe, einer C3- bis C30-Cycloalkylgruppe, einer C2- bis C30-Heterocycloalkylgruppe oder einer C6- bis C30-Arylgruppe substituiert ist; 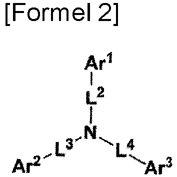
wobei in Formel 2
L² bis L⁴ unabhängig voneinander eine Einfachbindung, eine substituierte oder unsubstituierte C6- bis C30-Arylengruppe oder eine substituierte oder unsubstituierte C2- bis C30-Heteroarylengruppe sind,
Ar¹ bis Ar³ unabhängig voneinander eine substituierte oder unsubstituierte C6- bis C30-Arylgruppe, eine substituierte oder unsubstituierte heterocyclische C2- bis C30-Gruppe oder eine Kombination davon sind, wobei
wenn eine spezifische Definition nicht explizit angegeben ist, "substituiert" in den Formeln 1 und 2 sich auf das Ersetzen mindestens eines Wasserstoffs durch Deuterium, ein Halogen, eine Hydroxylgruppe, eine Aminogruppe, eine C1- bis C30-Amingruppe, eine C6- bis C30-Arylamingruppe, eine Nitrogruppe, eine C1- bis C40-Silylgruppe, eine C1- bis C30-Alkylgruppe, eine C3- bis C30-Cycloalkylgruppe, eine C2- bis C30-Heterocycloal-kylgruppe, eine C6- bis C30-Arylgruppe, eine heterocyclische C2- bis C30-Gruppe, eine C1- bis C20-Alkoxygruppe, eine C1- bis C10-Trifluoral-kylgruppe oder eine Cyanogruppe bezieht, und
die erste Verbindung und die zweite Verbindung in einem Gewichtsverhältnis von 1:9 bis 9:1 enthalten sind.

2. Zusammensetzung für eine organische optoelektronische Vorrichtung nach Anspruch 1, wobei Formel 1 durch eine der Formeln 1-I bis 1-IV dargestellt ist: 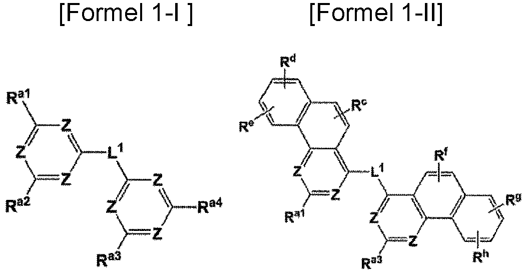 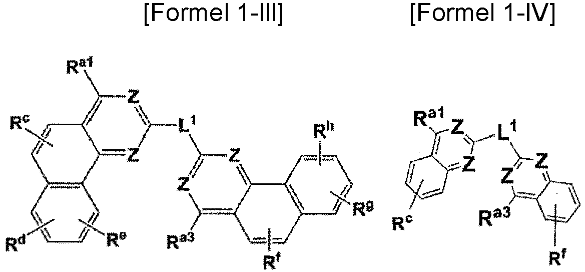 wobei in den Formeln 1-I bis 1-IV,
die Komponenten Z unabhängig voneinander N oder CR^{a} sind,
in jedem Ring, der Z enthält, mindestens eine Komponente Z N ist,
R^{a}, R^{a1} bis R^{a4}, R^{c}, R^{d}, R^{e}, R^{f}, R^{g} und R^{h} unabhängig voneinander Wasserstoff, Deuterium, eine substituierte oder unsubstituierte C1- bis C30-Alkylgruppe, eine substituierte oder unsubstituierte C6- bis C30-Arylgruppe, eine substituierte oder unsubstituierte C2- bis C30-Heteroarylgruppe oder eine Kombination davon sind,
L¹ eine C6- bis C30-Arylengruppe ist, die unsubstituiert oder mit Deuterium, einer C1- bis C40-Silylgruppe, einer C1- bis C30-Alkylgruppe, einer C3- bis C30-Cycloalkylgruppe, einer C2- bis C30-Heterocycloalkylgruppe oder einer C6- bis C30-Arylgruppe substituiert ist, wobei
wenn eine spezifische Definition nicht explizit angegeben ist, "substituiert" die gleiche Bedeutung wie in Anspruch 1 hat.

3. Zusammensetzung für eine organische optoelektronische Vorrichtung nach Anspruch 1, wobei
L¹ eine Phenylengruppe, die unsubstituiert oder mit Deuterium, einer C1- bis C40-Silylgruppe, einer C1- bis C30-Alkylgruppe oder einer C6- bis C30-Arylgruppe substituiert ist;
eine Biphenylengruppe, die unsubstituiert oder mit Deuterium, einer C1- bis C40-Silylgruppe, einer C1- bis C30-Alkylgruppe oder einer C6- bis C30-Arylgruppe substituiert ist;
eine Terphenylengruppe, die unsubstituiert oder mit Deuterium, einer C1- bis C40-Silylgruppe, einer C1- bis C30-Alkylgruppe oder einer C6- bis C30-Arylgruppe substituiert ist; oder
eine Quartphenylengruppe ist, die unsubstituiert oder mit Deuterium, einer C1- bis C40-Silylgruppe, einer C1- bis C30-Alkylgruppe oder einer C6-bis C30-Arylgruppe substituiert ist.

4. Zusammensetzung für eine organische optoelektronische Vorrichtung nach Anspruch 1, wobei L¹ ein substituierter oder unsubstituierter Linker ist, der ausgewählt ist aus Gruppen der Gruppe 2: 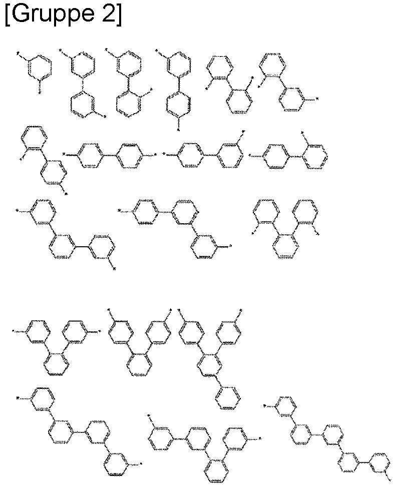 wobei in Gruppe 2 * ein Bindungspunkt mit einem benachbarten Atom ist.

5. Zusammensetzung für eine organische optoelektronische Vorrichtung nach Anspruch 1, wobei
X¹ bis X¹² in Formel 1 unabhängig voneinander N, C oder CR^{a} sind,
drei der Komponenten X¹ bis X⁶ N sind,
drei der Komponenten X⁷ bis X¹² N sind,
die Komponenten R^{a} unabhängig voneinander Wasserstoff, Deuterium, eine substituierte oder unsubstituierte C1- bis C30-Alkylgruppe, eine substituierte oder unsubstituierte C6- bis C30-Arylgruppe, eine substituierte oder unsubstituierte C2- bis C30-Heteroarylgruppe oder eine Kombination davon sind, und
L¹ eine C6- bis C30-Arylengruppe ist, die unsubstituiert oder mit Deuterium, einer C1- bis C30-Alkylgruppe oder einer C6- bis C30-Arylgruppe substituiert ist,
wobei, wenn eine spezifische Definition nicht explizit angegeben ist, "substituiert" sich auf das Ersetzen mindestens eines Wasserstoffs durch Deuterium, ein Halogen, eine Hydroxylgruppe, eine C1- bis C40-Silylgruppe, eine C1- bis C30-Alkylgruppe, eine C3- bis C30-Cycloalkylgruppe, eine C2-bis C30-Heterocycloalkylgruppe, eine C6- bis C30-Arylgruppe oder eine heterocyclische C2- bis C30-Gruppe bezieht.

6. Zusammensetzung für eine organische optoelektronische Vorrichtung nach Anspruch 5, wobei
die Komponenten R^{a} eine substituierte oder unsubstituierte C6- bis C30-Arylgruppe sind, und
die C6- bis C30-Arylgruppe eine substituierte oder unsubstituierte Phenylgruppe, eine substituierte oder unsubstituierte Biphenylgruppe, eine substituierte oder unsubstituierte Terphenylgruppe, eine substituierte oder unsubstituierte Quaterphenylgruppe, eine substituierte oder unsubstituierte Naphthylgruppe, eine substituierte oder unsubstituierte Anthracenylgruppe, eine substituierte oder unsubstituierte Fluorenylgruppe, eine substituierte oder unsubstituierte Triphenylengruppe oder eine substituierte oder unsubstituierte Phenanthrenylgruppe ist.

7. Zusammensetzung für eine organische optoelektronische Vorrichtung nach Anspruch 1, wobei
L² bis L⁴ in Formel 2 unabhängig voneinander eine Einfachbindung, eine substituierte oder unsubstituierte Phenylengruppe, eine substituierte oder unsubstituierte Biphenylengruppe, eine substituierte oder unsubstituierte Naphthylgruppe, eine substituierte oder unsubstituierte Fluorenylgruppe, eine substituierte oder unsubstituierte Pyridinylgruppe, eine substituierte oder unsubstituierte Pyrimidinylgruppe, eine substituierte oder unsubstituierte Chinolinylgruppe oder eine Kombination davon sind, und
Ar¹ bis Ar³ unabhängig voneinander eine substituierte oder unsubstituierte Phenylgruppe, eine substituierte oder unsubstituierte Biphenylgruppe, eine substituierte oder unsubstituierte Terphenylgruppe, eine substituierte oder unsubstituierte Quaterphenylgruppe, eine substituierte oder unsubstituierte Naphthylgruppe, eine substituierte oder unsubstituierte Fluorenylgruppe, eine substituierte oder unsubstituierte Carbazolylgruppe, eine substituierte oder unsubstituierte Dibenzofuranylgruppe, eine substituierte oder unsubstituierte Dibenzothiophenylgruppe, eine substituierte oder unsubstituierte Anthracenylgruppe, eine substituierte oder unsubstituierte Phenanthrenylgruppe, eine substituierte oder unsubstituierte Triphenylengruppe, eine substituierte oder unsubstituierte Chinolinylgruppe, eine substituierte oder unsubstituierte Pyridinylgruppe, eine substituierte oder unsubstituierte Pyrimidinylgruppe, eine substituierte oder unsubstituierte Thiophenylgruppe oder eine Kombination davon sind.

8. Zusammensetzung für eine organische optoelektronische Vorrichtung nach Anspruch 7, wobei Ar¹ bis Ar³ in Formel 2 ausgewählt sind aus substituierten oder unsubstituierten Gruppen der Gruppe 4: 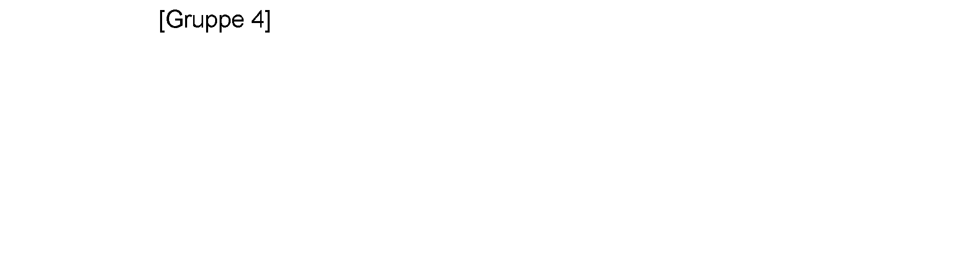 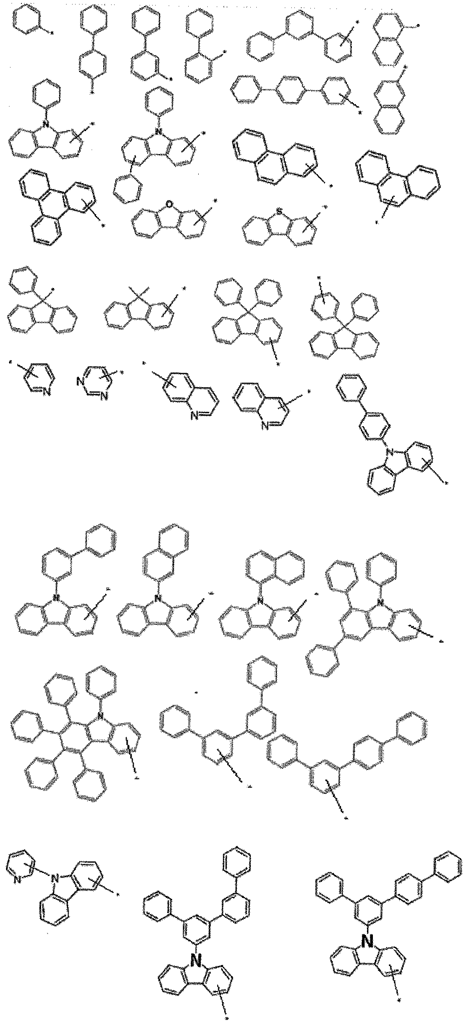 wobei in Gruppe 4 * ein Bindungspunkt mit einem benachbarten Atom ist.

9. Zusammensetzung für eine organische optoelektronische Vorrichtung nach Anspruch 1, wobei
die erste Verbindung durch die Formeln 1-la bis 1-IVa dargestellt ist, und
die zweite Verbindung die Formel 2 ist, wobei L² bis L⁴ unabhängig voneinander eine Einfachbindung oder eine substituierte oder unsubstituierte Phenylengruppe sind und Ar¹ bis Ar³ unabhängig voneinander eine substituierte oder unsubstituierte C6- bis C30-Arylgruppe oder eine substituierte oder unsubstituierte heterocyclische C2- bis C30-Gruppe sind, mit der Maßgabe, dass mindestens eine der Komponenten Ar¹ bis Ar³ eine substituierte oder unsubstituierte heterocyclische C2- bis C30-Gruppe ist: 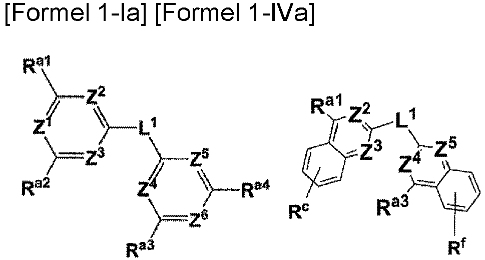 wobei in den Formeln 1-I a und 1-IVa
Z¹ bis Z⁶ unabhängig voneinander N oder CR^{a} sind,
mindestens zwei der Komponenten Z¹ bis Z³ N sind,
mindestens zwei der Komponenten Z⁴ bis Z⁶ N sind,
die Komponenten R^{a}, R^{a1} bis R^{a4}, R^{c} und R^{f} unabhängig voneinander Wasserstoff oder eine substituierte oder unsubstituierte C6- bis C30-Arylgruppe sind,
L¹ eine C6- bis C30-Arylengruppe ist, die unsubstituiert oder mit Deuterium, einer C1- bis C30-Alkylgruppe oder einer C6- bis C30-Arylgruppe substituiert ist, wobei
wenn eine spezifische Definition nicht explizit angegeben ist, "substituiert" die gleiche Bedeutung wie in Anspruch 1 hat.

10. Zusammensetzung für eine organische optoelektronische Vorrichtung nach Anspruch 1, wobei die Zusammensetzung ferner einen phosphoreszierenden Dotierstoff enthält.

11. Organisches optoelektronisches Bauelement, mit:
einer Anode (120) und einer Kathode (110), die einander zugewandt sind; und
mindestens einer zwischen der Anode und der Kathode angeordneten organischen Schicht (105),
wobei die organische Schicht die Zusammensetzung für eine organische optoelektronische Vorrichtung nach einem der Ansprüche 1 bis 10 enthält.

12. Organische optoelektronische Vorrichtung nach Anspruch 11, wobei
die organische Schicht (105) eine lichtemittierende Schicht (130) enthält, und
die lichtemittierende Schicht die Zusammensetzung für eine organische optoelektronische Vorrichtung enthält.

13. Anzeigevorrichtung mit der organischen optoelektronischen Vorrichtung nach Anspruch 11.

## Revendications

1. Composition pour un dispositif optoélectronique organique, comprenant :
au moins un premier composé représenté par la formule 1 ; et
au moins un deuxième composé représenté par la formule 2 : 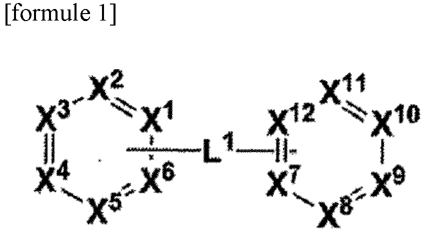 dans laquelle, dans la formule 1,
X¹ à X¹² sont indépendamment N, C, ou CR^{a},
au moins un de X¹ à X⁶ est N,
au moins un de X⁷ à X¹² est N,
R^{a'}s sont indépendamment un hydrogène, un deutérium, un groupement alkyle en C1 à C30 substitué ou non substitué, un groupement alcényle en C1 à C30 substitué ou non substitué, un groupement alcynyle en C1 à C30 substitué ou non substitué, un groupement aryle en C6 à C30 substitué ou non substitué, un groupement aryloxy en C6 à C30 substitué ou non substitué, un groupement arylthio en C6 à C30 substitué ou non substitué, un groupement hétéroaryle en C2 à C30 substitué ou non substitué, un groupement hydroxyle, un groupement thiol, ou une combinaison de ceux-ci,
R^{a'}s sont présents indépendamment ou sont reliés entre eux pour fournir un cycle,
L¹ est un groupement arylène en C6 à C30 substitué avec un deutérium ou non substitué, un groupement silyle en C1 à C40, un groupement alkyle en C1 à C30, un groupement cycloalkyle en C3 à C30, un groupement hétérocycloalkyle en C2 à C30, ou un groupement aryle en C6 à C30 ; 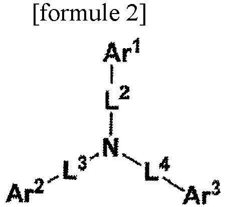
dans laquelle, dans la formule 2,
L² à L⁴ sont indépendamment une simple liaison, un groupement arylène en C6 à C30 substitué ou non substitué, ou un groupement hétéroarylène en C2 à C30 substitué ou non substitué,
Ar¹ à Ar³ sont indépendamment, un groupement aryle en C6 à C30 substitué ou non substitué, un groupement hétérocycloalkyle en C2 à C30 substitué ou non substitué, ou une combinaison de ceux-ci, et
lorsqu'une définition spécifique n'est pas fournie, «substitué » des formules 1 et 2 se réfère au remplacement d'au moins un hydrogène par un deutérium, un halogène, un groupement hydroxyle, un groupement amino, a groupement amine en C1 à C30, un groupement arylamine en C6 à C30, un groupement nitro, un groupement silyle en C1 à C40, un groupement alkyle en C1 à C30, un groupement cycloalkyle en C3 à C30, un groupement hétérocycloalkyle en C2 à C30, un groupement aryle en C6 à C30, un groupement hétérocyclique en C2 à C30, un groupement alcoxy en C1 à C20, un goupement trifluoroalkyle en C1 à C10, ou un groupement cyano ;
et le premier composé et le second composé sont inclus dans un rapport de poids de 1:9 à 9:1.

2. Composition pour un dispositif optoélectronique organique selon la revendication 1, dans laquelle la formule 1 est représentée par une de la formules 1-I à la formule 1-IV : 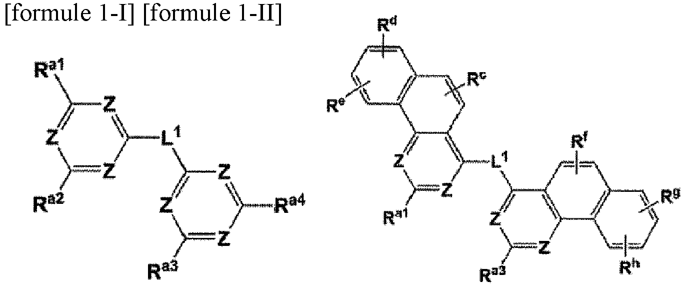 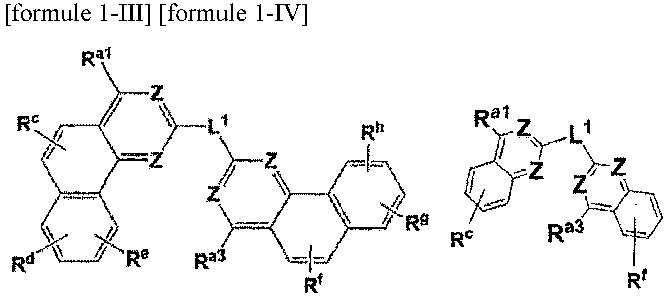 dans laquelle, dans les formules 1-I à 1-IV,
Z's sont indépendamment N, ou CR^{a},
dans chaque cycle incluant Z, au moins un Z est N,
R^{a}, R^{a1} à R^{a4}, R^{c}, R^{d}, R^{e}, R^{f}, R^{g}, et R^{h} sont indépendamment un hydrogène, un deutérium, un groupement alkyle en C1 à C30 substitué ou non substitué, un groupement aryle en C6 à C30 substitué ou non substitué, un groupement hétéroaryle en C2 à C30 substitué ou non substitué, un groupement hydroxyle, un groupement thiol, ou une combinaison de ceux-ci,
L¹ est un groupement arylène en C6 à C30 substitué avec un deutérium ou non substitué, un groupement silyle en C1 à C40, un groupement alkyle en C1 à C30, un groupement cycloalkyle en C3 à C30, un groupement hétérocycloalkyle en C2 à C30, ou un groupement aryle en C6 à C30 ; et
lorsqu'une définition spécifique n'est pas fournie, «substitué » porte la même définition que dans la revendication 1.

3. Composition pour un dispositif optoélectronique organique selon la revendication 1, dans laquelle
L¹ est un groupement phénylène substitué avec un deutérium ou non substitué, un groupement silyle en C1 à C40, un groupement alkyle en C1 à C30, ou un groupement aryle en C6 à C30 ;
un groupement biphénylène substitué avec un deutérium ou non substitué, un groupement silyle en C1 à C40, un groupement alkyle en C1 à C30, ou un groupement aryle en C6 à C30 ;
un groupement terphénylène substitué avec un deutérium ou non substitué, un groupement silyle en C1 à C40, un groupement alkyle en C1 à C30, ou un groupement aryle en C6 à C30 ; ou
un groupement quaterphénylène substitué avec un deutérium ou non substitué, un groupement silyle en C1 à C40, un groupement alkyle en C1 à C30, ou un groupement aryle en C6 à C30.

4. Composition pour un dispositif optoélectronique organique selon la revendication 1, dans laquelle
L¹ est un espaceur substitué ou non substitué sélectionné parmi les groupements du Groupe 2 : 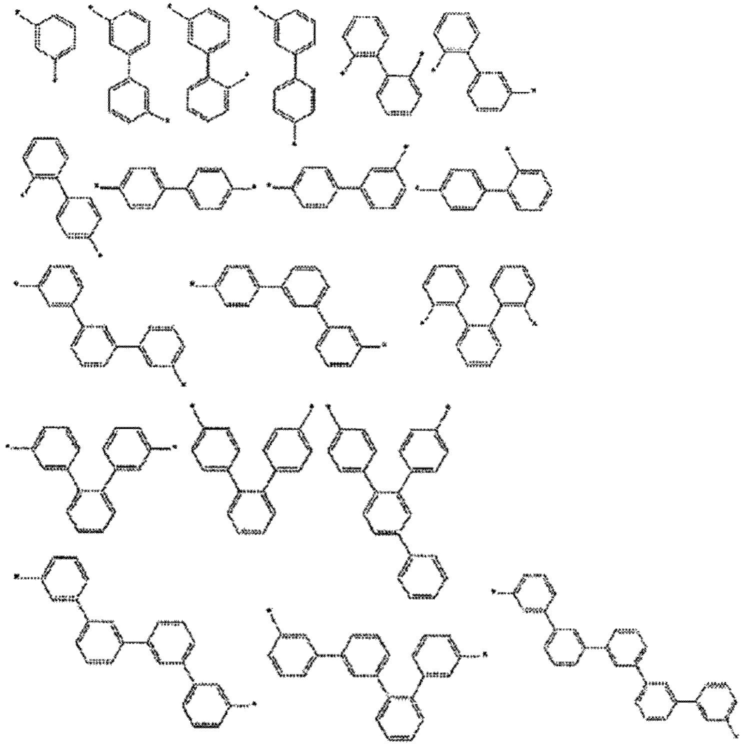 dans laquelle, dans le Groupe 2, ^{∗} est un point de liaison avec un atome adjacent.

5. Composition pour un dispositif optoélectronique organique selon la revendication 1, dans laquelle
X¹ à X¹² de la formule 1 sont indépendamment N, C, ou CR^{a},
trois de X¹ à X⁶ sont N,
trois de X⁷ à X¹² sont N,
R^{a'}s sont indépendamment un hydrogène, un deutérium, un groupement alkyle en C1 à C30 substitué ou non substitué, un groupement aryle en C6 à C30 substitué ou non substitué, un groupement hétéroaryle en C2 à C30 substitué ou non substitué, un groupement hydroxyle, un groupement thiol, ou une combinaison de ceux-ci, et
L¹ est un groupement arylène en C6 à C30 substitué avec un deutérium ou non substitué, un groupement alkyle en C1 à C30, ou un groupement aryle en C6 à C30 ;
dans laquelle lorsqu'une définition spécifique n'est pas fournie, «substitué » se réfère au remplacement d'au moins un hydrogène par un deutérium, un halogène, un groupement hydroxyle, un groupement silyle en C1 à C40, un groupement alkyle en C1 à C30, un groupement cycloalkyle en C3 à C30, un groupement hétérocycloalkyle en C2 à C30, un groupement aryle en C6 à C30, un groupement hétérocyclique en C2 à C30.

6. Composition pour un dispositif optoélectronique organique selon la revendication 5, dans laquelle
R^{a'}s sont un groupement aryle en C6 à C30 substitué ou non substitué, et
le groupement aryle en C6 à C30 est un groupement phényle substitué ou non substitué, un groupement biphényle substitué ou non substitué, un groupement terphényle substitué ou non substitué, un groupement quaterphényle substitué ou non substitué, un groupement naphtyle substitué ou non substitué, un groupement anthracényle substitué ou non substitué, un groupement fluorényle substitué ou non substitué, un groupement triphénylène substitué ou non substitué, ou un groupement phénanthrényle substitué ou non substitué.

7. Composition pour un dispositif optoélectronique organique selon la revendication 1, dans laquelle
L² à L⁴ de la formule 2 sont indépendamment une simple liaison, un groupement phénylène substitué ou non substitué, un groupement biphénylène substitué ou non substitué, un groupement naphtyle substitué ou non substitué, un groupement fluorényle substitué ou non substitué, un groupement pyridinyle substitué ou non substitué, un groupement pyrimidinyle substitué ou non substitué, un groupement quinolinyle substitué ou non substitué, ou une combinaison de ceux-ci,
Ar¹ à Ar³ sont indépendamment un groupement phényle substitué ou non substitué, un groupement biphényle substitué ou non substitué, un groupement terphényle substitué ou non substitué, un groupement quaterphényle substitué ou non substitué, un groupement naphtyle substitué ou non substitué, un groupement fluorényle substitué ou non substitué, un groupement carbazolyle substitué ou non substitué, un groupement dibenzofuranyle substitué ou non substitué, un groupement dibenzothiophényle substitué ou non substitué, un groupement anthracényle substitué ou non substitué, un groupement phénanthrényle substitué ou non substitué, un groupement triphénylène substitué ou non substitué, un groupement pyridinyle substitué ou non substitué, un groupement pyrimidinyle substitué ou non substitué, un groupement thiophényle substitué ou non substitué, ou une combinaison de ceux-ci.

8. Composition pour un dispositif optoélectronique organique selon la revendication 7, dans laquelle
Ar¹ à Ar³ de la formule 2 sont sélectionnés parmi les groupements substitués ou non substitués du Groupe 4 : 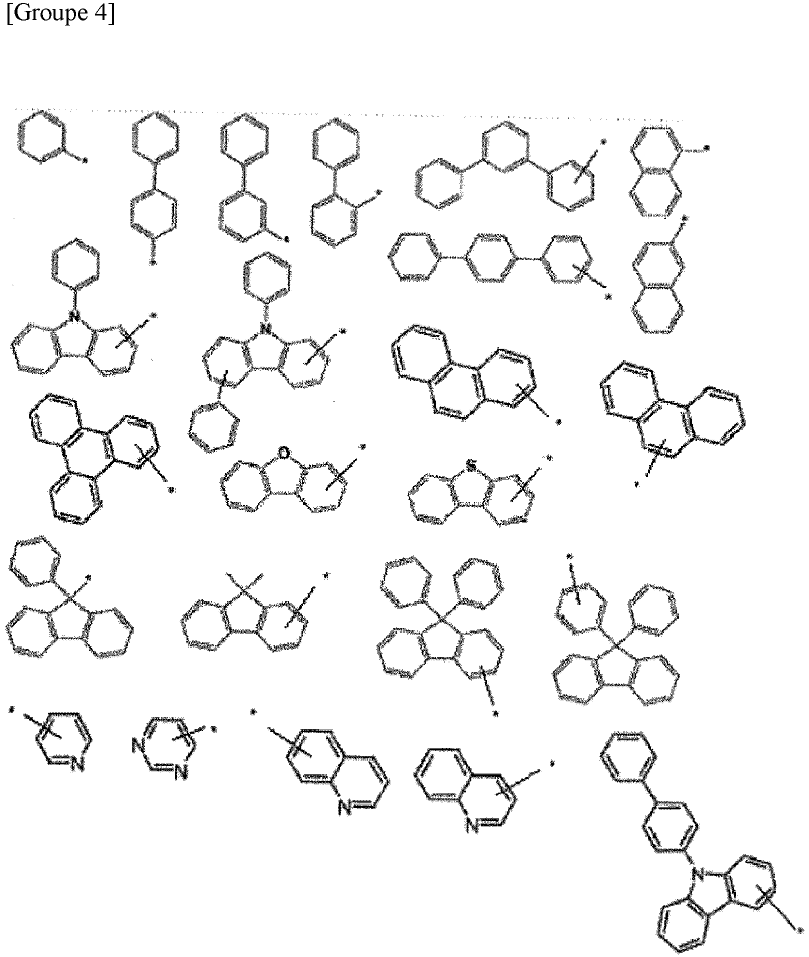 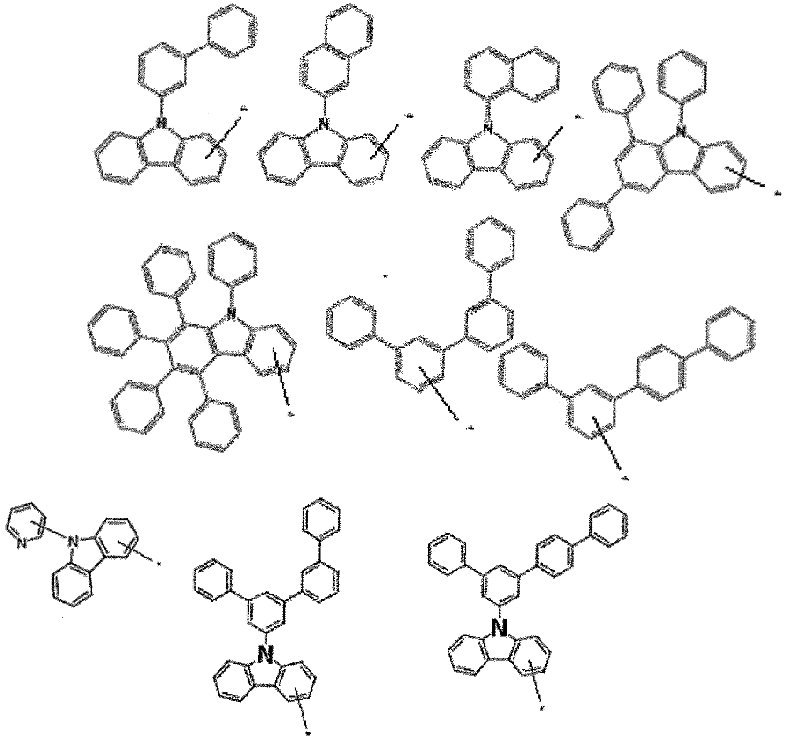 dans laquelle, dans le Groupe 4, ^{∗} est un point de liaison avec un atome adjacent.

9. Composition pour un dispositif optoélectronique organique selon la revendication 1, dans laquelle
le premier composé est représenté par les formules 1-Ia à 1-IVa, et
le second composé est représenté par la formule 2 dans laquelle L² à L⁴ sont indépendamment une simple liaison ou un groupement phénylène substitué ou non substitué, et Ar¹ à Ar³ sont indépendamment un groupement aryle en C6 à C30 substitué ou non substitué ou un groupement hétérocyclique en C2 à C30 substitué ou non substitué, pourvu que au moins un de Ar¹ à Ar³ soit un groupement hétérocyclique en C2 à C30 substitué ou non substitué : 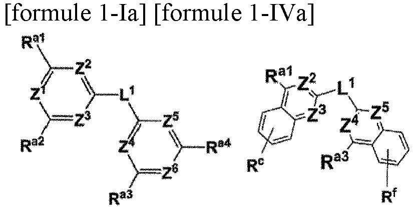 dans laquelle, dans les formules 1-Ia à 1-IVa,
Z¹ à Z⁶ sont indépendamment N, C, ou CR^{a},
au moins deux de Z¹ à Z³ sont N,
au moins deux de Z⁴ à Z⁶ sont N,
R^{a'}s, R^{a1} à R^{a4}, R^{c}, R^{f} sont indépendamment un hydrogène, ou un groupement aryle en C6 à C30 substitué ou non substitué,
L¹ est un groupement arylène en C6 à C30 substitué avec un deutérium ou non substitué, un groupement alkyle en C1 à C30, ou un groupement aryle en C6 à C30 ;
lorsqu'une définition spécifique n'est pas fournie, «substitué » porte la même définition que dans la revendication 1.

10. Composition pour un dispositif optoélectronique organique selon la revendication 1, dans laquelle la composition inclut en outre un agent dopant phosphorescent.

11. Dispositif optoélectronique organique, comprenant
une anode (120) et une cathode (110) se faisant face, et
une moins une couche organique (105) disposée entre l'anode et la cathode,
dans lequel la couche organique inclut la composition pour un dispositif optoélectronique organique selon l'une quelconque des revendications 1 à 10.

12. Dispositif optoélectronique organique selon la revendication 11, dans lequel
la couche organique (105) inclut une couche émettrice de lumière (130),
la couche émettrice de lumière inclut la composition pour un dispositif optoélectronique organique.

13. Dispositif d'affichage comprenant le dispositif optoélectronique organique selon la revendication 11.
